# EUROPEAN PATENT APPLICATION

(11) **EP 4 570 793 A1**
(43) Date of publication of application: **18.06.2025**
(21) Application number: 23849504.8
(22) Date of filing: 03.08.2023
(51) Int. Cl.: C07D 401/12, C07D 405/12, C07D 211/58, C07D 211/76, C07D 413/12, C07D 471/04, A61K 31/454, A61K 31/4535, A61K 31/4545, A61P 25/18, A61P 25/22, A61P 25/24, A61P 25/06, A61P 25/28

(54) **5-HT2A RECEPTOR INVERSE AGONIST, AND PREPARATION METHOD THEREFOR AND USE THEREOF**

(30) Priority: 03.08.2022 CN 202210925481; 01.10.2022 CN 202211218066; 20.10.2022 CN 202211289428; 30.12.2022 CN 202211724352; 02.06.2023 CN 202310646192
(71) Applicant: Luye Innomind Pharma Shijiazhuang Co., Ltd., Shijiazhuang, Hebei 050000 (CN)
(72) Inventor: TIAN, Jingwei, Yantai, Shandong 264670 (CN); YE, Liang, Yantai, Shandong 264670 (CN); MA, Mingxu, Yantai, Shandong 264670 (CN); WANG, Wenyan, Yantai, Shandong 264670 (CN); DU, Guangying, Yantai, Shandong 264670 (CN); ZHANG, Jianzhao, Yantai, Shandong 264670 (CN); DAI, Yusen, Yantai, Shandong 264670 (CN); ZHANG, Rui, Yantai, Shandong 264670 (CN)
(74) Representative: karo IP
(86) International application number: PCT/CN2023/111052
(87) International publication number: WO 2024/027800

(57) **Abstract**

The present invention relates to a new compound as a 5-HT_{2A} receptor inverse agonist, a preparation method therefor and a pharmaceutical composition. The present invention further relates to the use of the compound or the pharmaceutical composition in the preparation of a drug for treating 5-HT_{2A} receptor-related diseases. The diseases include non-motor symptoms caused by Parkinson's disease: delusions, hallucination, depression, anxiety, cognitive impairment and sleep disorders; dementia-related mental diseases; severe depression; or negative symptoms of schizophrenia, etc.

## Description

### TECHNICAL FIELD

The present disclosure relates to a compound serving as a 5-hydroxytryptamine 2A (5-HT_{2A}) receptor inverse agonist, a preparation method therefor, and use thereof in the field of 5-HT_{2A} receptor-related diseases.

### BACKGROUND

Parkinson's disease (PD) is a common neurodegenerative disease, with an average onset age of around 60 years (Degirmenci, Yildiz. Cumhuriyet Medical Journal (2017), 39(3), 509-517.). According to data from the National Institutes of Health (NIH) in 2018, there are about 4-6 million Parkinson's disease patients worldwide. Among them, up to 50% experience severe symptoms of hallucinations or delusions during the disease course, which seriously affects their quality of life. This disease has high incidence and mortality rate.

For a long time, antipsychotic drugs have been used primarily in clinical practice to treat hallucinations and delusions in Parkinson's disease patients. First-generation antipsychotic drugs primarily inhibit dopamine D₂ receptors and are associated with severe extrapyramidal side effects. Second-generation antipsychotic drugs, in addition to inhibiting D₂ receptors, also more significantly inhibit specific 5-HT receptors, particularly the 5-HT_{2A} receptor, offering better safety, i.e., fewer extrapyramidal side effects compared to the first-generation antipsychotic drugs. However, since the second-generation antipsychotic drugs still have inhibitory activity against the D₂ receptors, they still cause extrapyramidal side effects. Additionally, such drugs also lead to the side effect of weight gain to varying degrees. In 2016, the U.S. FDA approved pimavanserin for marketing to treat hallucination and delusion symptoms associated with Parkinson's disease, making it the first drug approved for this indication.

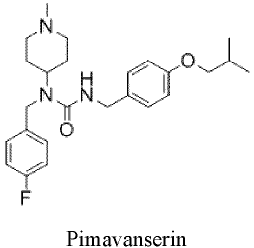

Pimavanserin is a 5-HT_{2A} receptor inverse agonist that can eliminate the extrapyramidal and weight gain side effects associated with dopamine receptor inhibition by first- and second-generation antipsychotic drugs, offering better safety. 5-HT_{2A} is a major excitatory receptor subtype in the 5-HT receptor family, belonging to ligand-gated channels and G protein-coupled receptors. The function of the 5-HT_{2A} receptor is closely related to neuronal excitation, behavior effects, learning and memory, anxiety, and the like. This receptor is an important action target for antipsychotic drugs and the treatment of schizophrenia (Price, D.L., et al. Behavioural Pharmacology (2012), 23(4), 426-433.).

The 5-HT_{2A} receptor has intrinsic activity and is able to exert its effect even in the absence of an agonist. Pimavanserin as a 5-HT_{2A} receptor inverse agonist, upon binding to the 5-HT_{2A} receptor, can inhibit the intrinsic activity of the receptor, making the receptor non-functional and resulting in an effect opposite to that of an agonist. It can still exhibit activity even in the absence of an agonist. In contrast, typical 5-HT_{2A} receptor antagonists cannot induce biological effects upon binding to the receptor and can only exhibit their activity by inhibiting agonists (WO2004064738A2). Thus, even if a compound has 5-HT_{2A} receptor antagonist activity, it may not necessarily have 5-HT_{2A} receptor inverse agonist activity.

5-HT_{2A} receptor inverse agonists have good application prospects as drugs in the pharmaceutical industry, but currently only pimavanserin has been approved for marketing. Therefore, there is still a need to develop more 5-HT_{2A} receptor inverse agonists to achieve better therapeutic effects and meet the needs of clinical patients.

### SUMMARY

In one aspect, the present disclosure provides a compound of formula (A) or a pharmaceutically acceptable salt, a stereoisomer or a deuteride thereof: 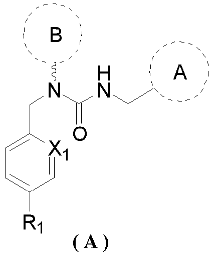 wherein R₁ is halogen; ring B is selected from the group consisting of 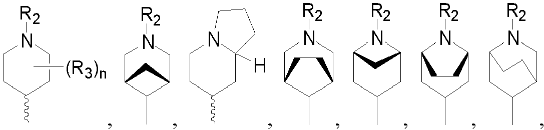 or 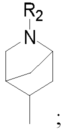
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 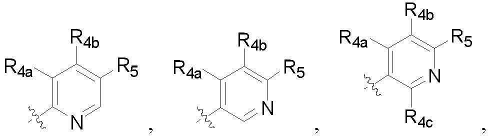 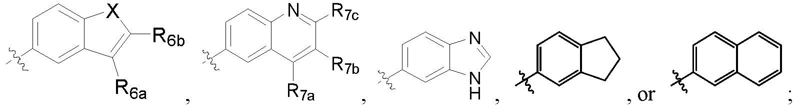
X is selected from the group consisting of NR_{6c}, O, or S;
X₁ is selected from the group consisting of N or CH;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; n is selected from the group consisting of 0, 1, 2, or 3.

In some embodiments of the compound of formula (A): ring A is selected from the group consisting of 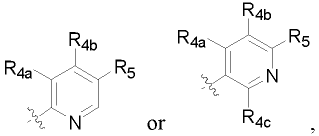 preferably 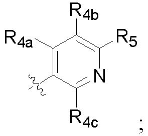 the other variables are as defined herein.

In some embodiments of the compound of formula (A): ring B is 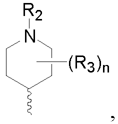 preferably 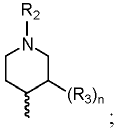
R₃ is halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2; the other variables are as defined herein.

In some embodiments of the compound of formula (A):
R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl; the other variables are as defined herein.

In some embodiments of the compound of formula (A):
R₁ is halogen;
ring B is 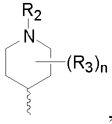 preferably 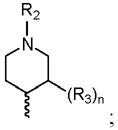 R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 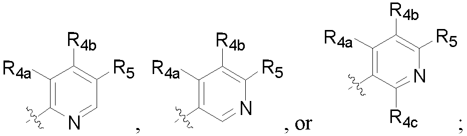
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3; the other variables are as defined herein.

In another aspect of the present disclosure, the present disclosure provides a compound of formula (I) or a pharmaceutically acceptable salt, a stereoisomer or a deuteride thereof: 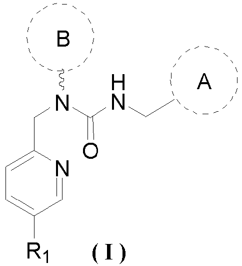 wherein
R₁ is halogen;
ring B is selected from the group consisting of 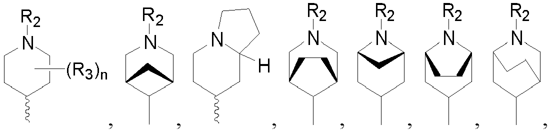 or 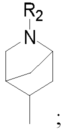
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 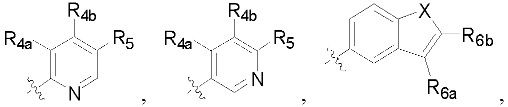 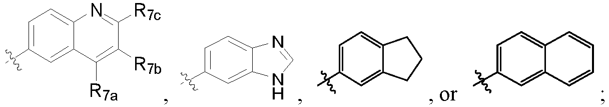
X is selected from the group consisting of NR_{6c}, O, or S;
R₄ₐ, R_{4b}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

In some embodiments of the compound of formula (I):
ring A is selected from the group consisting of 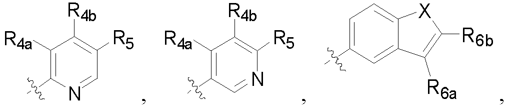 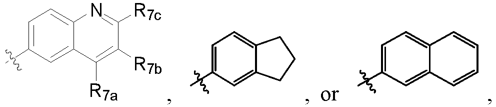 preferably 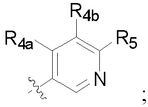 the other variables are as defined herein.

In some embodiments of the compound of formula (I): ring B is 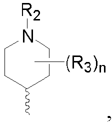 preferably 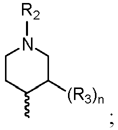
R₃ is halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2; the other variables are as defined herein.

In some embodiments of the compound of formula (I):
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl; the other variables are as defined herein.

In some embodiments of the compound of formula (I):
R₁ is F;
ring B is selected from the group consisting of 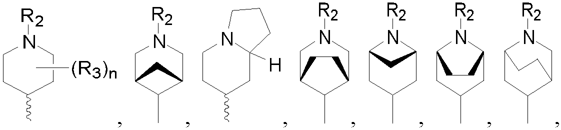 or 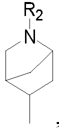 , preferably 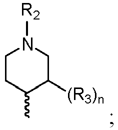
R₂ is selected from the group consisting of hydrogen, methyl, CD₃, ethyl, *n*-propyl, isopropyl, or cyclopropyl;
each R₃ is independently selected from the group consisting of hydrogen, F, or methyl, or two R₃ attached to the same carbon atom, together with the carbon atom to which they are attached, form cyclopropyl;
ring A is selected from the group consisting of 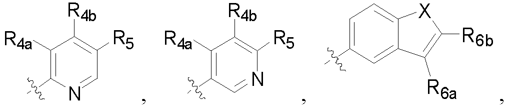 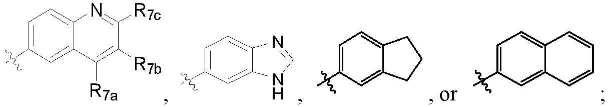
X is selected from the group consisting of NR_{6c}, O, or S;
R₄ₐ, R_{4b}, R₆ₐ, R_{6b}, R_{6c}, R₇ₐ, R_{7b}, and R_{7c} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl;
n is selected from the group consisting of 0, 1, 2, or 3.

In some embodiments of the compound of formula (I):
R₁ is F;
ring B is selected from the group consisting of 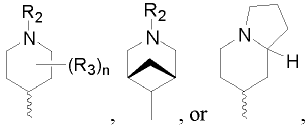 preferably 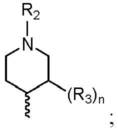
R₂ is selected from the group consisting of hydrogen, methyl, CD₃, ethyl, *n*-propyl, isopropyl, or cyclopropyl;
R₃ is selected from the group consisting of hydrogen, F, or methyl, or two R₃ attached to the same carbon atom, together with the carbon atom to which they are attached, form cyclopropyl;
ring A is selected from the group consisting of 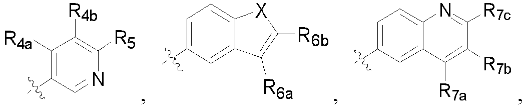 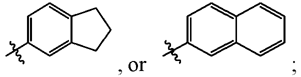
X is selected from the group consisting of NR_{6c}, O, or S;
R₄ₐ, R_{4b}, R₆ₐ, R_{6b}, R_{6c}, R₇ₐ, R_{7b}, and R_{7c} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl;
n is selected from the group consisting of 0, 1, or 2.

In some embodiments of the compound of formula (I):
R₁ is halogen;
ring B is 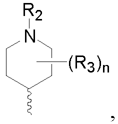 preferably 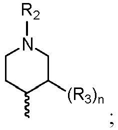
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 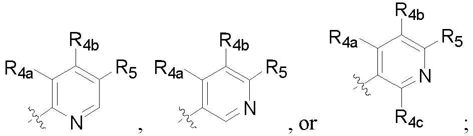
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3; the other variables are as defined herein.

In some embodiments of the compound of formula (I):
R₁ is F;
ring B is selected from the group consisting of 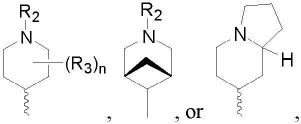 preferably 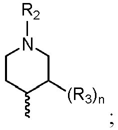
R₂ is selected from the group consisting of hydrogen or methyl;
ring A is 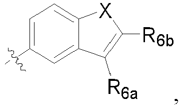 wherein X is selected from the group consisting of NR_{6c}, O, or S;
R₆ₐ, R_{6b}, and R_{6c} are each independently selected from the group consisting of hydrogen, F, Cl, Br, methyl, or trifluoromethyl.

In some embodiments of the compound of formula (I):
R₁ is F;
ring B is selected from the group consisting of 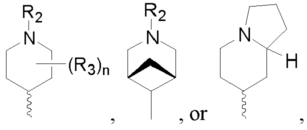 preferably 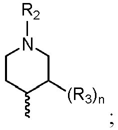
R₂ is selected from the group consisting of hydrogen or methyl;
R₃ is selected from the group consisting of hydrogen, F, or methyl, or two R₃ attached to the same carbon atom, together with the carbon atom to which they are attached, form cyclopropyl;
ring A is selected from the group consisting of 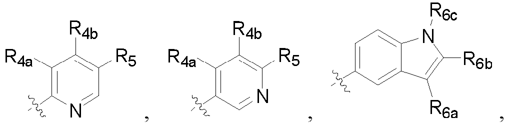 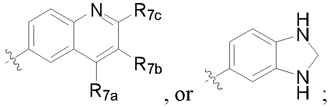
R₄ₐ, R_{4b}, R₆ₐ, R_{6b}, R_{6c}, R₇ₐ, R_{7b}, and R_{7c} are each independently selected from the group consisting of hydrogen, F, Cl, Br, methyl, or trifluoromethyl;
R₅ is -OR₅', wherein R₅' is selected from the group consisting of methyl, ethyl, n-propyl, isopropyl, n-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl;
n is selected from the group consisting of 0, 1, or 2.

In some embodiments of the compound of formula (I):
R₁ is halogen;
ring B is 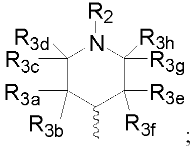
R₂ is independently selected from the group consisting of hydrogen, C₁₋₃ alkyl, or C₃₋₆ cycloalkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl;
ring A is selected from the group consisting of 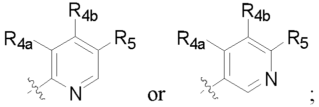
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl; preferably, R₅' is selected from the group consisting of C₁₋₆ haloalkyl, C₃₋₆ cycloalkyl, or C₃₋₆ halocycloalkyl.

In some embodiments of the compound of formula (I):
R₁ is F;
ring B is 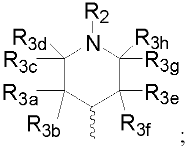
R₂ is independently selected from the group consisting of hydrogen, methyl, CD₃, ethyl, *n*-propyl, isopropyl, or cyclopropyl, preferably methyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
ring A is selected from the group consisting of 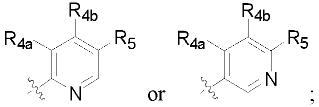
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl.

In another aspect of the present disclosure, the present disclosure provides a compound of formula (II) or a pharmaceutically acceptable salt, a stereoisomer or a deuteride thereof: 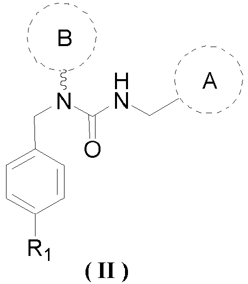 wherein
R₁ is halogen;
ring B is selected from the group consisting of 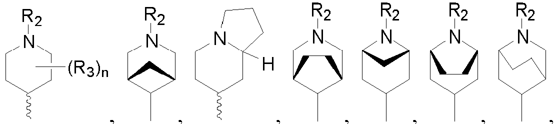 or 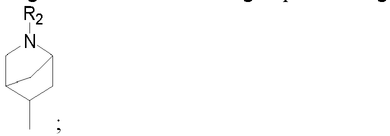
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 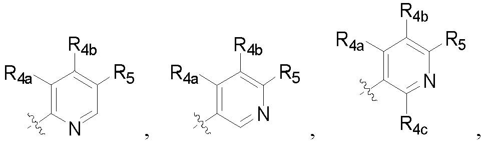 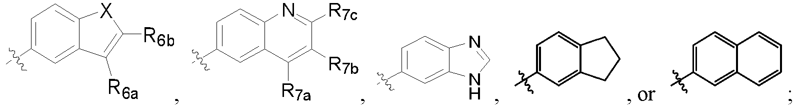
X is selected from the group consisting of NR_{6c}, O, or S;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

In some embodiments of the compound of formula (II):
ring A is selected from the group consisting of 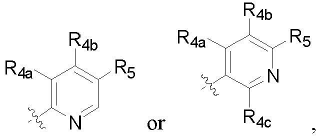 preferably 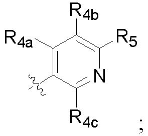 the other variables are as defined herein.

In some embodiments of the compound of formula (II):
ring B is 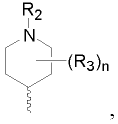 preferably 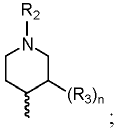
R₃ is halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2; the other variables are as defined herein.

In some embodiments of the compound of formula (II):
R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl; the other variables are as defined herein.

In some embodiments of the compound of formula (II):
R₁ is halogen;
ring B is 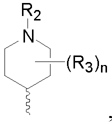 preferably 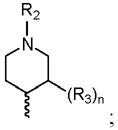
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 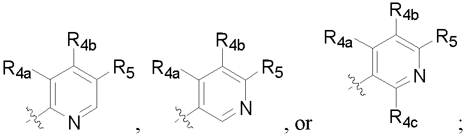
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3; the other variables are as defined herein.

In some embodiments of the compound of formula (II):
R1 is F;
ring B is selected from the group consisting of 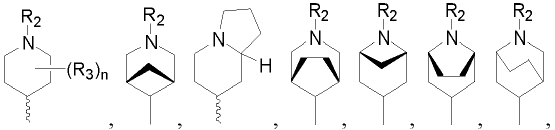 or 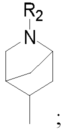
R₂ is selected from the group consisting of hydrogen, methyl, CD₃, ethyl, n-propyl, isopropyl, or cyclopropyl;
each R₃ is selected from the group consisting of hydrogen, F, or methyl, or two R₃ attached to the same carbon atom, together with the carbon atom to which they are attached, form cyclopropyl;
ring A is selected from the group consisting of 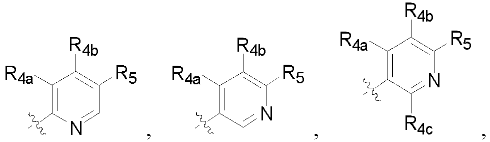 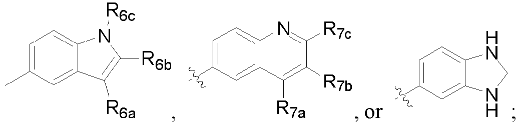
R₄ₐ, R_{4b}, R_{4c}, R₆ₐ, R_{6b}, R_{6c}, R₇ₐ, R_{7b}, and R_{7c} are each independently selected from the group consisting of hydrogen, F, Cl, Br, methyl, or trifluoromethyl;
R₅ is -OR₅', wherein R₅' is selected from the group consisting of methyl, ethyl, n-propyl, isopropyl, n-butyl, isobutyl, *tert-*butyl, 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl;
n is selected from the group consisting of 0, 1, 2, or 3.

In some embodiments of the compound of formula (II):
R₁ is halogen;
ring B is 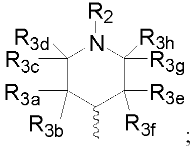
R₂ is independently selected from the group consisting of hydrogen, C₁₋₃ alkyl, or C₃₋₆ cycloalkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl, or R₃ₐ and R_{3b}, R_{3c} and R_{3d}, R₃ₑ and R_{3f}, or R_{3g} and R_{3b}, together with the carbon atom to which they are attached, form C₃₋₆ cycloalkyl;
ring A is selected from the group consisting of 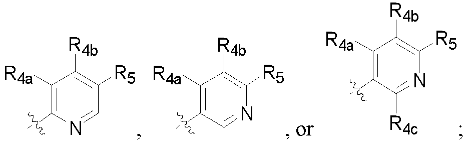
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl.

In some embodiments of the compound of formula (II):
R1 is F;
ring B is 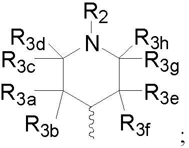
R₂ is independently selected from the group consisting of hydrogen, methyl, CD₃, ethyl, *n*-propyl, isopropyl, or cyclopropyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
ring A is selected from the group consisting of 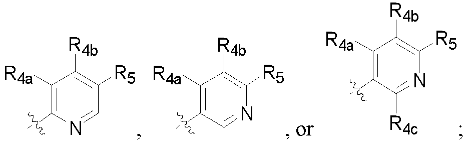
R₄ₐ, R_{4b}, and R_{4c} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert-butyl,* 2,2,2-trifluoroethyl, cyclopropyl, cyclobutyl, 3,3-difluorocyclobutyl, cyclopropylmethyl, or 2,2-dimethylcyclopropylmethyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl.

In one aspect, the present disclosure provides a compound of formula (IIA) or a pharmaceutically acceptable salt, a stereoisomer or a deuteride thereof: 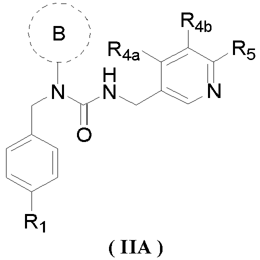 wherein
R₁ is halogen;
ring B is selected from the group consisting of 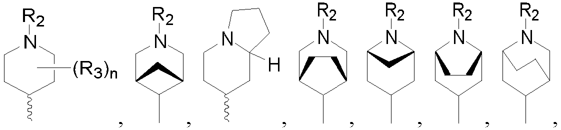 or 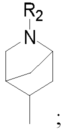
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl; preferably, R₅' is selected from the group consisting of C₁₋₆ haloalkyl, C₃₋₆ cycloalkyl, or C₃₋₆ halocycloalkyl;
n is selected from the group consisting of 0, 1, 2, or 3.

In some embodiments of the compound of formula (IIA):
ring B is 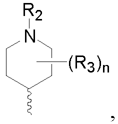 preferably 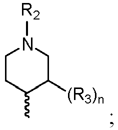
R₃ is halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2; the other variables are as defined herein.

In some embodiments of the compound of formula (IIA):
R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl; the other variables are as defined herein.

In some embodiments of the compound of formula (IIA):
R₁ is halogen;
ring B is 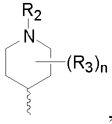 preferably 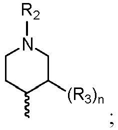
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3; the other variables are as defined herein.

In some embodiments of the compound of formula (IIA):
R1 is F;
ring B is selected from the group consisting of 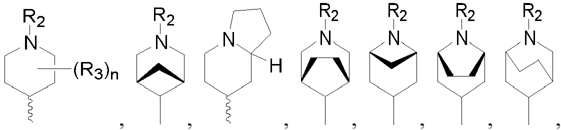 or 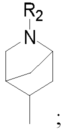
R₂ is selected from the group consisting of hydrogen, methyl, CD₃, ethyl, n-propyl, isopropyl, or cyclopropyl; each R₃ is independently selected from the group consisting of hydrogen, F, or methyl;
R₄ₐ and R_{4b} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, or 3,3-difluorocyclobutyl; preferably, R₅' is selected from the group consisting of 2,2,2-trifluoroethyl or 3,3-difluorocyclobutyl;
n is selected from the group consisting of 0, 1, 2, or 3.

In one aspect, the present disclosure provides a compound of formula (IIA-1) or (IIA-2) or a pharmaceutically acceptable salt, a stereoisomer or a deuteride thereof: 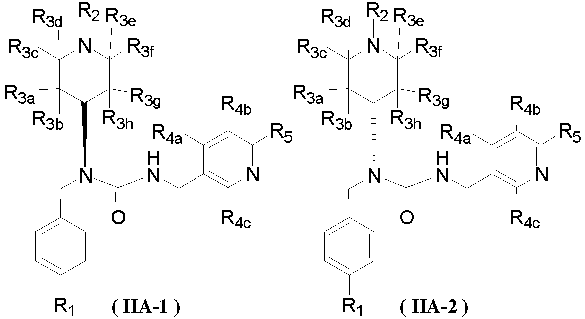 wherein
R₁ is halogen;
R₂ is independently selected from the group consisting of hydrogen, C₁₋₃ alkyl, or C₃₋₆ cycloalkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl, or R₃ₐ and R_{3b}, R_{3c} and R_{3d}, R₃ₑ and R_{3f}, or R_{3g} and R_{3b}, together with the carbon atom to which they are attached, form C₃₋₆ cycloalkyl;
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl.

In some embodiments of the compound of formula (IIA-1) or (IIA-2):
R₅' is selected from the group consisting of C₁₋₆ haloalkyl, C₃₋₆ cycloalkyl, or C₃₋₆ halocycloalkyl; preferably, R₅' is selected from the group consisting of 2,2,2-trifluoroethyl, cyclopropyl, cyclobutyl, or 3,3-difluorocyclobutyl;
the other variables are as defined herein.

In some embodiments of the compound of formula (IIA-1) or (IIA-2):
R1 is F;
R₂ is selected from the group consisting of hydrogen, methyl, CD₃, ethyl, *n*-propyl, isopropyl, or cyclopropyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
R₄ₐ, R_{4b}, and R_{4c} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl.

In one aspect, the present disclosure provides a compound of formula (IIB) or a pharmaceutically acceptable salt, a stereoisomer or a deuteride thereof: 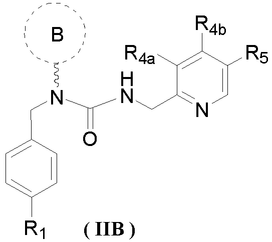 wherein
R₁ is halogen;
ring B is selected from the group consisting of 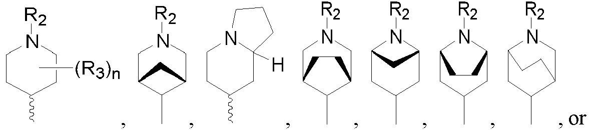 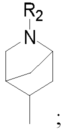
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl;
n is selected from the group consisting of 0, 1, 2, or 3.

In some embodiments of the compound of formula (IIB):
R1 is F;
ring B is selected from the group consisting of 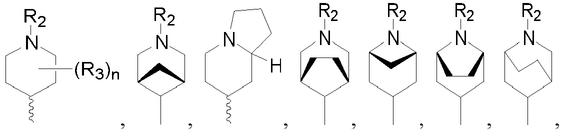 or 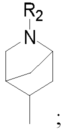
R₂ is selected from the group consisting of hydrogen, methyl, CD₃, ethyl, *n*-propyl, isopropyl, or cyclopropyl; each R₃ is independently selected from the group consisting of hydrogen, F, or methyl;
R₄ₐ and R_{4b} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, or 3,3-difluorocyclobutyl;
n is selected from the group consisting of 0, 1, 2, or 3.

In another aspect, the present disclosure provides a compound of formula (III) or a pharmaceutically acceptable salt, a stereoisomer or a deuteride thereof: 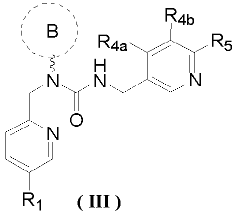 wherein
R₁ is halogen;
ring B is selected from the group consisting of 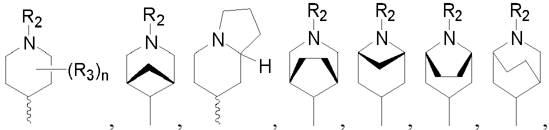 or 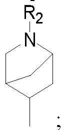
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

In some embodiments of the compound of formula (III):
ring B is 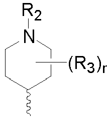 , preferably 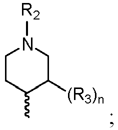
R₃ is halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2; the other variables are as defined herein.

In some embodiments of the compound of formula (III):
R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl; preferably, R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl; the other variables are as defined herein.

In some embodiments of the compound of formula (III):
R₁ is halogen;
ring B is 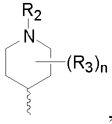 preferably 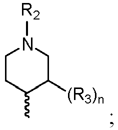
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 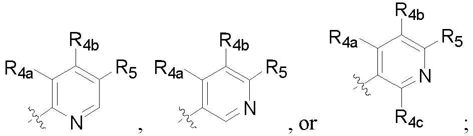
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3; the other variables are as defined herein.

In some embodiments of the compound of formula (III):
R₁ is halogen;
ring B is selected from the group consisting of 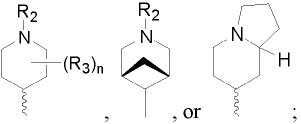
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, or 2.

In some embodiments of the compound of formula (III):
R₁ is F;
ring B is selected from the group consisting of 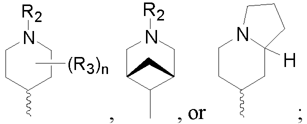
R₂ is independently selected from the group consisting of hydrogen or methyl;
each R₃ is independently selected from the group consisting of hydrogen, F, Cl, Br, or methyl, or two R₃ attached to the same carbon atom, together with the carbon atom to which they are attached, form cyclopropyl;
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, cyclopropyl, cyclobutyl, 3,3-difluorocyclobutyl, cyclopropylmethyl, or 2,2-dimethylcyclopropylmethyl;
n is selected from the group consisting of 0, 1, or 2.

In some embodiments of the compound of formula (III):
R₁ is halogen;
ring B is selected from the group consisting of 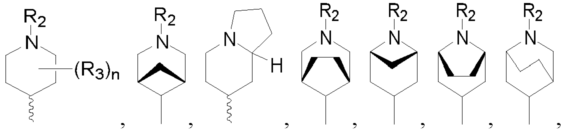 or 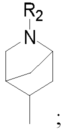
R₂ is independently selected from the group consisting of hydrogen, C₁₋₃ alkyl, or C₃₋₆ cycloalkyl;
each R₃ is independently selected from the group consisting of hydrogen, halogen, or C₁₋₃ alkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl;
n is selected from the group consisting of 0, 1, 2, or 3.

In some embodiments of the compound of formula (III):
R1 is F;
ring B is selected from the group consisting of 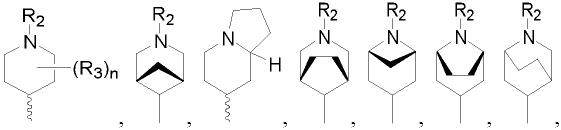 or 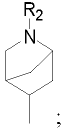
R₂ is selected from the group consisting of hydrogen, methyl, CD₃, ethyl, *n*-propyl, isopropyl, or cyclopropyl; each R₃ is independently selected from the group consisting of hydrogen, F, or methyl;
R₄ₐ and R_{4b} are each independently selected from the group consisting of hydrogen, F, Cl, Br, methyl, or trifluoromethyl;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, or 3,3-difluorocyclobutyl;
n is selected from the group consisting of 0, 1, 2, or 3.

In some embodiments of the compound of formula (III):
R₁ is halogen;
ring B is 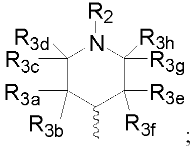
R₂ is independently selected from the group consisting of hydrogen, C₁₋₃ alkyl, or C₃₋₆ cycloalkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl, or R₃ₐ and R_{3b}, R_{3c} and R_{3d}, R₃ₑ and R_{3f}, or R_{3g} and R_{3b}, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl.

In some embodiments of the compound of formula (III):
R1 is F;
ring B is 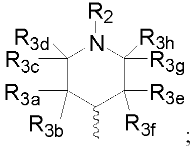
R₂ is independently selected from the group consisting of hydrogen or methyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert-*butyl, 2,2,2-trifluoroethyl, cyclopropyl, cyclobutyl, 3,3-difluorocyclobutyl, cyclopropylmethyl, or 2,2-dimethylcyclopropylmethyl.

In another aspect, the present disclosure provides a compound of formula (IIIA) or (IIIB) or a pharmaceutically acceptable salt, a stereoisomer or a deuteride thereof: 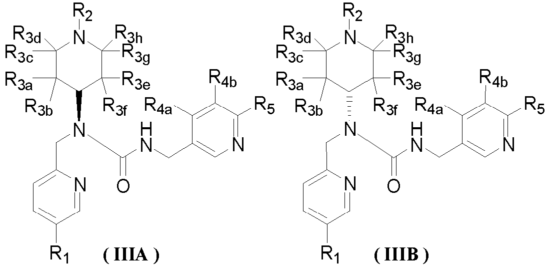 wherein
R₁ is halogen;
R₂ is selected from the group consisting of hydrogen or C₁₋₃ alkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl, or R₃ₐ and R_{3b}, R_{3c} and R_{3d}, R₃ₑ and R_{3f}, or R_{3g} and R_{3b}, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl.

In some embodiments of the compound of formula (IIIA) or (IIIB):
R1 is F;
R₂ is selected from the group consisting of hydrogen or methyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl.

In another aspect, the present disclosure provides a compound of formula (IIIC) or a pharmaceutically acceptable salt, a stereoisomer or a deuteride thereof: 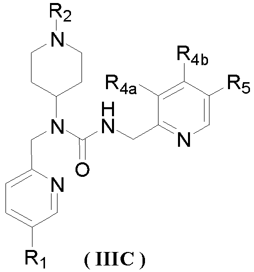 wherein
R₁ is halogen;
R₂ is selected from the group consisting of hydrogen or C₁₋₃ alkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl.

In some embodiments of the compound of formula (II):
R1 is F;
R₂ is selected from the group consisting of hydrogen or methyl;
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert*-butyl, 2,2,2-trifluoroethyl, cyclopropyl, cyclobutyl, 3,3-difluorocyclobutyl, cyclopropylmethyl, or 2,2-dimethylcyclopropylmethyl.

In another aspect, the present disclosure provides a compound of formula (IV) or a pharmaceutically acceptable salt, a stereoisomer or a deuteride thereof: 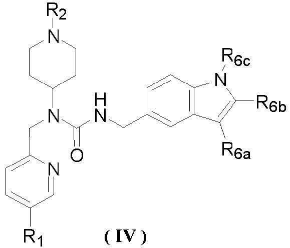 wherein
R₁ is halogen;
R₂ is selected from the group consisting of hydrogen or C₁₋₃ alkyl;
R₆ₐ and R_{6b} are each independently selected from the group consisting of hydrogen, halogen, or C₁₋₃ haloalkyl; R_{6c} is selected from the group consisting of hydrogen or C₁₋₃ alkyl.

In some embodiments of the compound of formula (IV):
R1 is F;
R₂ is selected from the group consisting of hydrogen or methyl;
R₆ₐ and R_{6b} are each independently selected from the group consisting of hydrogen, F, or trifluoromethyl;
R_{6c} is selected from the group consisting of hydrogen or methyl.

In another aspect, the present disclosure provides a compound of formula (V) or a pharmaceutically acceptable salt, a stereoisomer or a deuteride thereof: 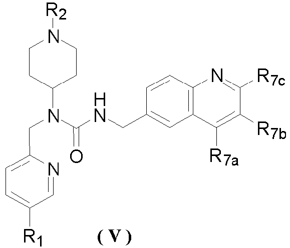 wherein
R₁ is halogen;
R₂ is selected from the group consisting of hydrogen or C₁₋₃ alkyl;
R₇ₐ, R_{7b}, and R_{7c} are each independently selected from the group consisting of hydrogen, halogen, or C₁₋₃ haloalkyl.

In some embodiments of the compound of formula (V):
R1 is F;
R₂ is selected from the group consisting of hydrogen or methyl;
R₇ₐ, R_{7b}, and R_{7c} are each independently selected from the group consisting of hydrogen, F, or trifluoromethyl.

In another aspect of the present disclosure, the present disclosure provides a compound of formula (VI) or a pharmaceutically acceptable salt, a stereoisomer or a deuteride thereof: 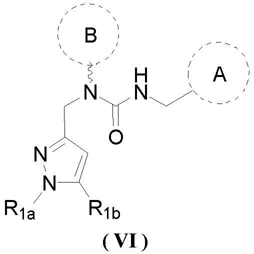 wherein
R₁ₐ and R_{1b} are each independently selected from the group consisting of hydrogen or C₁₋₃ alkyl;
ring B is selected from the group consisting of 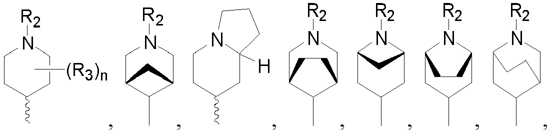 or 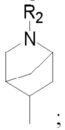
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 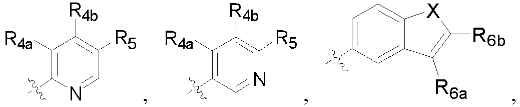 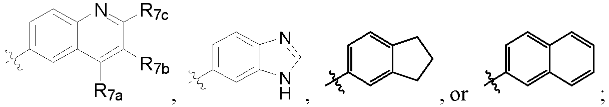
X is selected from the group consisting of NR_{6c}, O, or S;
R₄ₐ, R_{4b}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

In some embodiments of the compound of formula (VI):
R₁ₐ and R_{1b} are each independently selected from the group consisting of hydrogen or C₁₋₃ alkyl;
ring B is 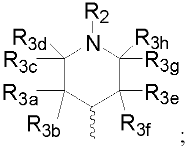
R₂ is independently selected from the group consisting of hydrogen, C₁₋₃ alkyl, or C₃₋₆ cycloalkyl;
R₃ₐ, R_{3b}, R_{3c}, R_{3d}, R₃ₑ, R_{3f}, R_{3g}, and R₃ₕ are each independently selected from the group consisting of hydrogen, halogen, or C₁₋₃ alkyl;
ring A is selected from the group consisting of 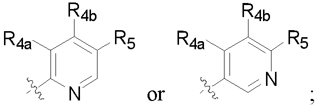
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl.

In some embodiments of the compound of formula (VI):
R₁ₐ and R_{1b} are each independently selected from the group consisting of hydrogen or methyl;
ring B is 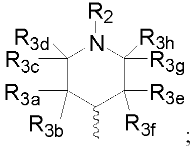
R₂ is independently selected from the group consisting of hydrogen, methyl, CD₃, ethyl, *n*-propyl, isopropyl, or cyclopropyl;
R₃ₐ, R_{3b}, R_{3c}, R_{3d}, R₃ₑ, R_{3f}, R_{3g}, and R₃ₕ are each independently selected from the group consisting of hydrogen, F, or methyl, or R₃ₐ and R_{3b}, R_{3c} and R_{3d}, R₃ₑ and R_{3f}, or R_{3g} and R₃ₕ, together with the carbon atom to which they are attached, form cyclopropyl;
ring A is selected from the group consisting of 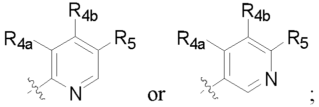
R₄ₐ and R_{4b} are each independently selected from the group consisting of hydrogen, F, Cl, or Br;
R₅ is -OR₅', wherein R₅' is selected from the group consisting of methyl, ethyl, *n*-propyl, isopropyl, *n*-butyl, isobutyl, *tert-*butyl*,* 2,2,2-trifluoroethyl, cyclopropyl, cyclobutyl, 3,3-difluorocyclobutyl, cyclopropylmethyl, or 2,2-dimethylcyclopropylmethyl.

In another aspect, the present disclosure provides the following compounds or pharmaceutically acceptable salts, stereoisomers or deuterides thereof:

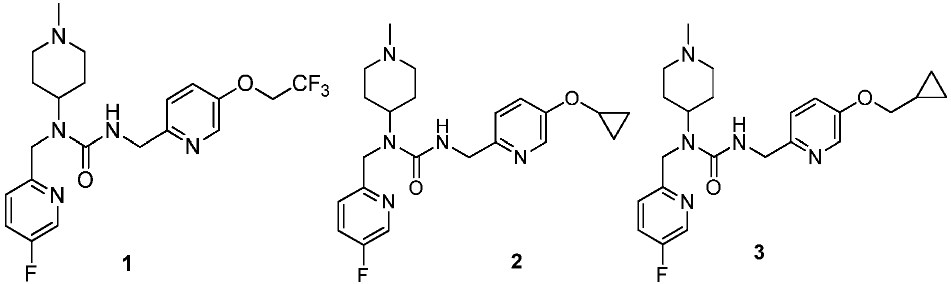

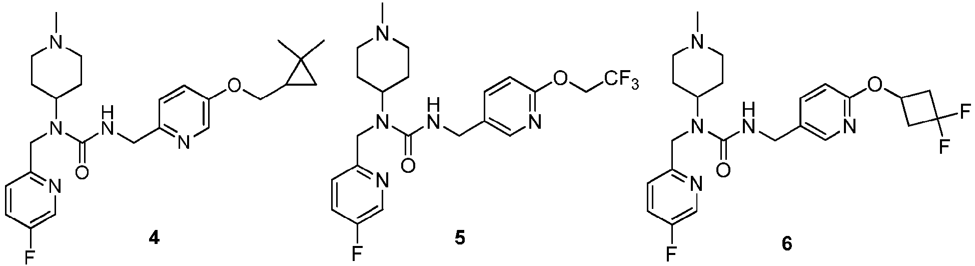

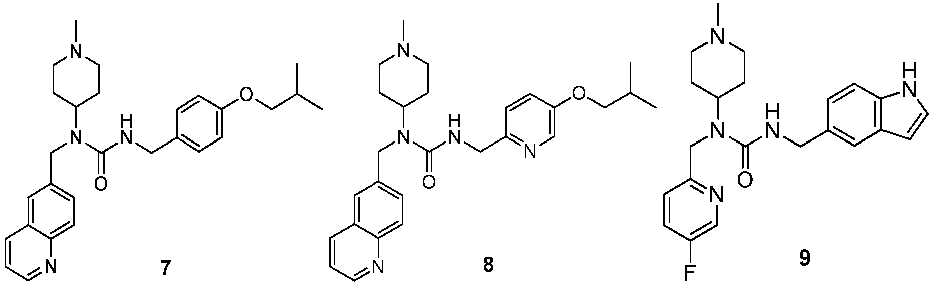

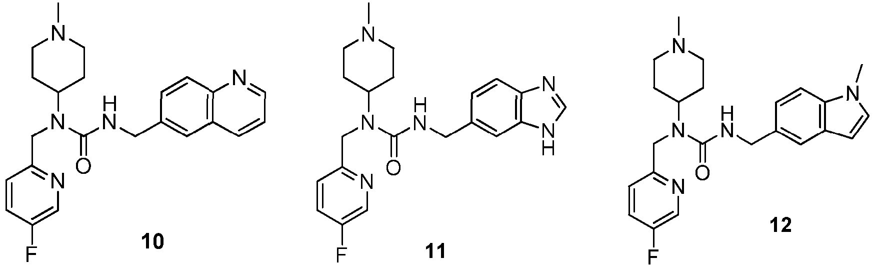

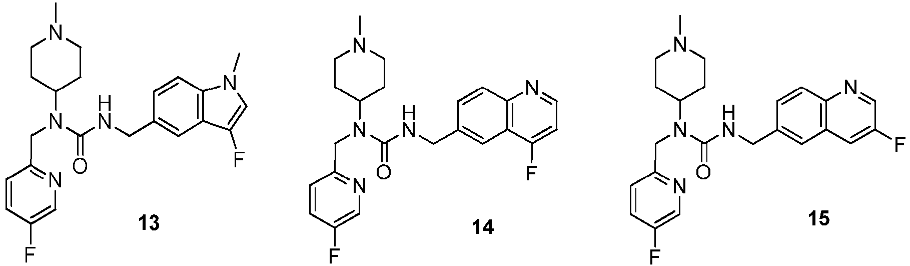

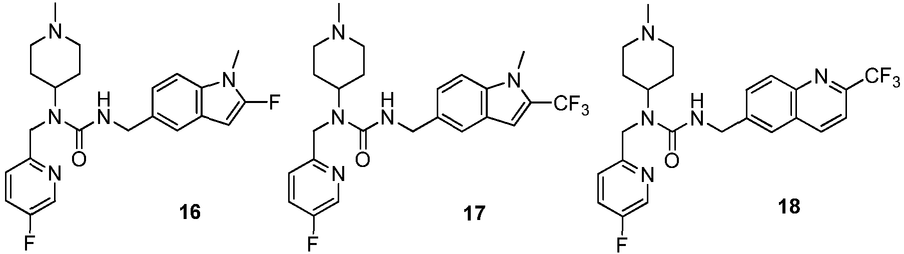

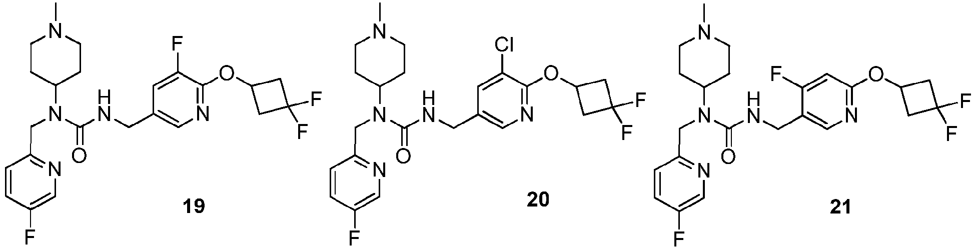

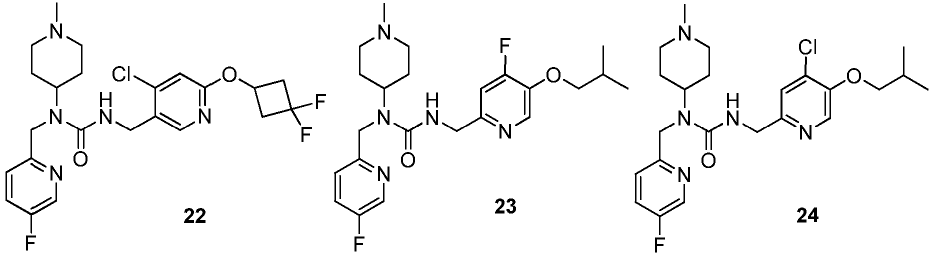

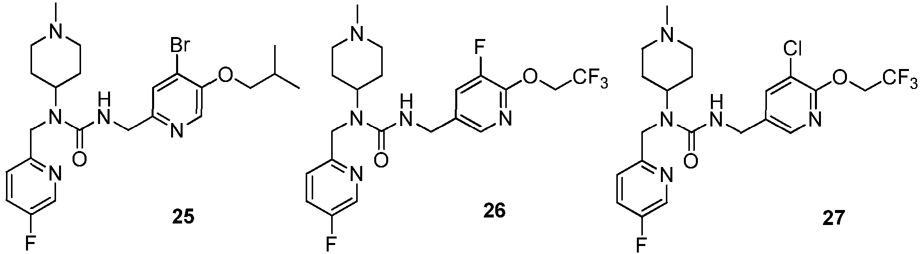

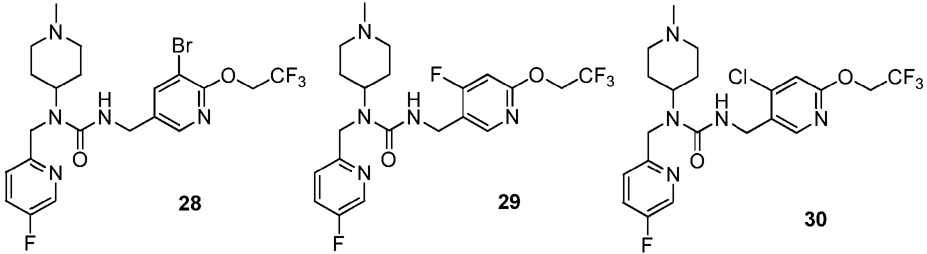

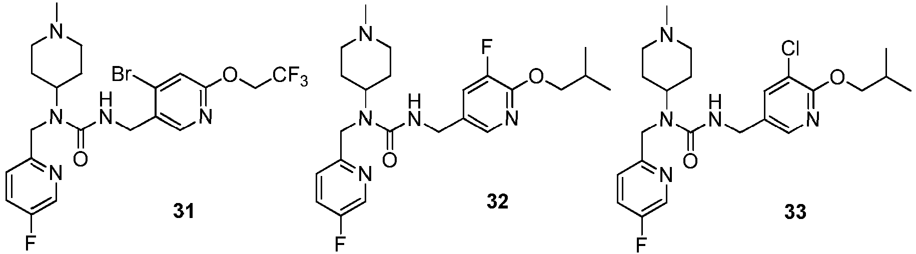

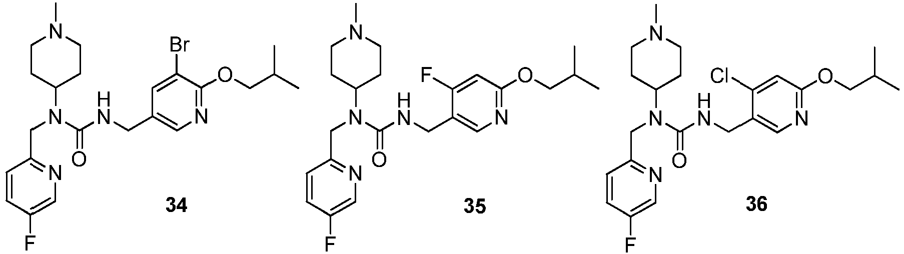

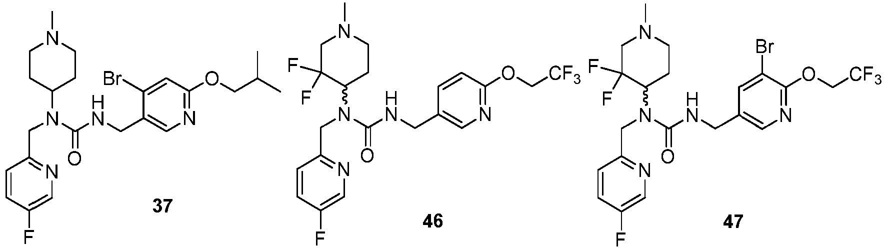

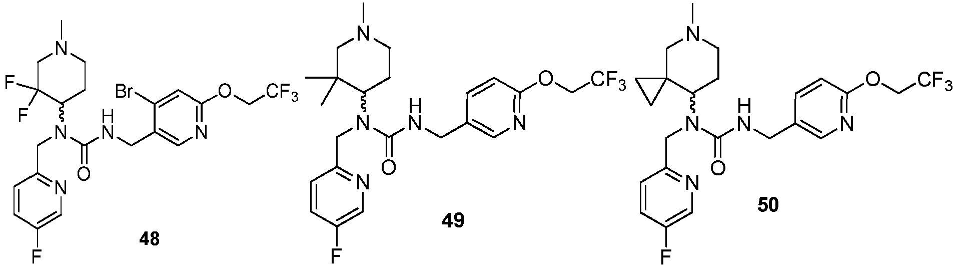

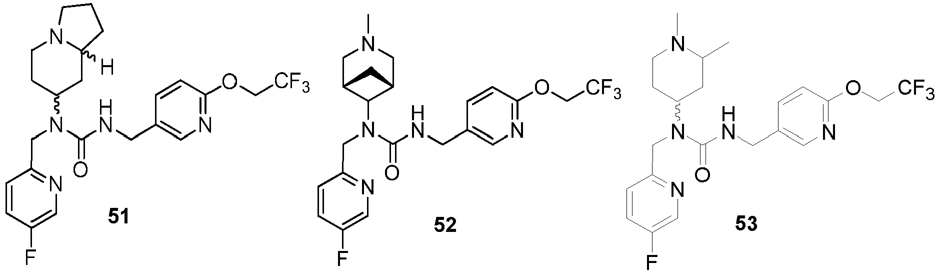

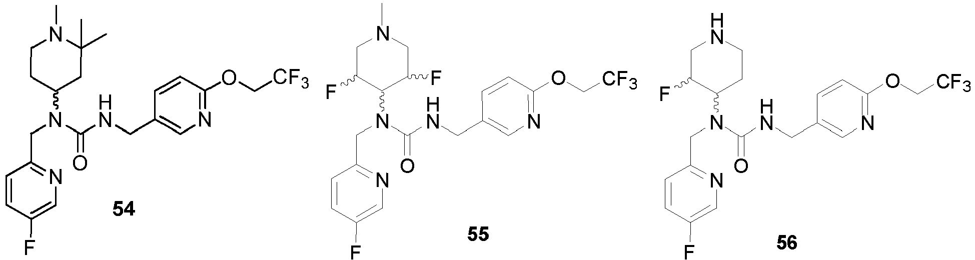

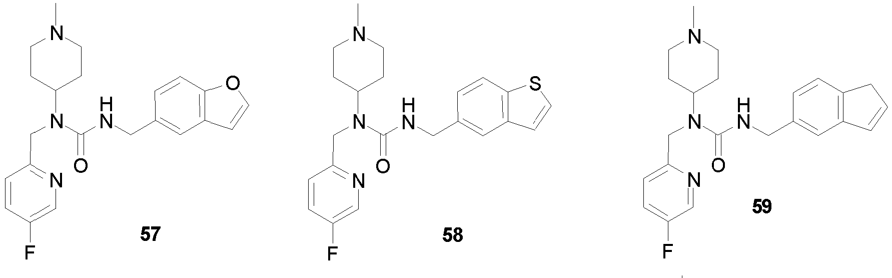

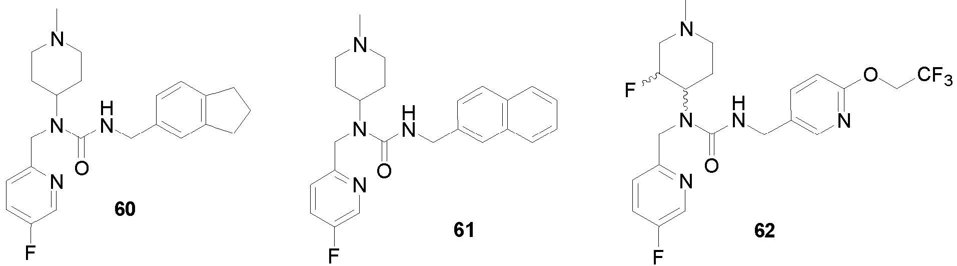

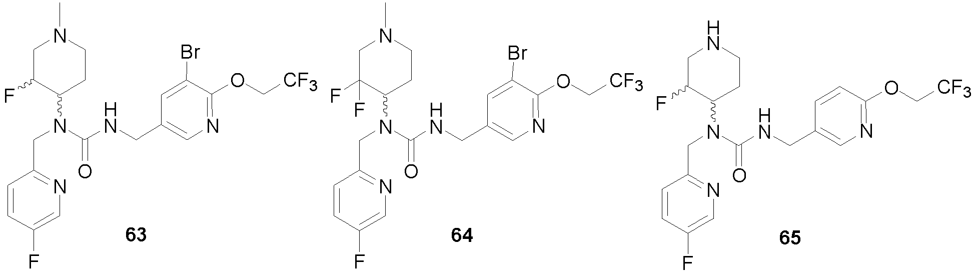

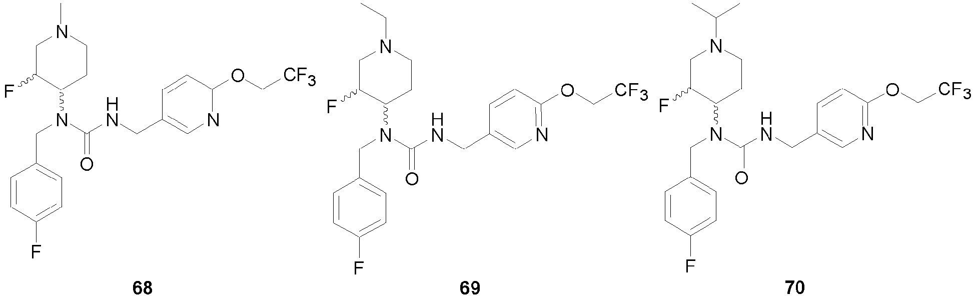

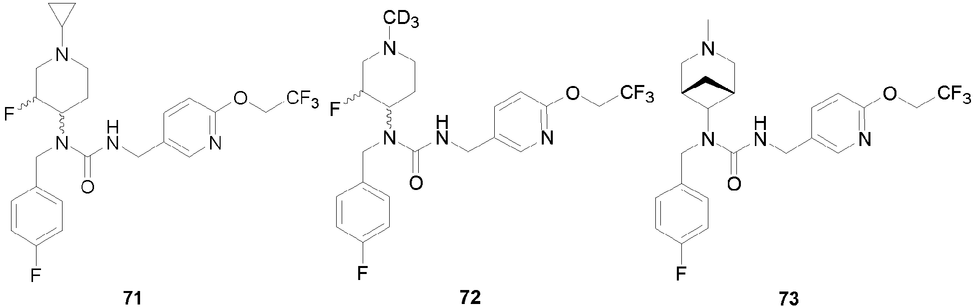

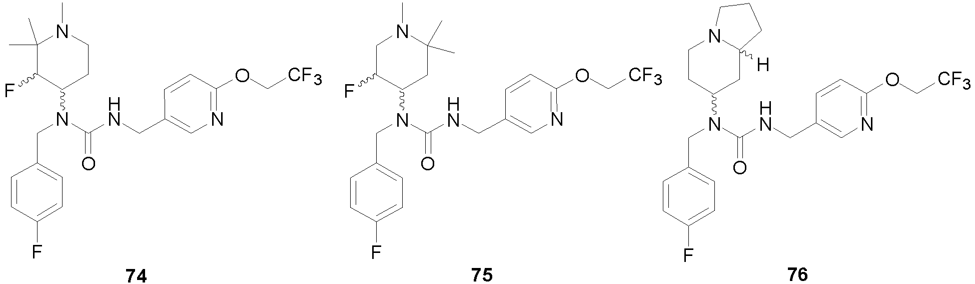

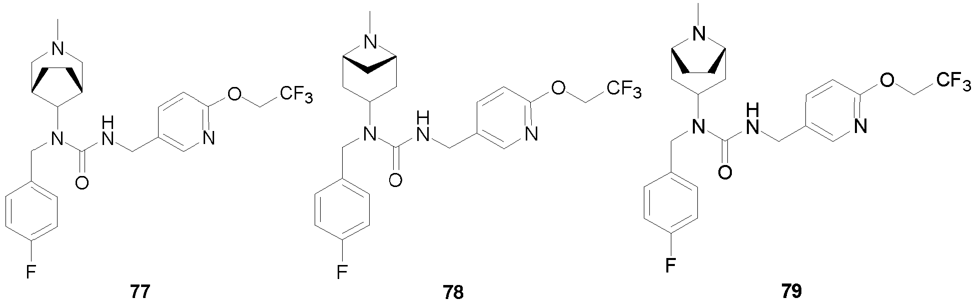

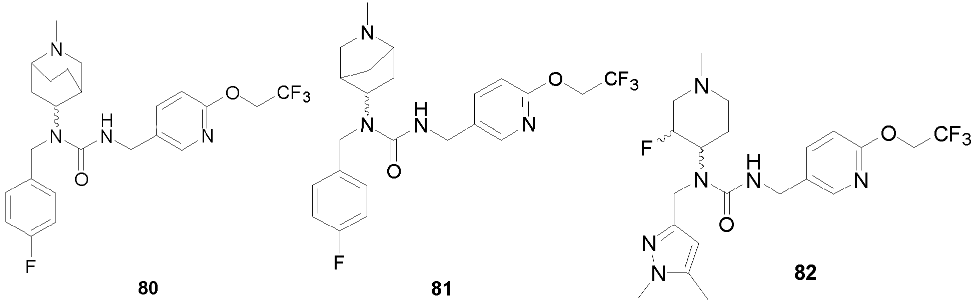

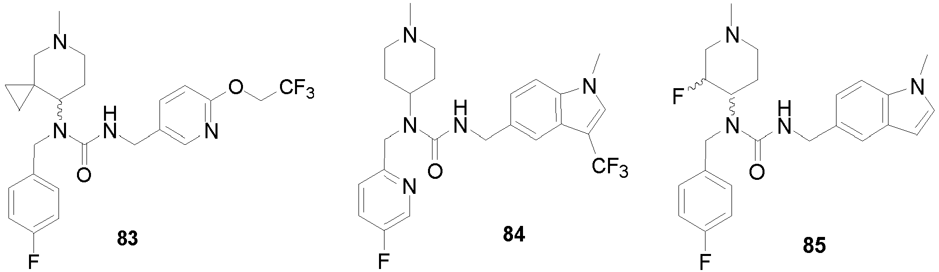

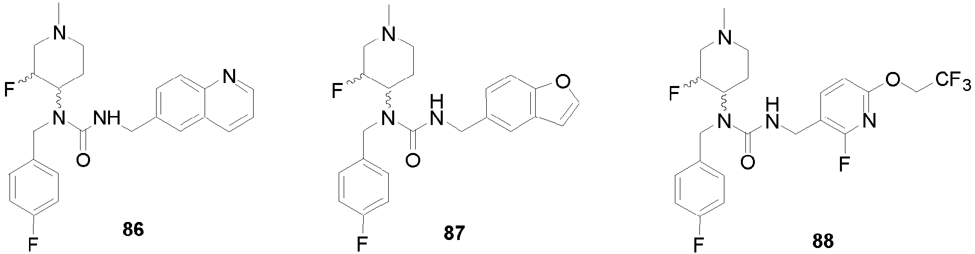

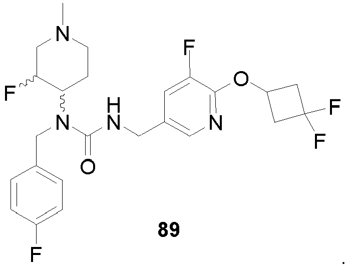

In another aspect, the present disclosure provides a compound or a pharmaceutically acceptable salt or deuteride thereof, wherein the compound is selected from the group consisting of the following:

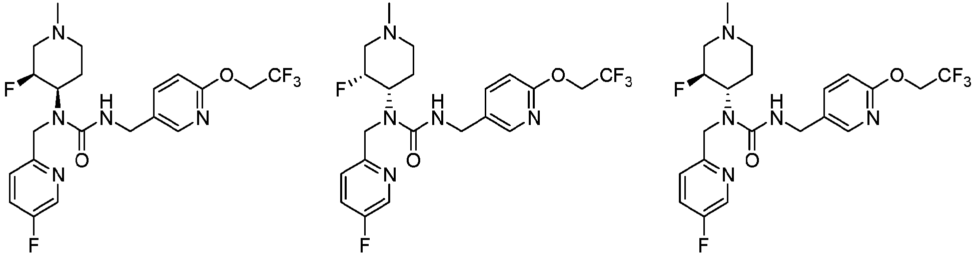

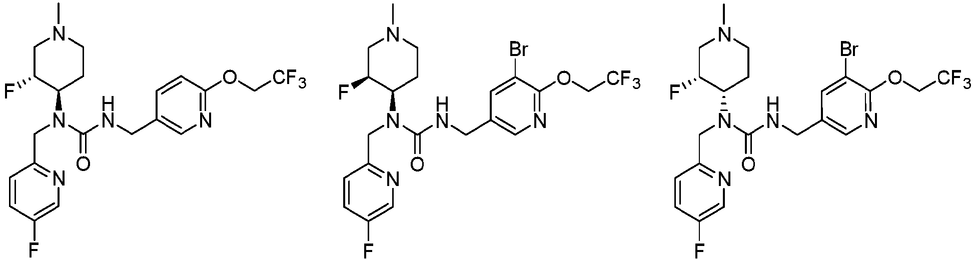

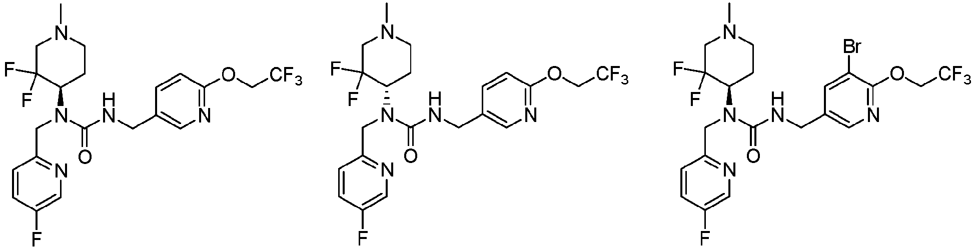

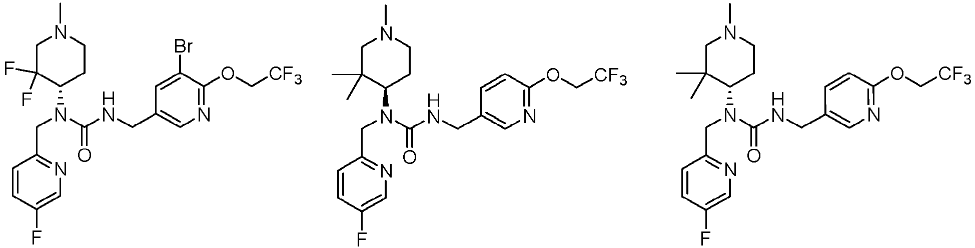

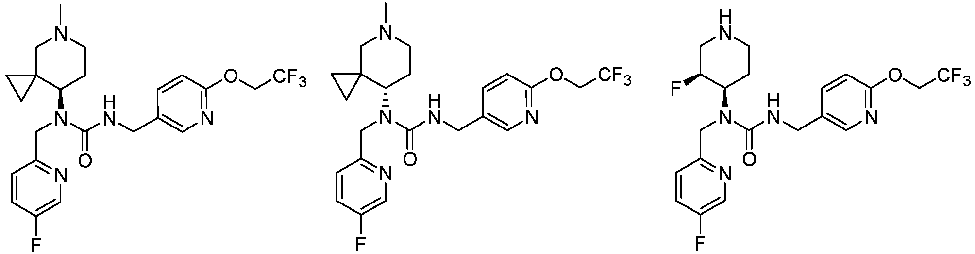

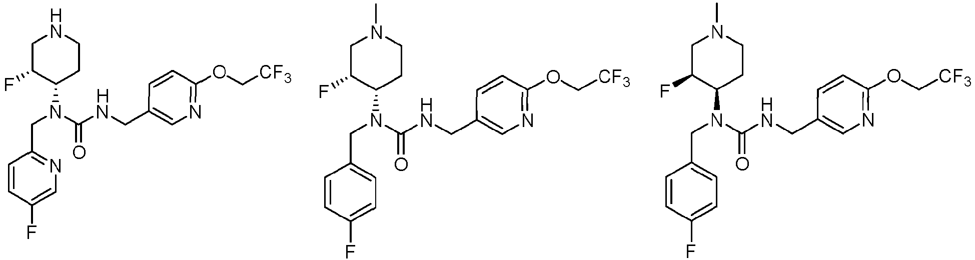

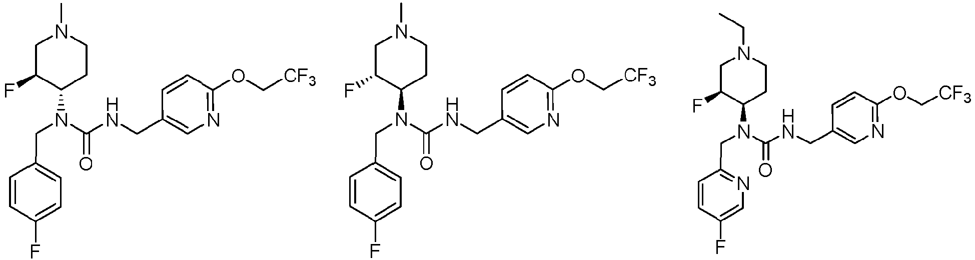

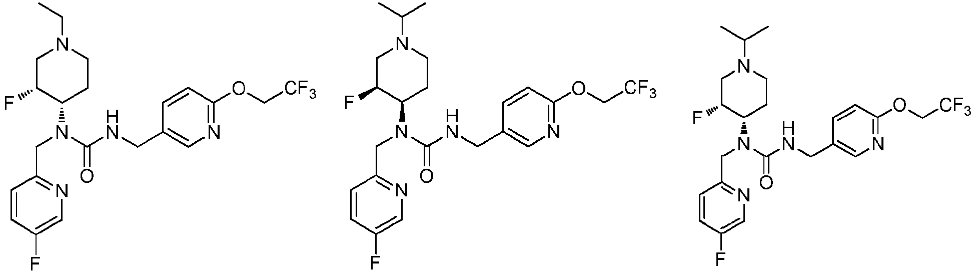

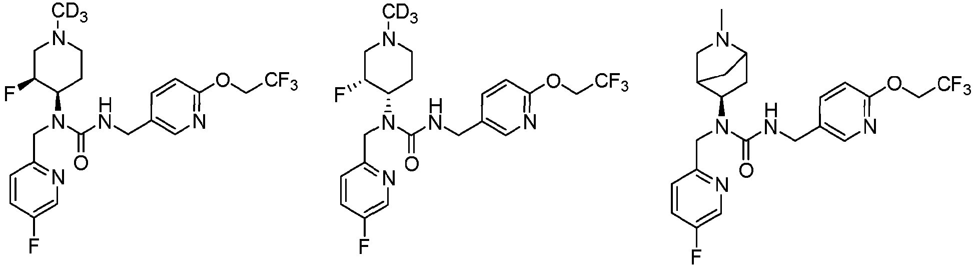

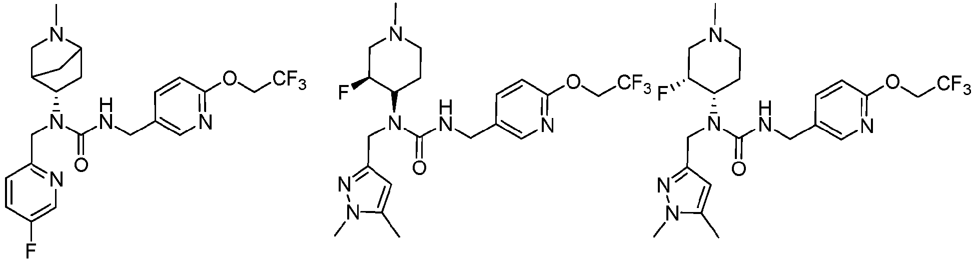

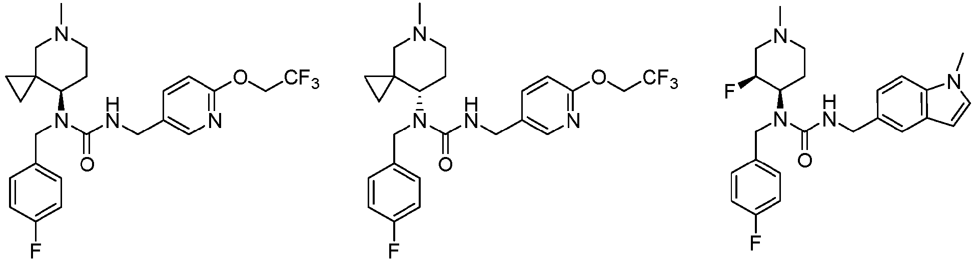

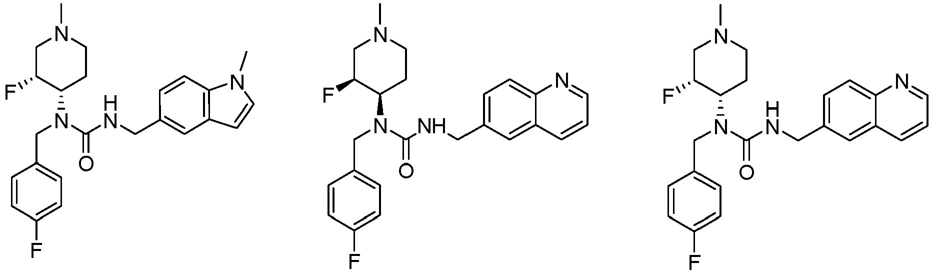

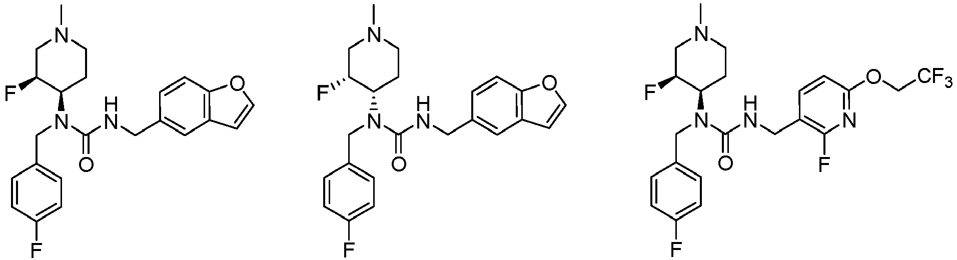

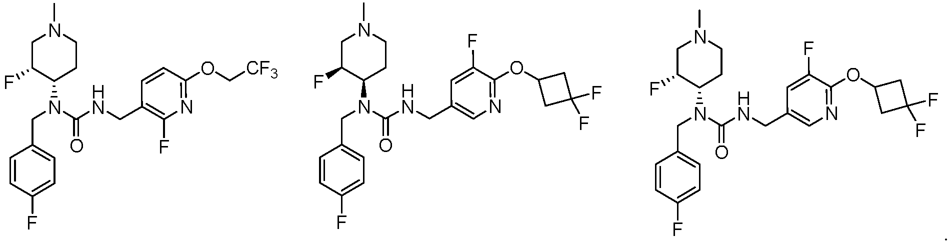

In one aspect, the present disclosure provides a pharmaceutical composition comprising a therapeutically effective amount of any one of the compounds or the stereoisomers or the pharmaceutically acceptable salts thereof described above, or a crystalline form of any one of the compounds described above, and a pharmaceutically acceptable carrier. The carrier includes conventional auxiliary ingredients in the art, such as fillers, binders, diluents, disintegrants, lubricants, colorants, flavoring agents, antioxidants, or wetting agents.

The pharmaceutical composition may be prepared into various pharmaceutically acceptable dosage forms, such as tablets, capsules, oral liquids, suspensions, granules, powders, microgranules, pills, mini-tablets, fast-dissolving films, nasal sprays, transdermal patches, injections, or various sustained-release and controlled-release preparations. The pharmaceutical compositions may be administered via oral, transmucosal, rectal, or parenteral routes (including intravascular, intravenous, intraperitoneal, subcutaneous, intramuscular, and intrasternal administration). The administration dose may be appropriately adjusted depending on the age, sex and type of disease of the patient.

For oral administration, the pharmaceutical composition may be in the form of, for example, a tablet, a capsule, a liquid capsule, a suspension, or a liquid. The pharmaceutical composition is preferably prepared in a dosage unit form containing a specific amount of the active ingredient. For example, the pharmaceutical composition may be provided in a tablet or capsule containing the active ingredient in an amount ranging from about 0.1 to 1000 mg, preferably from about 0.25 to 250 mg, and more preferably from about 0.5 to 100 mg. Suitable daily doses for humans or other mammals may vary widely depending on the condition of the patient and other factors, but can be determined using conventional methods.

In one aspect, the present disclosure provides use of any one of the compounds or the pharmaceutically acceptable salts or stereoisomers thereof described above in the preparation of a medicament for treating a 5-HT_{2A} receptor-related disease. The disease or symptom includes: schizophrenia, psychosis, schizoaffective disorder, manic disorder, psychotic depression, affective disorder, dementia, anxiety disorder, sleep disorder, appetite disorder, bipolar disorder, psychosis secondary to hypertension, migraine, hypertension, thrombosis, vasospasm, ischemia, motor tics, depression, major depressive disorder, anxiety, sleep disturbance and appetite disturbance, non-motor symptoms caused by Parkinson's disease (including delusion, hallucination, depression, anxiety, cognitive disorder, or sleep disorder), dementia-related mental diseases, negative symptoms of schizophrenia, Parkinson's disease, Huntington's chorea, Alzheimer's disease, spinocerebellar atrophy, Tourette's syndrome, Friedreich's ataxia, Machado-Joseph disease, Lewy body dementia, movement disorder, dystonia, myoclonus, tremor, progressive supranuclear palsy, and frontotemporal dementia; or other disease states and conditions that are apparent to those skilled in the art.

### Definitions and Description

Unless otherwise specified, the following terms and phrases used herein are intended to have the following meanings. A particular term or phrase, unless otherwise specifically defined, should not be considered indefinite or unclear, but construed according to its common meaning. When referring to a trade name, it is intended to refer to its corresponding commercial product or its active ingredient.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of the compound of the present disclosure, which is prepared from the compound having particular substituents disclosed herein and a relatively nontoxic acid or base. When the compound of the present disclosure contains a relatively acidic functional group, a base addition salt can be obtained by contacting the neutral form of such a compound with a sufficient amount of a base in a pure solution or a suitable inert solvent. Examples of the pharmaceutically acceptable acid addition salt include inorganic acid salts and organic acid salts, and also include salts of amino acids such as arginine, and salts of organic acids such as glucuronic acid. Certain specific compounds of the present disclosure contain both basic and acidic functional groups that allow the compounds to be converted into either base or acid addition salts.

The pharmaceutically acceptable salt of the present disclosure can be synthesized from the parent compound containing an acid radical or a basic group by conventional chemical methods. In general, the salt is prepared by reacting the free acid or base form of the compound with a stoichiometric amount of an appropriate base or acid in water or an organic solvent or a mixture thereof.

Certain compounds of the present disclosure may have asymmetric carbon atoms (optical centers) or double bonds. Racemates, diastereoisomers, geometric isomers, and individual isomers are all included within the scope of the present disclosure.

The compounds of the present disclosure may be in the form of a particular geometric isomer or stereoisomer. All such compounds are contemplated herein, including cis and trans isomers, (-)- and (+)-enantiomers, (R)- and (S)-enantiomers, diastereoisomers, (*D*)-isomers, (*L*)-isomers, and racemic mixtures and other mixtures thereof, such enantiomerically or diastereoisomerically enriched mixtures, all of which fall within the scope of the present disclosure. Additional asymmetric carbon atoms may be present in substituents such as alkyl. All these isomers and mixtures thereof are encompassed within the scope of the present disclosure.

Optically active (*R*)- and (*S*)-isomers as well as *D*- and *L*-isomers can be prepared by chiral synthesis or chiral reagents or other conventional techniques. If one enantiomer of a certain compound of the present disclosure is to be obtained, the desired pure enantiomer can be prepared by asymmetric synthesis or derivatization using a chiral auxiliary, where the resulting diastereoisomeric mixture is separated and the auxiliary group is cleaved. Alternatively, when the molecule contains a basic functional group (e.g., amino) or an acidic functional group (e.g., carboxyl), diastereoisomeric salts are formed with an appropriate optically active acid or base, followed by diastereoisomeric resolution through conventional methods well known in the art, and the pure enantiomer are subsequently recovered. Furthermore, separation of enantiomers and diastereoisomers is generally accomplished by chromatography using a chiral stationary phase, optionally in combination with chemical derivatization (e.g., carbamate formation from amines).

The term "pharmaceutically acceptable carrier" refers to any preparation or carrier medium capable of delivering an effective amount of the active substance of the present disclosure, without interfering with the biological activity of the active substance and without causing toxic and side effects on the host or patient; representative carriers include, but are not limited to: binders, fillers, lubricants, disintegrants, wetting agents, dispersing agents, solubilizers, suspending agents, and the like.

For a drug or pharmacologically active agent, the term "effective amount" or "therapeutically effective amount" refers to an amount of the drug or medicament that is sufficient to achieve the desired effect but is non-toxic. For oral dosage forms of the present disclosure, an "effective amount" of one active substance in the composition refers to the amount required to achieve the desired effect when the active substance is combined with another active substance in the composition. The determination of the effective amount varies from person to person. It depends on the age and general condition of the recipient, as well as the specific active substance used. The appropriate effective amount in a case may be determined by those skilled in the art in the light of routine tests.

The present disclosure is intended to include all isotopes of atoms present in the compounds of the present disclosure. Isotopes include those atoms having the same atomic number but different mass numbers. As a general and non-limiting example, isotopes of hydrogen include deuterium and tritium. Isotopes of carbon include ¹³C and ¹⁴C. Isotopically labeled compounds of the present disclosure can generally be prepared by conventional techniques known to those skilled in the art or by processes analogous to those described herein using an appropriate isotopically labeled reagent in place of the non-labeled reagent otherwise used.

The term "deuterated analog" refers to an analog resulting from replacement of one or more hydrogen atoms in a compound with a deuterium atom. The term "optional" or "optionally" means that the subsequently described event or circumstance may, but not necessarily, occur, and the description includes instances where the event or circumstance occurs and instances where it does not. For example, "optionally substituted with one or more deuterium atoms" means that the group may be unsubstituted with deuterium atoms or substituted with one or more deuterium atoms, i.e., it includes instances where the group is not deuterated, partially deuterated, and/or fully deuterated.

The term "substituted" means that any one or more hydrogen atoms on a specific atom are substituted with substituents which may include deuterium and hydrogen variants, as long as the valence of the specific atom is normal and the compound after substitution is stable. When the substituent is keto (i.e., =O), it means that two hydrogen atoms are substituted. Substitution with keto does not occur on aromatic groups.

When any variable (e.g., R) occurs more than once in the constitution or structure of a compound, the definition of the variable in each case is independent. Thus, for example, if a group is substituted with 0-2 R, the group may be optionally substituted with two R at most, and the definition of R in each case is independent. Furthermore, a combination of a substituent and/or a variant thereof is permissible only if the combination can result in a stable compound.

Unless otherwise specified, the term "alkyl" is used to refer to a linear or branched saturated hydrocarbon group, which may be monosubstituted (e.g., -CH₂F) or polysubstituted (e.g., -CF₃) and may be monovalent (e.g., methyl), divalent (e.g., methylene), or polyvalent (e.g., methine). For example, C₁-C₁₀ refers to 1 to 10 carbon atoms, where C₁₋₁₀ is selected from the group consisting of C₁, C₂, C₃, C₄, C₅, C₆, C₇, C₈, C₉, and C₁₀. Examples of the alkyl include methyl (Me), ethyl (Et), propyl (e.g., *n*-propyl and isopropyl), butyl (e.g., *n*-butyl, isobutyl, *s*-butyl, and *t-*butyl), pentyl (e.g., *n*-pentyl, isopentyl, neopentyl, and 1-ethylpropyl), hexyl (e.g., *n*-hexyl, isohexyl, 1,1-dimethylbutyl, 2,2-dimethylbutyl, 3,3-dimethylbutyl, and 2-ethylbutyl), heptyl, octyl, nonyl, decyl, and the like. Unless otherwise specified, the term "halo" or "halogen" by itself or as part of another substituent refers to a fluorine, chlorine, bromine, or iodine atom. The term "haloalkyl" is intended to include monohalogenated alkyl and polyhalogenated linear or branched haloalkyl. For example, the term "C₁₋₁₀ haloalkyl" is intended to include, but not be limited to, fluoromethyl, difluoromethyl, trichloromethyl, trifluoromethyl, 2-fluoroethyl, 2,2-difluoroethyl, 2,2,2-trifluoroethyl, tetrafluoroethyl, pentafluoroethyl, 3-fluoropropyl, 3,3-difluoropropyl, 2,2'-difluoroisopropyl, 3,3,3-trifluoropropyl, 4-fluorobutyl, 4,4-difluorobutyl, 4,4,4-trifluorobutyl, 2-fluoro-2-methylpropyl, 5,5,5-trifluoropentyl, and 6,6,6-trifluorohexyl.

Unless otherwise specified, cycloalkyl includes any stable cyclic or polycyclic hydrocarbon group with any carbon atom being saturated, which may be monosubstituted or polysubstituted and may be monovalent, divalent, or polyvalent. Examples of such cycloalkyl groups include, but are not limited to, cyclopropyl, cyclobutyl, cyclopentyl, cyclohexyl, cycloheptyl, cyclooctyl, norbornyl, [2.2.2]bicyclooctane, [4.4.0]bicyclodecane, and the like. The cycloalkyl may be optionally further substituted with halogen or C₁-C₃ alkyl.

Unless otherwise specified, "cycloalkylalkyl" refers to C₃₋₆ cycloalkyl-C₁₋₃ alkyl-, which may be substituted or unsubstituted, and non-limiting examples thereof include cyclopropylmethyl, cyclobutylmethyl, cyclopentylmethyl, cyclopropylethyl, cyclobutylethyl, cyclopentylethyl, cyclopropylpropyl, cyclobutylpropyl, cyclopentylpropyl, and the like. The cycloalkylalkyl may be optionally further substituted with halogen or C₁-C₃ alkyl.

Compounds are named either manually or by ChemDraw^{®} software, and supplier's catalog names are given for commercially available compounds.

### BRIEF DESCRIPTION OF THE DRAWING

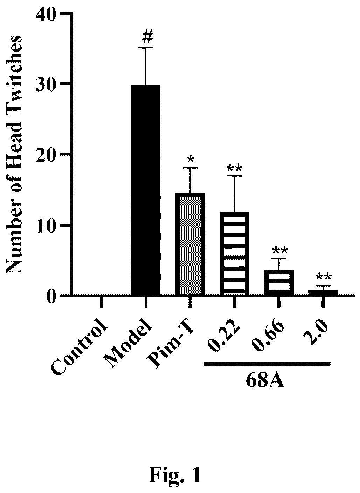

FIG. 1: Number of head twitches in SD rats 1.0-1.5 h after single intragastric administration (where # indicates *P <* 0.05, as compared to the Control group; * indicates *P* < 0.05 and ** indicates *P* < 0.01, as compared to the Model group).

### DETAILED DESCRIPTION

The present disclosure will be further illustrated with reference to the following specific examples and test examples, but the scope of the present disclosure is not limited in any way.

### Example 1

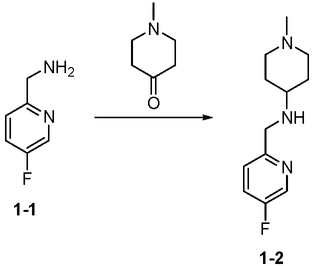

Under a nitrogen atmosphere and an ice-water bath, 2-(aminomethyl)-5-fluoropyridine (504 mg, 4.0 mmol) was dissolved in 10 mL of methanol, and N-methyl-4-piperidone (452 mg, 4.0 mmol) and sodium triacetoxyborohydride (933 mg, 4.4 mmol) were added. The mixture was warmed to room temperature and allowed to react for 15 h. An aqueous sodium bicarbonate solution was added to adjust the pH to alkaline. The organic phase was concentrated and then extracted with dichloromethane (10 mL × 3). The organic phases were combined, dried over anhydrous sodium sulfate, filtered, and concentrated under reduced pressure to obtain compound **1-2** (538 mg).

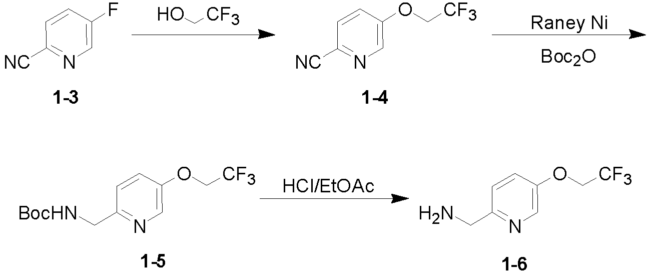

Dimethyl sulfoxide (16 mL), compound **1-3** (4.00 g, 1.00 *eq),* compound trifluoroethanol (9.83 g, 3.00 *eq),* and cesium carbonate (16.01 g, 1.50 eq) were sequentially added to a 100 mL three-necked flask. The mixture was heated to 105 °C and incubated for 12 h. Water (200 mL) was added to the reaction solution, and the mixture was extracted with ethyl acetate (200 mL × 2). The organic phases were combined, washed with 200 mL of saturated brine, dried, and concentrated to dryness to obtain a crude product. The crude product was purified by column chromatography to obtain compound **1-4** (orange oily substance, 5.6 g).

Tetrahydrofuran (160 mL), compound **1-4** (4.0 g, 1.00 *eq*), di-*tert*-butyl dicarbonate (4.32 g, 1.0 eq), and Raney Ni (1.7 g, 1.0 eq) were sequentially added to in a 500 mL hydrogenation flask, and the mixture was allowed to react at 70 °C under 50 Psi for 12 h, then filtered, and concentrated to dryness by rotary evaporation to obtain a crude product. The crude product was purified by preparative chromatography to obtain compound **1-5** (white solid, 2.37 g).

Ethyl acetate (5 mL), compound **1-5** (1.00 g, 1.00 *eq*), and HCl/EtOAc (5 mL) were sequentially added to a 100 mL single-necked flask, and the mixture was incubated with stirring at 25 °C for 17 h. The reaction solution was directly concentrated to dryness by rotary evaporation to obtain a crude product of compound **1-6** (light pink solid, 903 mg).

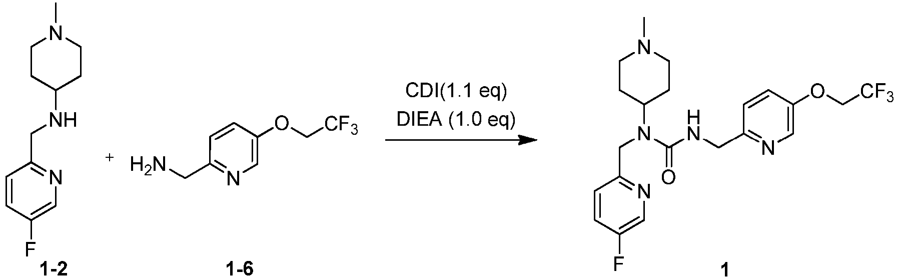

Tetrahydrofuran (2 mL), compound 1-6 (200 mg, 1.00 *eq*), and diisopropylethylamine (DIEA) (125 mg, 1.00 eq) were sequentially added to a 10 mL single-necked flask, and the mixture was stirred under a nitrogen atmosphere for 15 min and then cooled to 0 °C with an ice-water bath. Carbonyldiimidazole (CDI) (173 mg, 1.10 *eq*) was added to the reaction solution, and the mixture was allowed to react at 0 °C for 1 h. Compound **1-2** (216 mg, 1.0 *eq*) was dissolved in 1 mL of tetrahydrofuran, and the resulting solution was added to the reaction solution. The resulting mixture was incubated with stirring at 25 °C for 12 h. The reaction solution was then concentrated to dryness to obtain a crude product. The crude product was purified by thin-layer chromatography and preparative chromatography to obtain **compound 1** (yellow oily substance, 160 mg). ¹H NMR (400 MHz, CDCl₃) *δ* 8.35 (d, *J =* 2.5 Hz, 1H), 8.24 (d, *J* = 2.8 Hz, 1H), 7.41 - 7.32 (m, 2H), 7.24 - 7.20 (m, 1H), 7.00 (br d, *J* = 2.1 Hz, 1H), 4.50 (d, *J =* 5.4 Hz, 2H), 4.47 (s, 2H), 4.39 (q, *J* = 8.0 Hz, 2H), 4.27 (br s, 1H), 2.94 (br d, *J* = 11.6 Hz, 2H), 2.32 (s, 3H), 2.20 - 2.08 (m, 2H), 1.82 (br dd, *J* = 3.4, 12.1 Hz, 2H), 1.73 - 1.61 (m, 2H). MS m/z(ESI): 456.2 [M+1].

### Example 2

Compounds 2-4, 7-8, 11, 77, and 79 were obtained using a synthesis method similar to that in Example 1. The characterization data for compounds 2-4, 7-8, 11, 77, and 79 are shown in the table below:

| Compound No./Structure | ¹H NMR | MS |
|---|---|---|
| 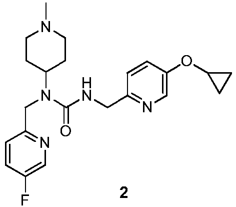 | ¹H NMR (400 MHz, CDCl₃) *δ* 8.35 (d, *J* = 2.3 Hz, 1H), 8.28 (d, *J =* 2.8 Hz, 1H), 7.37 - 7.34 (m, 1H), 7.33 - 7.29 (m, 1H), 7.22 - 7.19 (m, 1H), 6.87 (br s, 1H), 4.49 (s, 1H), 4.47 (d, *J =* 1.6 Hz, 2H), 4.28 (tt, *J =* 4.1, 12.0 Hz, 1H), 3.77 (tt, *J =* 3.1, 5.9 Hz, 1H), 2.94 (br d, *J =* 11.5 Hz, 2H), 2.32 (s, 3H), 2.14 (br t, *J =* 11.0 Hz, 2H), 1.88 - 1.76 (m, 2H), 1.74 - 1.65 (m, 2H), 0.85 - 0.75 (m, 4H). | MS m/z(ESI): 414.2[M+1]. |
| 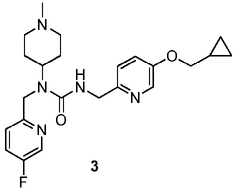 | ¹H NMR (400 MHz, DMSO-*d₆*) *δ* 8.49 (d, *J =* 3.0 Hz, 1H), 8.16 (d*, J* = 2.8 Hz, 1H), 7.69 (dt, *J* = 2.9, 8.8 Hz, 1H), 7.40 - 7.32 (m, 2H), 7.20 - 7.13 (m, 2H), 4.49 (s, 2H), 4.28 (d, *J =* 5.6 Hz, 2H), 3.99 - 3.90 (m, 1H), 3.87 (d, *J =* 7.0 Hz, 2H), 2.75 (br d, *J =* 11.3 Hz, 2H), 2.13 (s, 3H), 1.93 (br t*, J =* 10.8 Hz, 2H), 1.62 - 1.43 (m, 4H), 1.27 - 1.16 (m, 1H), 0.60 - 0.55 (m, 2H), 0.35 - 0.30 (m, 2H). | MS m/z(ESI): 428.2 [M+1]. |
| 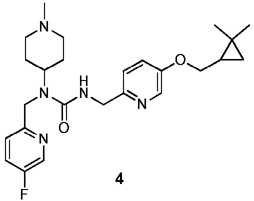 | ¹HNMR (400 MHz, CDCl₃) *δ* 8.34 (s, 1H), 8.19 (d, *J =* 2.6 Hz, 1H), 7.42 - 7.33 (m, 2H), 7.23 - 7.09 (m, 3H), 4.50 - 4.45 (m, 4H), 4.36 (ddd, *J =* 3.9, 8.4, 12.1 Hz, 1H), 4.10 (dd, *J* = 6.4, 10.2 Hz, 1H), 3.84 (dd, *J* = 8.6, 10.1 Hz, 1H), 3.12 (br d, *J =* 11.5 Hz, 2H), 2.45 (s, 3H), 2.34 (br t, *J =* 11.4 Hz, 2H), 1.97 (dq, *J =* 3.0, 12.3 Hz, 2H), 1.72 (br d, *J* = 10.9 Hz, 2H), 1.12 (d*, J =* 1.4 Hz, 6H), 1.06 (ddd, *J =* 2.4, 5.7, 8.3 Hz, 1H), 0.61 (dd, *J =* 4.6, 8.7 Hz, 1H), 0.26 (t, *J =* 4.9 Hz, 1H). | MS m/z(ESI): 456.3[M+1]. |
| 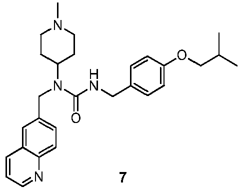 | ¹H NMR (400 MHz, CDCl₃) *δ* 8.89 (br s, 1H), 8.12 - 7.96 (m, 2H), 7.65 - 7.51 (m, 2H), 7.40 (br s, 1H), 6.95 (br d, *J* = 6.9 Hz, 2H), 6.67 (br d, *J =* 6.9 Hz, 2H), 4.59 - 4.46 (m, 3H), 4.65 (br s, 1H), 4.28 (br s, 2H), 3.62 (br d, *J =* 5.1 Hz, 2H), 2.99 (br d, *J =* 8.1 Hz, 3H), 2.35 (br s, 3H), 2.25 (br s, 2H), 2.09 - 1.98 (m, 1H), 1.88 (br d, *J =* 10.5 Hz, 1H), 1.81 (br s, 2H), 1.00 (br d, *J =* 5.5 Hz, 6H). | MS m/z(ESI): 461.3[M+1]. |
| 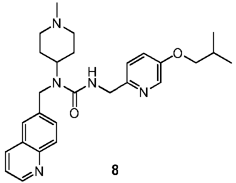 | ¹H NMR (400 MHz, CDCl₃) *δ* 8.89 (dd, *J =* 1.6, 4.3 Hz, 1H), 8.06 (d*, J =* 8.4 Hz, 2H), 7.86 - 7.82 (m, 1H), 7.67 (s, 1H), 7.62 (dd, *J =* 1.9, 8.8 Hz, 1H), 7.39 (dd, *J* = 4.3, 8.3 Hz, 1H), 7.07 - 7.03 (m, 2H), 5.70 (s, 1H), 4.64 (s, 2H), 4.58 (br s, 1H), 4.40 (d, *J =* 4.9 Hz, 2H), 3.65 (d, *J =* 6.5 Hz, 2H), 3.26 (br d, *J =* 11.8 Hz, 2H), 2.55 (s, 3H), 2.53 (br s, 2H), 2.12 - 1.98 (m, 3H), 1.86 (br d, *J =* 10.8 Hz, 2H), 1.01 (s, 3H), 0.99 (s, 3H). | MS m/z(ESI): 462.3[M+1]. |
| 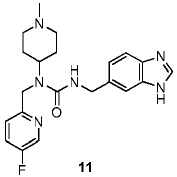 | ¹H NMR (400 MHz, DMSO-*d₆*) *δ* 12.52 - 12.18 (m, 1H), 8.47 (d, *J* = 2.9 Hz, 1H), 8.16 (s, 1H), 7.71 - 7.61 (m, 1H), 7.54 - 7.40 (m, 2H), 7.33 (dd, *J* = 4.4, 8.7 Hz, 1H), 7.16 (br t, *J =* 5.2 Hz, 1H), 7.08 (br d, *J =* 8.1 Hz, 1H), 4.48 (s, 2H), 4.37 (br d, *J* = 5.5 Hz, 2H), 3.95 (br t, *J* = 11.6 Hz, 1H), 2.72 (br d, *J =* 10.8 Hz, 3H), 2.14 (s, 1H), 2.10 (s, 3H), 1.90 (br t, *J =* 10.8 Hz, 2H), 1.55 (dq*, J =* 3.3, 11.9 Hz, 2H), 1.49 - 1.42 (m, 2H), 1.37 - 1.21 (m, 1H). | MS m/z(ESI): 397.2 [M+1]. |
| 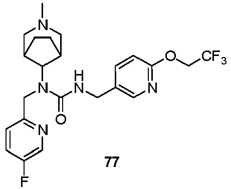 | ¹H NMR (400 MHz, CDCl₃) *δ* 8.39 (d, J=2.88 Hz, 1 H), 8.06 (d, J=2.13 Hz, 1 H), 7.61 (dd, J=8.50, 2.38 Hz, 1 H), 7.38 (td, J=8.25, 2.88 Hz, 1 H), 7.20 (dd, J=8.57, 4.31 Hz, 2 H), 6.81 (d, J=8.38 Hz, 1 H), 4.70 - 4.80 (m, 3 H), 4.54 (s, 2 H), 4.39 (d, J=5.63 Hz, 2 H), 3.42 (t, J=4.13 Hz, 1 H), 2.69 (br s, 2 H), 2.34 (br d, J=6.38 Hz, 2 H), 2.03 (br d, J=10.88 Hz, 2 H), 1.66 - 1.82 (m, 6 H). | MS(ESI) m/z : 482.2 [M+1]. |
| 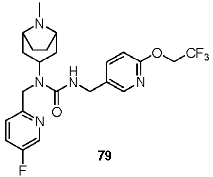 | ¹H NMR (400 MHz, CDCl₃) *δ* 8.27 (t*, J* = 1.6 Hz, 1H), 8.12 - 8.01 (m, 1H), 7.61 (dd, *J =* 2.4, 8.5 Hz, 1H), 7.40 (dd, *J =* 1.9, 6.5 Hz, 2H), 6.82 (d, *J =* 8.4 Hz, 1H), 6.71 (br s, 1H), 4.75 (q, *J =* 8.6 Hz, 2H), 4.39 (s, 2H), 4.35 (d*, J =* 5.6 Hz, 2H), 4.29 - 4.16 (m, 1H), 3.47 (br s, 2H), 2.47 (br s, 1H), 2.43 (br s, 3H), 2.39 (br s, 1H), 2.25 - 2.16 (m, 2H), 1.95 - 1.78 (m, 4H). | MS(ESI) m/z : 482.2 [M+1]. |

### Example 3

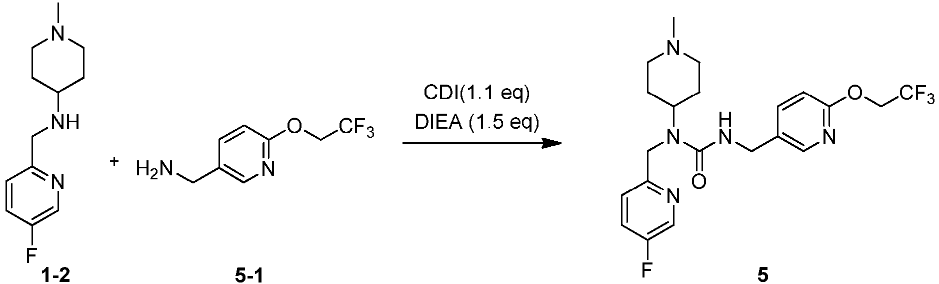

Tetrahydrofuran (3 mL), compound 5-1 (300 mg, 1.00 eq), and diisopropylethylamine (282.10 mg, 1.50 eq) were added to a 100 mL single-necked flask, and the mixture was cooled to 0 °C and stirred under a nitrogen atmosphere for 15 min. Carbonyldiimidazole (259.55 mg, 1.10 eq) was added to the reaction solution, and the mixture was allowed to react at 0 °C for 1 h. A solution of compound **1-2** (324.92 mg, 1.00 eq) and 2 mL of tetrahydrofuran were added to the reaction solution, and the mixture was allowed to react at 25 °C for 12 h, then filtered, and concentrated to dryness by rotary evaporation to obtain a crude product. The crude product was purified by preparative chromatography to obtain **compound 5** (yellow solid, 440 mg). ¹H NMR (400 MHz, DMSO-*d₆*) *δ* 8.47 (d, *J =* 2.9 Hz, 1H), 8.04 (d, *J =* 1.9 Hz, 1H), 7.69 - 7.62 (m, 2H), 7.29 (dd, *J =* 4.4, 8.8 Hz, 1H), 7.15 (t, *J =* 5.6 Hz, 1H), 6.93 (d, *J =* 8.5 Hz, 1H), 4.96 (q, *J =* 9.1 Hz, 2H), 4.46 (s, 2H), 4.21 (d*, J =* 5.5 Hz, 2H), 3.97 (br t*, J* = 11.4 Hz, 1H), 2.88 (br d, *J* = 10.6 Hz, 2H), 2.26 (s, 3H), 2.16 (br t, *J =* 9.9 Hz, 2H), 1.65 - 1.54 (m, 2H), 1.53 - 1.47 (m, 2H). MS m/z(ESI): 456.2 [M+1].

### Example 4

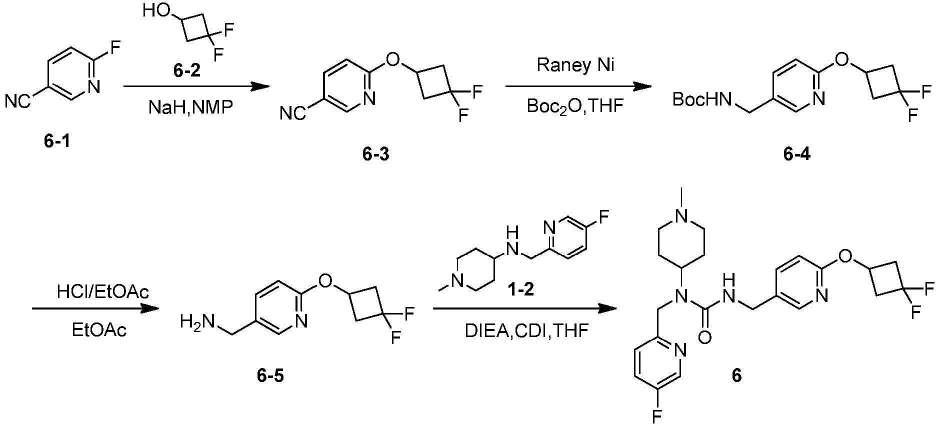

Methylpyrrolidone (25 mL) and compound 6-2 (2.55 g, 1.20 eq) were added to a 100 mL three-necked flask, and the mixture was cooled to 0 °C. Sodium hydride (1.18 g, 1.50 eq) was added to the reaction solution in portions, and the mixture was stirred at 0 °C for 0.5 h. Compound **6-1** (2.40 g, 1.00 eq) was dissolved in 20 mL of methylpyrrolidone, and the resulting solution was added dropwise to the reaction solution. The resulting mixture was stirred at 25 °C for 12 h. Water (100 mL) was added to the reaction solution, and the mixture was extracted with ethyl acetate (200 mL × 3). The organic phase was dried, filtered, and concentrated to dryness by rotary evaporation to obtain a crude product. The crude product was purified by column chromatography to obtain compound **6-3** (white solid, 1.20 g).

Tetrahydrofuran (12 mL), compound 6-3 (1.20 g, 1.00 eq), di-*tert*-butyl dicarbonate (1.25 g, 1.00 eq), and Raney Ni (1.20 g, 2.45 eq) were added to a 250 mL hydrogenation flask, and the mixture was allowed to react at 70 °C under 50 Psi for 24 h, then filtered, and concentrated to dryness by rotary evaporation to obtain a crude product. The crude product was purified by column chromatography to obtain compound **6-4** (white solid, 720 mg).

Ethyl acetate (2 mL) and compound **6-4** (720 mg, 1.00 eq) were added to a 100 mL single-necked flask, and a solution of hydrochloric acid in ethyl acetate (8 mL, 4 M) was slowly added dropwise to the reaction solution. The mixture was allowed to react at 25 °C for 12 h and then concentrated to dryness by rotary evaporation. The residue was dissolved in 10 mL of dichloromethane, and the resulting solution was adjusted to pH 8 with a saturated aqueous sodium bicarbonate solution and then extracted with dichloromethane (10 mL × 3). The organic phase was dried, filtered, and concentrated to dryness by rotary evaporation to obtain compound **6-5** (440 mg).

Tetrahydrofuran (4 mL), compound **6-5** (390 mg, 1.00 eq), and diisopropylethylamine (352.96 mg, 1.50 eq) were added to a 100 mL single-necked flask, and the mixture was cooled to 0 °C and stirred under a nitrogen atmosphere for 15 min. Carbonyldiimidazole (324.73 mg, 1.10 eq) was added to the reaction solution, and the mixture was allowed to react at 0 °C for 1 h. A solution of compound **1-2** (406.53 mg, 1.00 eq) and 2 mL of tetrahydrofuran were added to the reaction solution, and the mixture was allowed to react at 25 °C for 12 h, then filtered, and concentrated to dryness by rotary evaporation to obtain a crude product. The crude product was purified by preparative chromatography to obtain **compound 6** (yellow gelatinous substance, 460 mg). ¹H NMR (400 MHz, DMSO-*d₆*) *δ* 8.46 (d, *J =* 2.9 Hz, 1H), 8.00 (d, *J =* 2.1 Hz, 1H), 7.69 - 7.59 (m, 2H), 7.30 (dd*, J =* 4.5, 8.8 Hz, 1H), 7.11 (t, *J* = 5.6 Hz, 1H), 6.81 (d, *J* = 8.5 Hz, 1H), 5.13 - 5.02 (m, 1H), 4.46 (s, 2H), 4.19 (d, *J =* 5.5 Hz, 2H), 3.99 - 3.89 (m, 1H), 3.20 - 3.07 (m, 2H), 2.81 (br d, *J =* 11.3 Hz, 2H), 2.74 - 2.61 (m, 2H), 2.19 (s, 3H), 2.04 (br t, *J =* 10.9 Hz, 2H), 1.57 (dq, *J* = 3.4, 12.0 Hz, 2H), 1.50 - 1.43 (m, 2H). MS m/z(ESI): 464.2 [M+1].

### Example 5

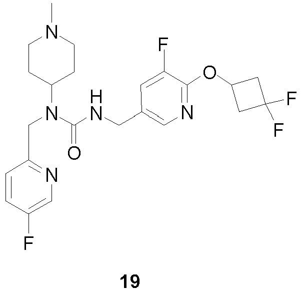

**Compound 19** was obtained using a synthesis method similar to that in Example 4. ¹H NMR (400 MHz, CDCl₃) *δ* 8.30 (d, *J =* 2.4 Hz, 1H), 7.82 (d, *J =* 2.0 Hz, 1H), 7.41 - 7.33 (m, 4H), 5.17 - 5.14 (m, 1H), 4.38 (s, 2H), 4.35 - 4.34 (m, 2H), 4.32 - 4.28 (m, 1H), 3.14 - 3.12 (m, 2H), 2.99 - 2.96 (m, 2H), 2.79 - 2.75 (m, 2H), 2.52 (br s, 1H), 2.35 (s, 3H), 2.18 - 2.15 (m, 2H), 1.91 - 1.87 (m, 2H), 1.68 - 1.65 (m, 2H). MS(ESI) m/z : 482.2 [M+1].

### Example 6

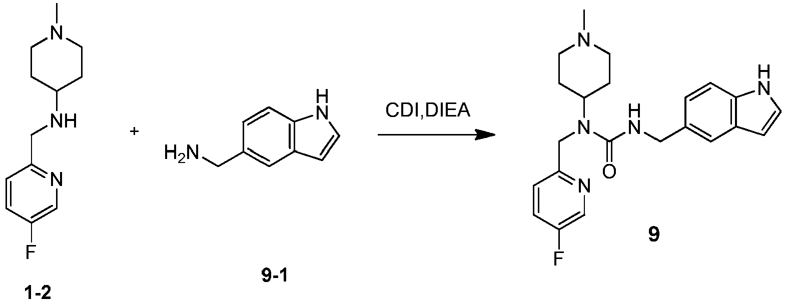

*N,N*-Dimethylformamide (5 mL), compound **9-1** (300 mg, 1.00 *eq*), and diisopropylethylamine (265 mg, 1.00 eq) were sequentially added to a 10 mL single-necked flask, and the mixture was stirred under a nitrogen atmosphere for 15 min and then cooled to 0 °C with an ice-water bath. Carbonyldiimidazole (366 mg, 1.10 *eq)* was added to the reaction solution, and the mixture was allowed to react at 0 °C for 1 h. Compound **1-2** (504 mg, 1.0 *eq)* was dissolved in 2 mL of DMF, and the resulting solution was added to the reaction solution. The resulting mixture was incubated with stirring at 25 °C for 12 h. Water (50 mL) was added to the reaction solution, and the mixture was extracted with ethyl acetate (50 mL × 3). The organic phases were combined, dried, and concentrated to dryness to obtain a crude product. The crude product was purified by column chromatography to obtain **compound 9** (yellow oily substance, 310 mg). ¹HNMR (400 MHz, CDCl₃) *δ* 8.38 (br s, 1H), 8.23 (d, *J* = 2.6 Hz, 1H), 7.52 (s, 1H), 7.35 - 7.29 (m, 3H), 7.21 (t, *J =* 2.8 Hz, 1H), 7.11 (dd, *J =* 1.5, 8.4 Hz, 1H), 6.66 (br s, 1H), 6.50 (ddd, *J =* 0.8, 2.0, 3.0 Hz, 1H), 4.51 (d, *J* = 5.1 Hz, 2H), 4.39 (s, 2H), 4.34 (br t, *J* = 4.1 Hz, 1H), 2.95 (br d, *J =* 11.5 Hz, 2H), 2.32 (s, 3H), 2.14 (br t, *J =* 11.1 Hz, 2H), 1.89 - 1.76 (m, 2H), 1.73 - 1.66 (m, 2H). MS m/z(ESI): 396.2 [M+1].

### Example 7

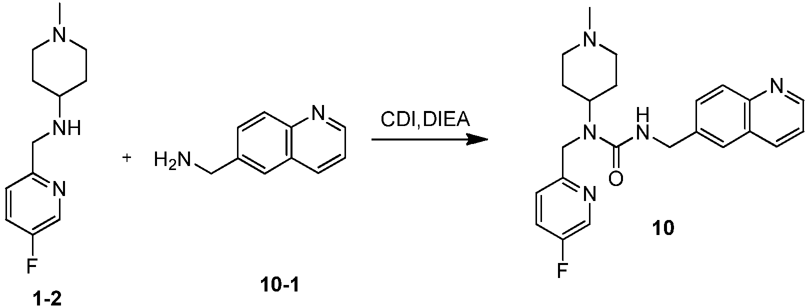

*N,N*-Dimethylformamide (5 mL), compound **10-1** (300 mg, 1.00 *eq*), and diisopropylethylamine (245 mg, 1.00 eq) were sequentially added to a 10 mL single-necked flask, and the mixture was stirred under a nitrogen atmosphere for 15 min and then cooled to 0 °C with an ice-water bath. Carbonyldiimidazole (338 mg, 1.10 *eq*) was added to the reaction solution, and the mixture was allowed to react at 0 °C for 1 h. Compound **1-2** was dissolved in 2 mL of DMF (*N,N*-dimethylformamide), and the resulting solution was added to the reaction solution. The resulting mixture was incubated with stirring at 25 °C for 12 h. Water (40 mL) was added to the reaction solution, and the mixture was extracted with ethyl acetate (50 mL × 2). The organic phases were combined, dried, and concentrated to dryness to obtain a crude product. The crude product was purified by column chromatography to obtain **compound 10** (yellow oily substance, 200 mg). ¹H NMR (400 MHz, CDCl₃) *δ* 8.89 (dd, *J =* 1.6, 4.3 Hz, 1H), 8.26 (d, *J =* 2.0 Hz, 1H), 8.13 - 8.03 (m, 2H), 7.72 - 7.63 (m, 2H), 7.45 - 7.35 (m, 3H), 4.62 (d, *J =* 5.5 Hz, 2H), 4.44 (s, 2H), 4.39 - 4.26 (m, 1H), 2.99 (br d, *J =* 11.5 Hz, 2H), 2.36 (s, 3H), 2.19 (br t, *J =* 11.1 Hz, 2H), 1.98 - 1.82 (m, 2H), 1.71 (br dd, *J* = 1.9, 11.8 Hz, 2H). MS m/z(ESI): 408.2 [M+1].

### Example 8

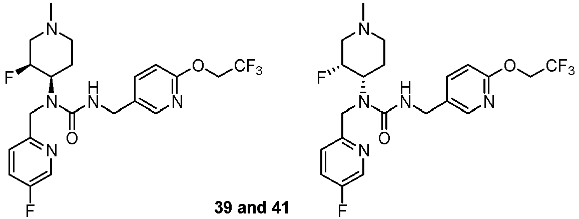

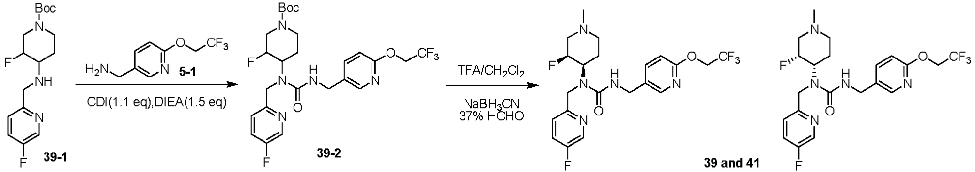

Tetrahydrofuran (5 mL), compound **5-1** (400 mg, 1.00 eq), and diisopropylethylamine (376 mg, 1.50 eq) were added to a 100 mL single-necked flask, and the mixture was cooled to 0 °C and stirred under a nitrogen atmosphere for 15 min. Carbonyldiimidazole (346 mg, 1.10 eq) was added to the reaction solution, and the mixture was allowed to react at 0 °C for 1 h. A solution of compound **39-1** (635 mg, 1.00 eq) and 4 mL of tetrahydrofuran were added to the reaction solution, and the mixture was allowed to react at 25 °C for 12 h and then filtered to obtain a filtrate. The filtrate was purified by preparative chromatography to obtain compound **39-2** (yellow oily substance, 400 mg, yield: 33.5%).

Dichloromethane (20 mL), compound **39-2** (350 mg, 1.00 eq), and trifluoroacetic acid (6 mL, 129 eq) were added to a 100 mL single-necked flask, and the mixture was stirred at 25 °C for 20 min and then concentrated to dryness by rotary evaporation to obtain a yellow oily substance. Tetrahydrofuran (20 mL), the yellow oily substance, sodium cyanoborohydride (78 mg, 2.00 eq), and a 37% aqueous formaldehyde solution (76 mg, 1.50 eq) were added to a 100 mL single-necked flask, and the mixture was stirred at 25 °C for 20 min and then concentrated to dryness by rotary evaporation. Methanol (10 mL) was added, and the mixture was stirred at 75 °C for 80 min and then concentrated to dryness by rotary evaporation to obtain a crude product. The crude product was subjected to chiral resolution (chromatographic column: DAICEL CHIRALPAK AD (250 mm × 30 mm, 10 µm); mobile phase: [A: CO₂, B: (0.1% NH₃H₂O EtOH)]; B%: 20%-20%) to obtain **compound 39** (retention time: 1.778 min, yellow viscous gel, 120 mg, yield: 39.9%) and **compound 41** (retention time: 1.876 min, yellow viscous gel, 130 mg, yield: 43.3%). **Compound 39:** ¹H NMR (400 MHz, DMSO-*d₆*) *δ* 8.47 (d, J = 2.9 Hz, 1H), 8.02 (d, J = 2.0 Hz, 1H), 7.69 - 7.62 (m, 2H), 7.34 (dd, J = 4.5, 8.8 Hz, 1H), 7.20 (t, J = 5.6 Hz, 1H), 6.92 (d, J = 8.5 Hz, 1H), 4.96 (q, J = 9.1 Hz, 2H), 4.71 - 4.45 (m, 3H), 4.29 - 4.14 (m, 2H), 4.13 - 3.99 (m, 1H), 3.13 - 3.04 (m, 1H), 2.66 (br d, J = 10.6 Hz, 1H), 2.18 (s, 3H), 1.99 - 1.87 (m, 2H), 1.62 - 1.52 (m, 2H). MS m/z(ESI): 474.3 [M+1].

**Compound 41:** ¹H NMR (400 MHz, DMSO-*d₆*) *δ* 8.47 (d, J = 2.9 Hz, 1H), 8.03 (d, J = 2.0 Hz, 1H), 7.70 - 7.62 (m, 2H), 7.35 (dd, J = 4.5, 8.8 Hz, 1H), 7.20 (t, J = 5.6 Hz, 1H), 6.93 (d, J = 8.4 Hz, 1H), 4.97 (q, J = 9.1 Hz, 2H), 4.62 - 4.45 (m, 3H), 4.28 - 4.14 (m, 2H), 4.12 - 4.01 (m, 1H), 3.13 - 3.06 (m, 1H), 2.66 (br d, J = 11.0 Hz, 1H), 2.18 (s, 3H), 1.98 - 1.87 (m, 2H), 1.64 - 1.51 (m, 2H). MS m/z(ESI): 474.3 [M+1].

### Example 9

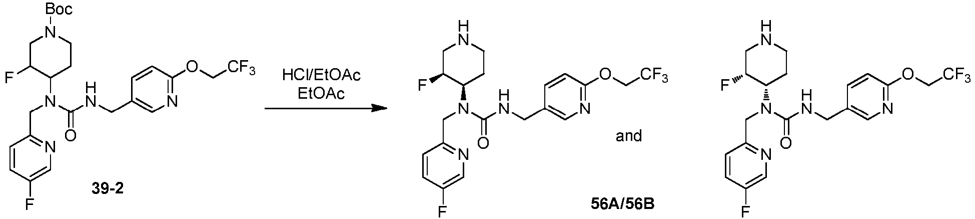

Compound **39-2** (360 mg, 1 *eq*) was added to 3.3 mL of ethyl acetate, and HCl/EtOAc (4 M, 2.41 mL, 15 *eq*) was added. The mixture was allowed to react at room temperature for 12 h. The reaction solution was concentrated to dryness to obtain a crude product, and then an aqueous sodium bicarbonate solution was added to adjust the pH to 7. The mixture was then concentrated to dryness by rotary evaporation. The crude product was subjected to chiral resolution (chromatographic column: DAICEL CHIRALPAK AD (250 mm × 30 mm, 10 µm); mobile phase: [A: CO₂, B: (0.1% NH₃H₂O EtOH)]; B%: 20%-20%), concentrated, and lyophilized to obtain **compound 56A** (retention time: 1.744 min, yellow oily substance, 95.0 mg, yield: 30.0%) and **compound 56B** (retention time: 2.519 min, yellow oily substance, 90 mg, yield: 29.4%).

**Compound 56A:** ¹H NMR (400 MHz, CDCl₃) *δ* 1.76 - 1.83 (m, 2 H), 2.58 - 2.68 (m, 2 H), 3.03 (br d, *J*=12.26 Hz, 1 H), 3.36 - 3.48 (m, 1 H), 4.27 (td, *J*=10.66, 5.19 Hz, 1 H), 4.35 (br d, *J*=5.38 Hz, 2 H), 4.45 - 4.50 (m, 2 H), 4.59 - 4.67 (m, 1 H), 4.71 - 4.78 (m, 2 H), 6.54 - 6.69 (m, 1 H), 6.81 (d, *J*=8.50 Hz, 1 H), 7.35 - 7.43 (m, 2 H), 7.57 (dd, *J*=8.44, 2.31 Hz, 1 H), 7.97 - 8.06 (m, 1 H), 8.30 (d, *J*=2.38 Hz, 1 H). MS(ESI) m/z : 460.2 [M+1].

**Compound 56B:** ¹H NMR (400 MHz, CDCl₃) *δ* 1.67 (br s, 2 H), 2.52 (br d, J=3.50 Hz, 2 H), 2.86 - 2.98 (m, 1 H), 3.30 (br s, 1 H), 4.14 (br s, 1 H), 4.23 (br s, 2 H), 4.37 (br s, 2 H), 4.53 (br s, 1 H), 4.63 (br d, J=6.13 Hz, 2 H), 6.41 - 6.61 (m, 1 H), 6.69 (br d, J=5.88 Hz, 1 H), 7.27 (br s, 2 H), 7.37 - 7.52 (m, 1 H), 7.91 (br s, 1 H), 8.18 (br s, 1 H). MS(ESI) m/z : 460.2 [M+1].

### Example 10

Compounds 27-28, 42, 44, 66/67, 69A/69B, and 70A/70B were obtained using a synthesis method and/or a resolution method similar to those in Example 8. Compounds 46A/46B, 47A/47B, 72A/72B, 81A/81B, 82A/82B, and 83A/83B were obtained using resolution conditions (chromatographic column: DAICEL CHIRALCEL OX (250 mm × 50 mm, 10 µm); mobile phase: [A: CO₂, B: (0.1% NH₃H₂O EtOH)]; B%: 25%-25%). The characterization data for the relevant compounds are shown in the table below:

| Compound No./Structure | ¹H NMR | MS | SFC retention time |
|---|---|---|---|
| 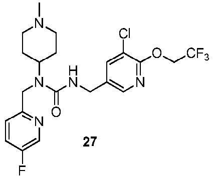 | ¹H NMR (400 MHz, CDCl₃) *δ* 8.33 (d, J = 2.8 Hz, 1H), 7.57 (d, J *=* 2.3 Hz, 1H), 7.46 - 7.39 (m, 1H), 7.35 (dd, J = 4.4, 8.5 Hz, 2H), 7.23 (d, J = 0.9 Hz, 1H), 4.63 (q, J = 8.5 Hz, 2H), 4.37 (s, 2H), 4.23 (tt, J = 3.9, 12.1 Hz, 1H), 4.13 (d, J = 5.5 Hz, 2H), 2.93 (br d, J = 11.6 Hz, 2H), 2.31 (s, 3H), 2.12 (dt, J = 2.0, 11.8 Hz, 2H), 1.81 (dq, J = 3.7, 12.2 Hz, 2H), 1.69 - 1.55 (m, 2H). | MS (ESI) m/z : 490.2 [M+1]. | / |
| 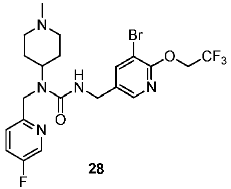 | ¹H NMR (400 MHz, CDCl₃) *δ* 8.34 (d, J = 2.8 Hz, 1H), 7.79 (d, J = 2.1 Hz, 1H), 7.49 - 7.33 (m, 3H), 4.63 (q, J = 8.5 Hz, 2H), 4.38 (s, 2H), 4.25 (tt, J = 4.0, 12.1 Hz, 1H), 4.13 (d, J = 5.5 Hz, 2H), 2.97 (br d, J = 11.6 Hz, 2H), 2.34 (s, 3H), 2.20 - 2.15 (m, 2H), 1.86 (dq, J = 3.7, 12.3 Hz, 2H), 1.65 (br d, J = 9.9 Hz, 2H). | MS (ESI) m/z : 534.1 [M+1]. | / |
| 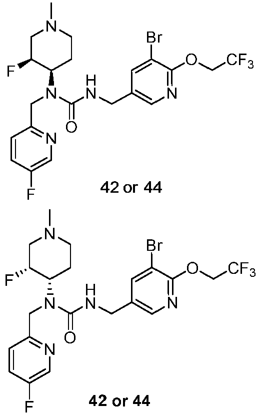 | Compound 42: ¹H NMR (400 MHz, CDCl₃) *δ* 1.73 - 1.83 (m, 2 H), 2.01 - 2.19 (m, 3 H), 2.33 (s, 3 H), 2.81 (br d, J=11.01 Hz, 1 H), 3.14 - 3.28 (m, 1 H), 4.34 (br t, J=5.44 Hz, 3 H), 4.45 (d, J=1.88 Hz, 2 H), 4.52 - 4.73 (m, 1 H), 4.78 (q, J=8.51 Hz, 2 H), 6.88 (br s, 1 H), 7.31 - 7.47 (m, 2 H), 7.78 (d, J=1.75 Hz, 1 H), 7.97 (d, J=1.63 Hz, 1 H), 8.33 (d, J=2.63 Hz, 1 H). | MS (ESI) m/z : 552.1[M+1]. | 1.465 min |
| | Compound 44: ¹H NMR (400 MHz, CDCl₃) *δ* 1.72 - 1.84 (m, 2 H), 2.02 - 2.17 (m, 3 H), 2.33 (s, 3 H), 2.81 (br d, J=11.01 Hz, 1 H), 3.16 - 3.24 (m, 1 H), 4.34 (t, J=5.44 Hz, 2 H), 4.45 (d, J=2.25 Hz, 2 H), 4.52 - 4.72 (m, 1 H), 4.78 (q, J=8.50 Hz, 2 H), 6.88 (br s, 1 H), 7.31 - 7.51 (m, 1 H), 7.32 - 7.46 (m, 1 H), 7.78 (d, J=2.00 Hz, 1 H), 7.97 (d, J=1.88 Hz, 1 H), 8.33 (d, J=2.75 Hz, 1 H). | MS (ESI) m/z : 552.1[M+1]. | 2.730 min |
| 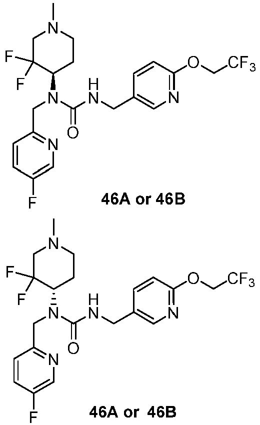 | Compound 46A: ¹H NMR (400 MHz, CDCl₃) *δ* 8.33 (d, J=2.88 Hz, 1 H) 7.98 - 8.07 (m, 1 H) 7.60 (dd, J=8.50, 2.38 Hz, 1 H) 7.46 - 7.52 (m, 1 H) 7.33 - 7.41 (m, 1 H) 6.76 - 6.83 (m, 1 H) 5.29 - 5.41 (m, 1 H) 4.71 (q, J=8.59 Hz, 2 H) 4.31 (dd, J=5.50, 1.88 Hz, 2 H) 4.07 - 4.23 (m, 2 H) 3.84 - 3.99 (m, 2 H) 2.87 - 3.01 (m, 2 H) 2.75 - 2.85 (m, 1 H) 2.42 (s, 3 H) 1.83 - 1.96 (m, 2 H). | MS (ESI) m/z: 492.2[M+1]. | 1.212 min |
| | Compound 46B: ¹H NMR (400 MHz, CDCl₃) *δ* 8.34 - 8.43 (m, 1 H) 7.97 - 8.12 (m, 1 H) 7.59 - 7.70 (m, 1 H) 7.47 - 7.56 (m, 1 H) 7.35 - 7.44 (m, 1 H) 6.78 - 6.88 (m, 1 H) 5.05 (br d, J=4.63 Hz, 1 H) 4.74 (q, J=8.55 Hz, 2 H) 4.34 (br d, J=5.25 Hz, 2 H) 4.13 (br d, J=12.26 Hz, 2 H) 3.86 - 4.02 (m, 2 H) 2.89 - 3.06 (m, 2 H) 2.77 - 2.87 (m, 1 H) 2.45 (br s, 3 H) 1.91 (br d, J=2.50 Hz, 2 H). | MS (ESI) m/z: 492.2[M+1]. | 1.385 min |
| 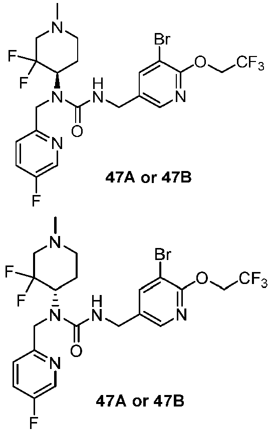 | Compound 47A: ¹H NMR (400 MHz, CDCl₃) *δ* 1.72 - 1.80 (m, 2 H), 2.09 - 2.34 (m, 3 H), 2.37 (s, 3 H), 2.91 - 3.15 (m, 2 H), 4.24 - 4.44 (m, 2 H), 4.47 - 4.61 (m, 2 H), 4.75 - 4.82 (m, 2 H), 4.83 - 4.89 (m, 1 H), 7.37 - 7.51 (m, 3 H), 7.72 - 7.80 (m, 1 H), 7.94 - 8.00 (m, 1 H), 8.26 - 8.34 (m, 1 H). | MS (ESI) m/z : 556.07[M+1]. | 0.855 min |
| | Compound 47B: ¹H NMR (400 MHz, CDCl₃) *δ* 1.71 - 1.81 (m, 2 H), 2.07 - 2.33 (m, 3 H), 2.37 (s, 3 H), 2.91 - 3.17 (m, 2 H), 4.22 - 4.44 (m, 2 H), 4.48 - 4.62 (m, 2 H), 4.75 - 4.83 (m, 2 H), 4.83 - 4.89 (m, 1 H), 4.83 - 4.90 (m, 1 H), 7.35 - 7.51 (m, 1 H), 7.70 - 7.79 (m, 1 H), 7.92 - 8.02 (m, 1 H), 8.25 - 8.36 (m, 1 H). | MS (ESI) m/z : 556.07[M+1]. | 0.974 min |
| 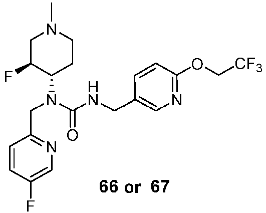 | Compound 66: ¹H NMR (400 MHz, CDCl₃) *δ* 1.50 (br dd, J=12.01, 2.38 Hz, 1 H) 2.10 - 2.22 (m, 2 H) 2.29 (s, 4 H) 2.93 - 2.98 (m, 1 H) 3.11 (td, J=10.69, 2.25 Hz, 1 H) 4.29 - 4.39 (m, 3 H) 4.53 (d, J=2.75 Hz, 2 H) 4.72 (q, J=8.63 Hz, 2 H) 4.86 (br s, 1 H) 6.79 (d, J=8.50 Hz, 1 H) 7.32 - 7.39 (m, 2 H) 7.58 (dd, J=8.50, 2.38 Hz, 1 H) 7.67 (br t, J=5.32 Hz, 1 H) 8.04 (d, J=2.13 Hz, 1 H) 8.23 (d, J=2.63 Hz, 1 H). | MS (ESI) m/z : 474.2 [M+1]. | 1.746 min |
| 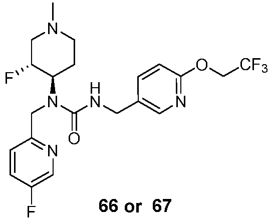 | Compound 67: ¹H NMR (400 MHz, CDCl₃) *δ* 1.45 (br d, J=10.13 Hz, 1 H) 2.01 - 2.19 (m, 2 H) 2.22 - 2.31 (m, 4 H) 2.92 (br d, J=10.38 Hz, 1 H) 3.07 (br t, J=11.76 Hz, 1 H) 4.25 - 4.39 (m, 3 H) 4.48 (br d, J=1.88 Hz, 2 H) 4.67 (q, J=8.42 Hz, 2 H) 4.82 (br s, 1 H) 6.74 (d, J=8.50 Hz, 1 H) 7.27 - 7.36 (m, 2 H) 7.53 (dd, J=8.44, 1.81 Hz, 1 H) 7.63 (br t, J=5.00 Hz, 1 H) 7.99 (s, 1 H) 8.18 (br d, J=2.13 Hz, 1 H). | MS (ESI) m/z : 474.2 [M+1]. | 1.844 min |
| 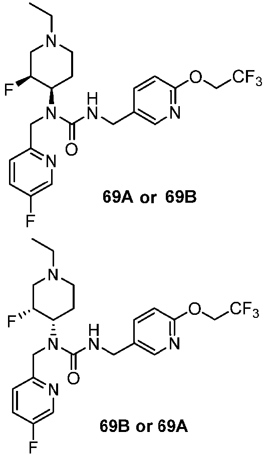 | Compound 69A: ¹H NMR (400 MHz, CDCl₃) *δ* 8.29 (d, J=2.00 Hz, 1 H), 8.03 (d, J=2.13 Hz, 1 H), 7.58 (dd, J=8.50, 2.38 Hz, 1 H), 7.37 - 7.41 (m, 2 H), 6.81 (d, J=8.50 Hz, 1 H), 6.68 - 6.78 (m, 1 H), 4.75 (q, J=8.55 Hz, 3 H), 4.46 (s, 2 H), 4.36 (d, J=5.38 Hz, 3 H), 3.28 - 3.36 (m, 1 H), 2.93 (brd, J=10.38 Hz, 1 H), 2.46 - 2.58 (m, 2 H), 2.05 - 2.17 (m, 3 H), 1.81 (br s, 3 H), 1.10 (t, J=7.13 Hz, 3 H). | MS(ESI) m/z : 488.2 [M+1]. | 1.204 min |
| | Compound 69B: ¹H NMR (400 MHz, CDCl₃) *δ* 8.29 (d, J=2.13 Hz, 1 H), 8.03 (d, J=2.00 Hz, 1 H), 7.58 (dd, J=8.50, 2.38 Hz, 1 H), 7.37 - 7.41 (m, 2 H), 6.81 (d, J=8.38 Hz, 1 H), 6.74 (br d, J=3.25 Hz, 1 H), 4.75 (q, J=8.63 Hz, 2 H), 4.46 (s, 2 H), 4.33 - 4.40 (m, 3 H), 3.28 - 3.36 (m, 1 H), 2.92 (br d, J=10.76 Hz, 1 H), 2.45 - 2.57 (m, 2 H), 2.05 - 2.16 (m, 2 H), 1.80 (br s, 3 H), 1.10 (t, J=7.19 Hz, 3 H). | MS(ESI) m/z : 488.2 [M+1]. | 2.174 min |
| 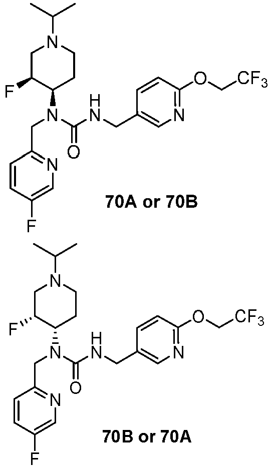 | Compound 70A: ¹H NMR (400 MHz, CDCl₃) *δ* 8.29 (s, 1 H), 8.03 (d, J=2.00 Hz, 1 H), 7.58 (dd, J=8.50, 2.38 Hz, 1 H), 7.40 (br d, J=6.25 Hz, 2 H), 6.81 (d, J=8.38 Hz, 1 H), 6.49 - 6.76 (m, 1 H), 4.75 (q, J=8.63 Hz, 2 H), 4.47 (s, 2 H), 4.36 (d, J=5.50 Hz, 3 H), 3.24 (br s, 1 H), 2.83 (br s, 2 H), 2.29 (br s, 2 H), 1.80 (br s, 2 H), 1.05 (br s, 7 H). | MS(ESI) m/z : 502.2 [M+1]. | 0.571 min |
| | Compound 70B: ¹H NMR (400 MHz, CDCl₃) *δ* 8.29 (s, 1 H), 8.03 (d, J=2.00 Hz, 1 H), 7.58 (dd, J=8.50, 2.38 Hz, 1 H), 7.40 (br d, J=6.50 Hz, 2 H), 6.81 (d, J=8.50 Hz, 1 H), 6.45 - 6.74 (m, 1 H), 4.75 (q, J=8.63 Hz, 2 H), 4.47 (s, 3 H), 4.31 - 4.38 (m, 3 H), 3.24 (br d, J=4.00 Hz, 1 H), 2.83 (br d, J=3.50 Hz, 2 H), 2.31 (br d, J=14.01 Hz, 2 H), 1.80 (br s, 2 H), 1.05 (br s, 7 H). | MS(ESI) m/z : 502.2 [M+1]. | 1. 084 min |
| | Compound 72A: ¹H NMR (400 MHz, CDCl₃) *δ* 8.29 (d, J=1.88 Hz, 1 H), 8.02 (s, 1 H), 7.55 - 7.60 (m, 1 H), 7.32 - 7.42 (m, 2 H), 6.80 (d, J=8.51 Hz, 1 H), 6.75 (br s, 1 H), 4.74 (q, J=8.59 Hz, 2 H), 4.48 - 4.69 (m, 1 H), 4.44 (s, 2 H), 4.28 - 4.39 (m, 3 H), 3.16 - 3.24 (m, 1 H), 2.80 (br d, J=11.26 Hz, 1 H), 2.06 - 2.15 (m, 2 H), 1.73 - 1.82 (m, 2 H). | MS(ESI) m/z : 477.2 [M+1]. | 1.365 min |
| 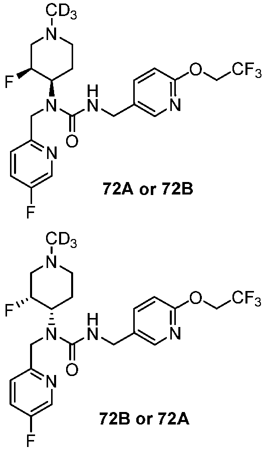 | Compound 72B: ¹H NMR (400 MHz, CDCl₃) *δ* 8.28 (br d, J=1.63 Hz, 1 H), 8.02 (s, 1 H), 7.57 (dd, J=8.38, 1.63 Hz, 1 H), 7.32 - 7.42 (m, 2 H), 6.80 (d, J=8.50 Hz, 1 H), 6.74 (br s, 1 H), 4.74 (q, J=8.71 Hz, 2 H), 4.50 - 4.69 (m, 1 H), 4.44 (s, 2 H), 4.25 - 4.38 (m, 3 H), 3.15 - 3.24 (m, 1 H), 2.80 (br d, J=11.13 Hz, 1 H), 2.02 - 2.18 (m, 2 H), 1.77 (br s, 2 H). | MS(ESI) m/z : 477.2 [M+1]. | 1.724 min |
| 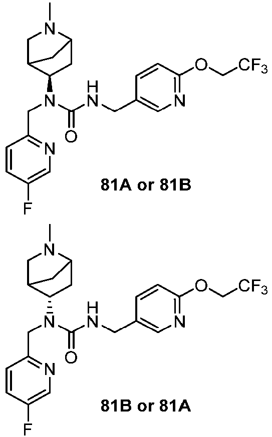 | Compound 81A: ¹H NMR (400 MHz, CDCl₃) *δ* 8.33 (s, 1H), 8.04 (d, J = 2.1 Hz, 1H), 7.60 (dd, J = 2.4, 8.5 Hz, 1H), 7.53 (br s, 1H), 7.37 (br d, J = 6.8 Hz, 2H), 6.81 (d, J = 8.5 Hz, 1H), 4.92 (d, J = 16.4 Hz, 1H), 4.75 (q, J = 8.6 Hz, 2H), 4.49 (d, J = 16.4 Hz, 1H), 4.35 (d, J *=* 5.4 Hz, 2H), 4.18 - 4.04 (m, 1H), 3.06 (br s, 1H), 2.95 (br d, J = 8.4 Hz, 1H), 2.68 (br s, 1H), 2.24 (s, 3H), 2.03 (br s, 1H), 1.77 (br s, 1H), 1.73 (br d, J = 10.8 Hz, 2H), 1.36 (br d, J = 10.3 Hz, 1H). | MS(ESI) m/z : 468.2 [M+1]. | 2.210 min |
| | Compound 81B: ¹H NMR (400 MHz, CDCl₃) *δ* 8.32 (d, J = 2.6 Hz, 1H), 8.07 (d, J = 2.1 Hz, 1H), 7.59 - 7.59 (m, 1H), 7.62 (dd, J = 2.4, 8.4 Hz, 1H), 7.56 (br t, J = 5.0 Hz, 1H), 7.45 - 7.41 (m, 1H), 7.41 - 7.35 (m, 1H), 6.82 (d, J = 8.5 Hz, 1H), 4.82 (s, 1H), 4.75 (q, J = 8.6 Hz, 2H), 4.52 (br d, J = 16.6 Hz, 1H), 4.44 - 4.28 (m, 2H), 4.24 - 4.11 (m, 1H), 3.61 - 3.43 (m, 1H), 3.52 - 3.41 (m, 1H), 3.40 - 3.28 (m, 1H), 3.04 (br d, J = 4.3 Hz, 1H), 2.55 (br s, 3H), 2.17 (br d, J = 14.8 Hz, 1H), 1.97 - 1.82 (m, 2H), 1.63 (br d, J = 9.3 Hz, 1H). | MS(ESI) m/z : 468.2 [M+1]. | 2.579 min |
| 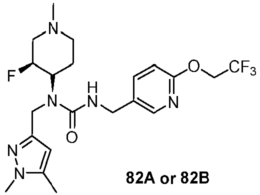 | Compound 82A: ¹H NMR (400 MHz, CDCl₃) *δ* 1.78 - 1.86 (m, 2 H), 2.11 - 2.17 (m, 1 H), 2.21 (s, 4 H), 2.37 (s, 3 H), 2.85 (br d, J=10.88 Hz, 1 H), 3.21 - 3.28 (m, 1 H), 3.59 (s, 3 H), 4.21 (s, 2 H), 4.34 (dd, J=5.13, 1.50 Hz, 2 H), 4.44 - 4.65 (m, 2 H), 4.75 (d, J=8.63 Hz, 2 H), 5.94 (s, 1 H), 6.44 (br s, 1 H), 6.77 (d, J=8.50 Hz, 1 H), 7.54 (dd, J=8.50, 2.38 Hz, 1 H), 7.97 (d, J=2.00 Hz, 1 H). | MS(ESI) m/z : 473.2 [M+1]. | 1.197 min |
| 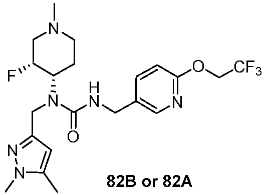 | Compound 82B: ¹H NMR (400 MHz, CDCl₃) *δ* 1.82 (br d, J=4.38 Hz, 2 H), 2.10 - 2.16 (m, 1 H), 2.21 (s, 4 H), 2.79 - 2.89 (m, 1 H), 3.19 - 3.30 (m, 1 H), 3.59 (s, 3 H), 4.21 (s, 2 H), 4.34 (dd, J=5.19, 1.56 Hz, 2 H), 4.40 - 4.68 (m, 2 H), 4.75 (d, J=8.63 Hz, 2 H), 5.94 (s, 1 H), 6.37 - 6.51 (m, 1 H), 6.77 (d, J=8.50 Hz, 1 H), 7.54 (dd, J=8.44, 2.31 Hz, 1 H), 7.97 (d, J=2.00 Hz, 1 H). | MS(ESI) m/z : 473.2 [M+1]. | 1.706 min |

### Example 11

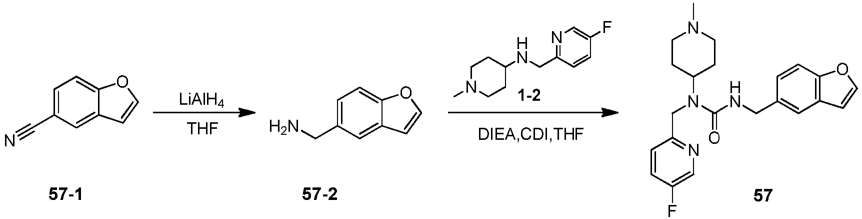

Tetrahydrofuran (20 mL) and compound 57-1 (1.80 g, 1.00 eq) were added to a 100 mL three-necked flask, and the mixture was cooled to 0 °C. Lithium aluminum hydride (954 mg, 2.00 eq) was added to the reaction solution in portions, and the mixture was stirred at 25 °C for 12 h. Water (1 mL), a 15% aqueous sodium hydroxide solution (1 mL), and water (3 mL) were sequentially added to the reaction solution, and the mixture was extracted three times with ethyl acetate. The resulting organic phase was dried, filtered, and concentrated to dryness by rotary evaporation to obtain a crude product. The crude product was purified by preparative chromatography to obtain compound 57-2 (620 mg, yield: 32.8%).

Tetrahydrofuran (6 mL), compound 57-2 (570 mg, 1.00 eq), and diisopropylethylamine (750 mg, 1.50 eq) were added to a 100 mL single-necked flask, and the mixture was cooled to 0 °C and stirred under a nitrogen atmosphere for 15 min. Carbonyldiimidazole (690 mg, 1.10 eq) was added to the reaction solution, and the mixture was allowed to react at 0 °C for 1 h. Compound **1-2** (864 mg, 1.00 eq) was added to the reaction solution, and the mixture was allowed to react at 25 °C for 12 h and then filtered to obtain a crude product. The crude product was purified by preparative chromatography to obtain **compound 57** (off-white solid, 950 mg, yield: 60.9%). ¹H NMR (400 MHz, DMSO-*d₆*) *δ* 8.47 (d, J = 2.8 Hz, 1H), 7.95 (d, J = 2.1 Hz, 1H), 7.65 (dt, J = 2.9, 8.8 Hz, 1H), 7.52 - 7.45 (m, 2H), 7.34 (dd, J = 4.5, 8.6 Hz, 1H), 7.23 - 7.14 (m, 2H), 6.92 (d, J = 1.4 Hz, 1H), 4.49 (s, 2H), 4.36 (br d, J = 5.5 Hz, 2H), 3.96 (brt, J = 11.5 Hz, 1H), 2.71 (br d, J = 11.1 Hz, 2H), 2.09 (s, 3H), 1.94 - 1.83 (m, 2H), 1.61 - 1.50 (m, 2H), 1.49 - 1.41 (m, 2H). MS (ESI) m/z : 397.2 [M+1].

### Example 12

Compounds 12-13, 58-61, and 84 were obtained using a synthesis method similar to that in Example 11. The characterization data for compounds 12-13, 58-61, and 84 are shown in the table below:

| Compound No./Structure | ¹H NMR | MS |
|---|---|---|
| 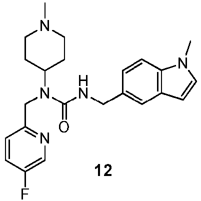 | ¹H NMR (400 MHz, CDCl₃) *δ* 8.22 (d, J = 2.9 Hz, 1H), 7.59 - 7.55 (m, 1H), 7.54 (s, 1H), 7.38 (dt, J = 2.9, 8.4 Hz, 1H), 7.30 (d, J = 5.3 Hz, 1H), 7.17 (dd, J = 1.5, 8.5 Hz, 1H), 7.06 (d, J = 3.1 Hz, 1H), 6.46 - 6.42 (m, 1H), 5.95 - 5.30 (m, 2H), 4.61 - 4.54 (m, 1H), 4.51 (d, J = 5.3 Hz, 2H), 4.46 (s, 2H), 3.79 (s, 3H), 3.36 (br d, J = 12.0 Hz, 2H), 2.79 (dt, J = 2.1, 12.4 Hz, 2H), 2.65 (s, 3H), 2.55 - 2.41 (m, 2H), 1.76 (br d, J = 11.1 Hz, 2H). | MS (ESI) m/z : 410.2 [M+1]. |
| 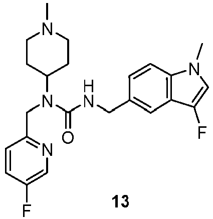 | ¹H NMR (400 MHz, CDCl₃) *δ* 8.46 (d, J = 2.9 Hz, 1H), 7.64 (dt, J = 2.9, 8.8 Hz, 1H), 7.39 - 7.29 (m, 4H), 7.11 (br d, J = 6.9 Hz, 2H), 4.48 (s, 2H), 4.34 (br d, J = 5.6 Hz, 2H), 3.96 (br t, J = 11.6 Hz, 1H), 3.70 (s, 3H), 2.73 (br d, J = 11.1 Hz, 2H), 2.11 (s, 3H), 1.91 (br t, J = 10.9 Hz, 2H), 1.62 - 1.41 (m, 4H). | MS(ESI) m/z : 428.2 [M+1]. |
| 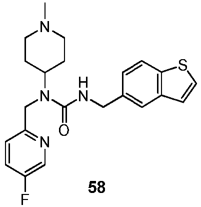 | ¹H NMR (400 MHz, DMSO-*d₆*) *δ* 8.48 (br d, J = 2.3 Hz, 1H), 7.91 (br d, J = 8.3 Hz, 1H), 7.74 - 7.71 (m, 1H), 7.70 - 7.67 (m, 1H), 7.67 - 7.62 (m, 1H), 7.41 (br d, J = 5.3 Hz, 1H), 7.35 (br dd, J = 4.4, 8.6 Hz, 1H), 7.28 (br d, J = 8.3 Hz, 1H), 7.21 (br t, J = 5.1 Hz, 1H), 4.51 (s, 2H), 4.40 (br d, J = 5.3 Hz, 2H), 3.97 (br t, J = 11.1 Hz, 1H), 2.71 (br d, J = 10.6 Hz, 2H), 2.09 (s, 3H), 1.88 (br t, J = 10.9 Hz, 2H), 1.63 - 1.42 (m, 4H). | MS (ESI) m/z : 413.2 [M+1]. |
| 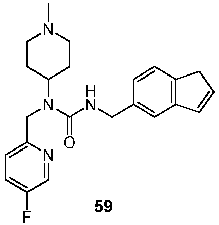 | ¹H NMR (400 MHz, CDCl₃) *δ* 1.80 (br d, J=12.88 Hz, 2 H), 2.38 (q, J=11.84 Hz, 2 H), 2.77 (s, 3 H), 2.86 (br d, J=10.76 Hz, 3 H), 3.38 (s, 2 H), 3.54 (br d, J=10.88 Hz, 2 H), 4.45 (br d, J=15.01 Hz, 4 H), 4.55 - 4.65 (m, 1 H), 6.56 (br dd, J=13.70, 5.44 Hz, 1 H), 6.86 (br t, J=5.00 Hz, 1 H), 7.11 - 7.23 (m, 1 H), 7.33 - 7.49 (m, 4 H), 7.71 - 7.94 (m, 1 H), 8.24 (d, J=1.75 Hz, 1 H). | MS (ESI) m/z : 395.2 [M+1]. |
| 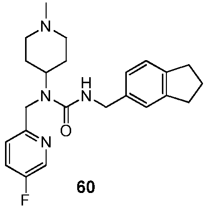 | ¹H NMR (400 MHz, CDCl₃) *δ* 8.28 (d, J = 2.8 Hz, 1H), 7.41 - 7.34 (m, 1H), 7.33 - 7.28 (m, 1H), 7.15 (d, J = 7.6 Hz, 1H), 7.10 (s, 1H), 7.02 (d, J = 7.8 Hz, 1H), 6.61 - 6.54 (m, 1H), 4.41 - 4.36 (m, 4H), 4.35 - 4.25 (m, 1H), 2.93 - 2.84 (m, 6H), 2.29 (s, 3H), 2.13 - 2.02 (m, 4H), 1.79 - 1.65 (m, 4H). | MS (ESI) m/z : 397.2 [M+1]. |
| 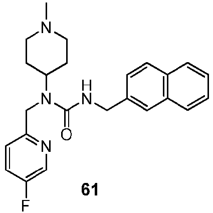 | ¹H NMR (400 MHz, CDCl₃) *δ* 8.24 (d, J = 2.8 Hz, 1H), 7.85 - 7.75 (m, 3H), 7.70 (s, 1H), 7.50 - 7.43 (m, 2H), 7.42 - 7.29 (m, 3H), 6.91 (br s, 1H), 4.59 (d, J = 5.4 Hz, 2H), 4.42 (s, 2H), 4.37 - 4.26 (m, 1H), 2.90 (br d, J = 11.6 Hz, 2H), 2.29 (s, 3H), 2.09 (dt, J = 2.8, 11.6 Hz, 2H), 1.79 - 1.65 (m, 4H). | MS (ESI) m/z : 407.2 [M+1]. |
| 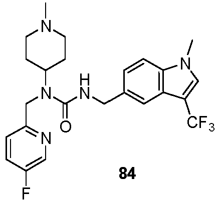 | ¹H NMR (400 MHz, CDCl₃) *δ* 8.25 (br d, J = 1.3 Hz, 1H), 7.62 (br s, 1H), 7.40 - 7.35 (m, 2H), 7.32 - 7.28 (m, 2H), 7.09 - 6.90 (m, 1H), 4.54 (br d, J = 5.3 Hz, 2H), 4.48 - 4.32 (m, 3H), 3.82 (s, 3H), 3.01 (br d, J = 10.5 Hz, 2H), 2.37 (br s, 3H), 2.22 (br t, J = 10.8 Hz, 3H), 2.11 - 1.83 (m, 4H), 0.99 - 0.75 (m, 1H). | MS(ESI) m/z : 478.2 [M+1]. |

### Example 13

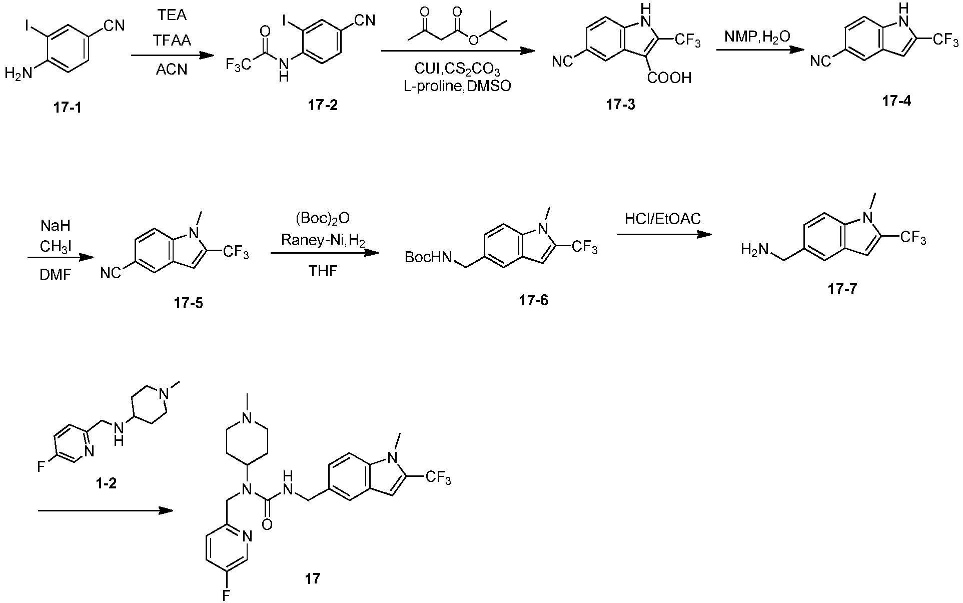

Acetonitrile (30 mL), compound **17-1** (10.0 g, 1.00 eq), and triethylamine (4.15 g, 1.00 eq) were sequentially added to a 100 mL three-necked flask under a N₂ atmosphere. Trifluoroacetic anhydride (9.47 g, 1.10 eq) was added in portions, and the reaction temperature was controlled at 35-40 °C. The mixture was incubated at 40 °C for half an hour, then diluted with 30 mL of water, and filtered. The filter cake was collected and dried under vacuum to obtain compound **17-2** (yellow oily substance, 11.8 g, yield: 85.3%).

Cesium carbonate (23.96 g, 2.50 eq), cuprous iodide (840 mg, 0.15 eq), and L-proline (2.27 g, 1.0 eq) were sequentially added to a 100 mL three-necked flask, and 20 mL of *N,N*-dimethylformamide was added to the reaction flask. The mixture was allowed to react at 25 °C for 15 min. Compound **17-2** (10 g, 1.00 eq) was dissolved in 10 mL of *N,N*-dimethylformamide, and the resulting solution was slowly added dropwise to the reaction solution. The mixture was allowed to react at 25 °C for 15 min. *tert-*Butyl acetoacetate (9.3 g, 2.00 eq) was added to the reaction solution, and the mixture was incubated at 90 °C for 12 h. After the reaction was completed, the contents of the container were cooled to 18-23 °C. Water (40 mL) was added over a period of more than 15 s to maintain the reaction temperature ed below 35 °C. Isopropyl acetate (40 mL), toluene (80 mL), and water (40 mL) were then added. The aqueous layer was drained, and the organic layer was washed with a saturated ammonium chloride solution (50 mL) and then reduced to the minimum by vacuum distillation. Dichloromethane (30 mL) was added, and the internal temperature was adjusted to 18-23 °C. Trifluoroacetic acid (10 mL) was added over 15 min. The solution was stirred overnight. The solid was filtered out and then washed with dichloromethane (20 mL × 2) to obtain compound **17-3** (yellow solid, 3.52 g, yield: 47.7%).

1-Methyl-2-pyrrolidone (5 mL), water (0.5 mL), and compound **17-3** (1.0 g, 1.00 eq) were sequentially added to a 50 mL single-necked flask. The mixture was incubated at 130 °C for 12 h and then cooled to room temperature, followed by the addition of 30 mL of water. The mixture was stirred for 40 min and then filtered. The filter cake was washed with water, and the product was dried under vacuum. The crude product was purified by column chromatography to obtain compound **17-4** (white solid, 760 mg, yield: 46.1%).

*N,N*-Dimethylformamide (15 mL), compound **17-4** (750 mg, 1.00 eq), and sodium hydride (356.85 mg, 60% purity, 2.50 eq) were sequentially added to a 50 mL three-necked flask, and the mixture was allowed to react at room temperature for 30 min. Iodomethane (1.01 g, 2.00 eq) was added to the reaction solution in portions at 0 °C, and the mixture was allowed to react at room temperature for 2 h. Ice water (150 mL) was added to the reaction solution, and the mixture was extracted with ethyl acetate (100 mL × 3). The organic phases were combined, dried, and concentrated to dryness to obtain compound **17-5** (white solid, 790 mg, yield: 92%).

Tetrahydrofuran (50 mL), compound **17-5** (790 mg, 1.00 eq), di-*tert*-butyl dicarbonate (769 mg, 1.00 eq), and Raney Ni (1.00 g, 3.31 eq) were sequentially added to a 200 mL hydrogenation flask, and the mixture was allowed to react at 70 °C under 50 Psi with H₂ for 16 h, then filtered, and concentrated to dryness by rotary evaporation to obtain compound **17-6** (white solid, 1.62 g, crude product).

Ethyl acetate (5 mL), compound **17-6** (1.62 g, 1.00 eq), and HCl/EtOAc (4 M, 20.0 mL, 16.21 eq) were sequentially added to a 50 mL single-necked flask. The mixture was allowed to react at room temperature for 2 h and then concentrated to dryness by rotary evaporation. The crude product was purified by preparative chromatography to obtain compound **17-7** (yellow oily substance, 297 mg, yield: 25.4%).

Tetrahydrofuran (4 mL), compound **1-2** (78.33 mg, 1.00 eq), and diisopropylethylamine (141.5 mg, 1.10 eq) were sequentially added to a 50 mL three-necked flask, and the mixture was allowed to react at room temperature for 15 min. Carbonyldiimidazole (195.39 mg, 1.10 eq) was added to the reaction solution at 0 °C, and the mixture was allowed to react at 0 °C for one hour. After a reaction intermediate was detected, compound **17-7** (244 mg, 1.00 eq) was added to the reaction solution, and the mixture was allowed to react at room temperature for 12 h. Water (20 mL) was added to the reaction solution, and the mixture was extracted with dichloromethane (20 mL × 2). The organic phases were combined, washed with 20 mL of saturated brine, dried, and concentrated to dryness to obtain a crude product. The crude product was purified by preparative chromatography to obtain **compound 17** (white solid, 145 mg, yield: 27.2%). ¹H NMR (400 MHz, CDCl₃) *δ* 8.22 (d, J = 2.5 Hz, 1H), 7.59 (s, 1H), 7.44 - 7.37 (m, 3H), 7.33 (s, 2H), 6.89 (s, 1H), 4.52 (d, J = 5.3 Hz, 2H), 4.49 - 4.43 (m, 1H), 4.41 (s, 2H), 3.84 (s, 3H), 3.28 (br d, J = 11.6 Hz, 2H), 2.58 (s, 3H), 2.56 - 2.48 (m, 2H), 2.17 (dq, J = 3.2, 12.6 Hz, 2H), 1.75 (br d, J = 11.6 Hz, 2H). MS (ESI) m/z : 478.2 [M+1].

### Example 14

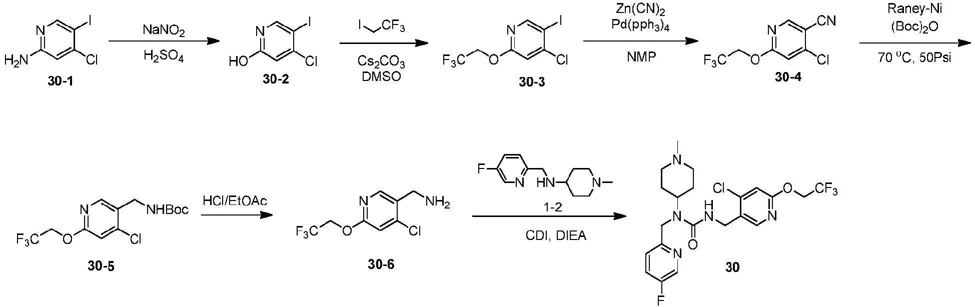

Compound **30-1** (10.0 g, 1.00 eq) was slowly added dropwise to 100 mL of sulfuric acid (184 g, 35.8 eq, 75% purity) at 0 °C, and the mixture was stirred for 10 min. Sodium nitrite dissolved in 81 mL of water was then slowly added dropwise at 0 °C, and the mixture was incubated with stirring for 3 h under an ice-water bath with the temperature maintained at 5-10 °C. Under an ice-water bath, 50 mL of an ammonium hydroxide solution was added to the reaction solution, and the reaction solution was filtered. The filter cake was washed with 100 mL of water and then concentrated to dryness to obtain compound **30-2** (yellow solid, 9.0 g, yield: 89.6%).

Dimethyl sulfoxide (40 mL), compound **30-2** (4.0 g, 1.00 eq), trifluoroiodoethane (4.93 g, 1.50 eq), and cesium carbonate (10.2 g, 2.00 eq) were sequentially added to a 250 mL three-necked flask, and the mixture was stirred at 100 °C for 1 h. Water (350 mL) was added to the reaction solution, and the mixture was extracted with ethyl acetate (100 mL × 3). The organic phases were combined and extracted with saturated brine (100 mL × 2). The resulting organic phase was dried and concentrated to dryness to obtain a crude product. The crude product was purified by column chromatography to obtain compound **25-3** (yellow solid, 1.3 g, yield: 24.6%).

N-Methylpyrrolidone (12 mL), compound **30-3** (1.20 g, 1.00 eq), and zinc cyanide (229 mg, 0.55 eq) were sequentially added to a 100 mL three-necked flask, and tetrakis(triphenylphosphine)palladium was added under a nitrogen atmosphere. The mixture was incubated with stirring at 130 °C for 12 h. Water (100 mL) was added to the reaction solution, and the mixture was extracted with ethyl acetate (100 mL × 3). The organic phases were combined and extracted with saturated brine (50 mL × 2). The resulting organic phase was dried and concentrated to dryness to obtain a crude product. The crude product was purified by column chromatography to obtain compound **30-4** (white solid, 400 mg, yield: 47.5%).

Tetrahydrofuran (5 mL), compound **30-4** (300 mg, 1.00 eq), di-*tert*-butyl dicarbonate (276 mg, 1.00 eq), and Raney Ni (300 mg, 2.76 eq) were sequentially added to a 100 mL steel bottle under an argon atmosphere. Under hydrogen catalysis, the mixture was incubated with stirring at 70 °C under 50 Psi for 12 h. The reaction solution was filtered, and the filtrate was concentrated to dryness to obtain a crude product. The crude product was purified by column chromatography to obtain compound **30-5** (yellow oily substance, 60 mg, yield: 27.7%) was obtained.

Ethyl acetate (0.5 mL), compound **30-5** (100 mg, 1.00 eq), and HCl/EtOAc (1 mL) were sequentially added to a 10 mL single-necked flask, and the mixture was incubated with stirring at 25 °C for 6 h. The reaction solution was concentrated to dryness to obtain a crude product of compound **30-6** (yellow solid, 20 mg), which was used directly in the next step.

Tetrahydrofuran (1 mL), compound **30-6** (20 mg, 1.00 eq), and diisopropylethylamine (10.7 mg, 1.00 eq) were sequentially added to a 10 mL single-necked flask, and the mixture was stirred under a nitrogen atmosphere for 15 min and then cooled to 0 °C with an ice-water bath. Carbonyldiimidazole (14.8 mg, 1.10 eq) was added to the reaction solution, and the mixture was allowed to react at 0 °C for 1 h. Compound **1-2** (20 mg, 1.00 eq) was dissolved in 0.5 mL of tetrahydrofuran, and the resulting solution was added to the reaction solution. The resulting mixture was incubated with stirring at 25 °C for 12 h. Water (10 mL) was added to the reaction solution, and the mixture was extracted with ethyl acetate (5 mL × 2). The organic phases were combined and extracted with saturated brine (5 mL × 2). The resulting organic phase was dried and concentrated to dryness to obtain a crude product. The crude product was separated and purified by preparative chromatography to obtain **compound 30** (white solid, 10 mg, yield: 24.3%). ¹H NMR (400 MHz, CDCl₃) *δ* 8.35 (s, 1H), 7.72 (br s, 1H), 7.47 (s, 1H), 7.44 - 7.41 (m, 2H), 6.72 (s, 1H), 4.57 (q, J = 8.6 Hz, 2H), 4.39 (s, 2H), 4.27 (br s, 1H), 4.23 (d, J = 5.9 Hz, 2H), 3.10 (br d, J = 11.6 Hz, 2H), 2.44 (s, 3H), 2.32 (br t, J = 11.4 Hz, 2H), 2.14 - 2.13 (m, 1H), 2.07 (br d, J = 11.4 Hz, 1H), 1.67 (br d, J = 11.9 Hz, 2H). MS (ESI) m/z : 490.2 [M+1].

### Example 15

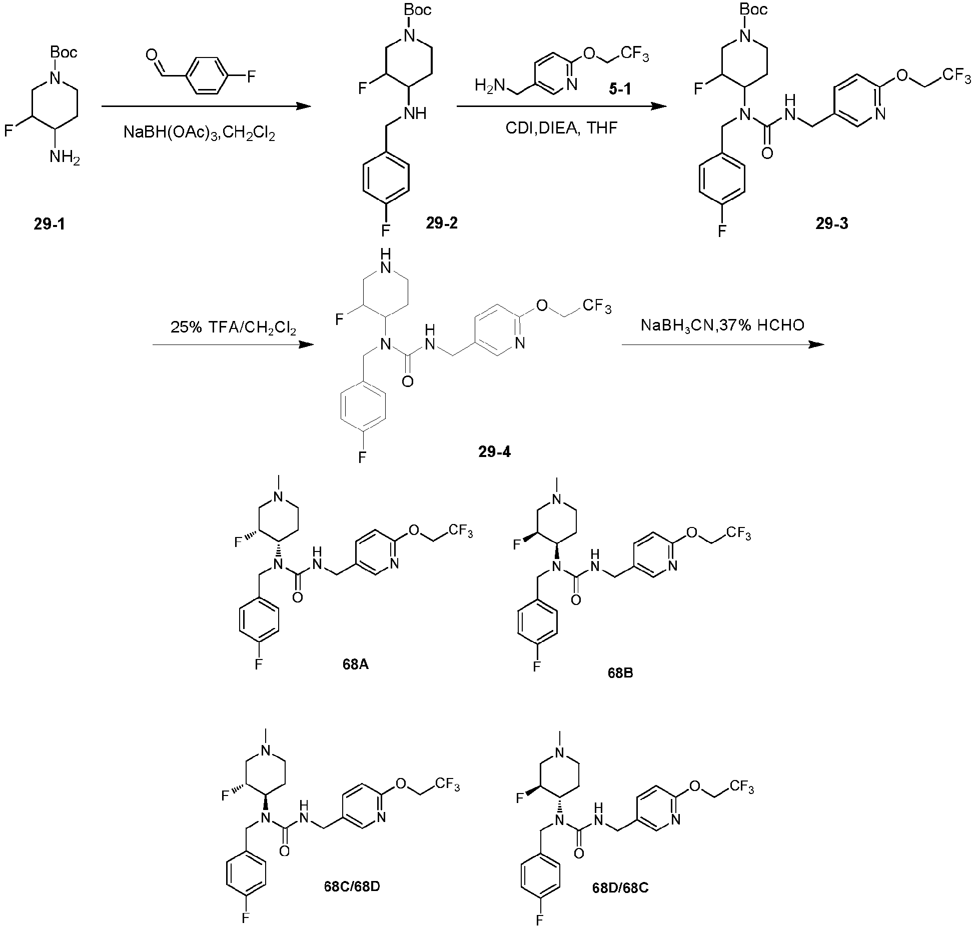

Dichloromethane (30 mL), compound **29-1** (2.80 g, 1.00 *eq),* and p-fluorobenzaldehyde (4.92 g, 1.00 *eq)* were sequentially added to a 100 mL three-necked flask at room temperature, and the mixture was uniformly stirred. Sodium triacetoxyborohydride (9.56 g, 2.00 *eq)* was then added to the reaction solution, and the mixture was stirred at room temperature for 16 h. A saturated sodium bicarbonate solution (30 mL) was added to the reaction solution, and the mixture was stirred for another 10 min and then left to stand for phase separation. The organic phase was isolated, and the aqueous phase was extracted with dichloromethane (15 mL × 3). The organic phases were combined, dried over anhydrous sodium sulfate, and filtered, and the filtrate was concentrated to dryness to obtain a crude product. The crude product was purified by column chromatography, and then further separated and purified by preparative chromatography to obtain compound **29-2** (colorless oily substance, 1.20 g).

Tetrahydrofuran (15 mL) and compound **5-1** (1.04 g, 1.00 *eq,* HCl) were sequentially added to a 100 mL three-necked flask, and diisopropylethylamine (1.11 g, 2.00 *eq)* was added dropwise under a nitrogen atmosphere. The mixture was stirred at 20 °C for 15 min. The reaction solution was then cooled to 0 °C, and carbonyldiimidazole (765 mg, 1.10 *eq)* was added thereto in portions. The reaction was monitored to find that compound 5-1 had completely reacted. Compound **29-2** (1.40 g, 1.00 *eq)* was dissolved in tetrahydrofuran (5 mL), and the resulting solution was slowly added dropwise to the reaction solution. The mixture was incubated with stirring at 25 °C for 12 h. Water (100 mL) was added to the reaction solution, and the mixture was extracted with ethyl acetate (50 mL × 3). The organic phases were combined, washed with 80 mL of saturated brine, dried, and concentrated to dryness to obtain a crude product. The crude product was purified by column chromatography to obtain compound **29-3** (colorless oily substance, 966 mg, yield: 38.8%).

Anhydrous dichloromethane (27 mL), compound **29-3** (966 mg, 1.00 *eq),* and trifluoroacetic acid (15.4 g, 78.0 *eq)* were sequentially added to a 50 mL single-necked flask, and the mixture was incubated with stirring at 25 °C for 20 min. The reaction solution was concentrated to dryness by rotary evaporation to obtain a crude product of compound 29-4 (light yellow oily substance, 850 mg, yield: 91.4%).

Tetrahydrofuran (8 mL), compound **29-4** (800 mg, 1.00 *eq),* sodium cyanoborohydride (219 mg, 2.00 *eq),* and formaldehyde (212 mg, 37%, 1.50 *eq)* were sequentially added to a 50 mL single-necked flask. The mixture was incubated with stirring at 25 °C for 12 h. Water (50 mL) was added to the reaction solution, and the mixture was extracted with ethyl acetate (30 mL × 3). The organic phases were combined, washed with 40 mL of saturated brine, dried, and concentrated to dryness to obtain a crude product. The crude product was purified by column chromatography and then further subjected to SFC resolution (chromatographic column: DAICEL CHIRALCEL OX (250 mm × 50 mm, 10 µm); mobile phase: [A: CO₂, B: (0.1% NH₃H₂O EtOH)]; B%: 25%-25%) to obtain **compound 68A** (retention time: 1.457 min, white solid, 159 mg, yield: 23.1%), **compound 68B** (retention time: 1.707 min, white solid, 163 mg, yield: 24.4%), **compound 68C** (retention time: 1.935 min, white solid, 142 mg, yield: 21.2%), and **compound 68D** (retention time: 2.356 min, white solid, 133 mg, yield: 19.8%).

**Compound 68A:** ¹H NMR (400 MHz, CDCl₃) *δ* 7.87 (d, *J =* 1.9 Hz, 1H), 7.35 (dd, *J =* 2.3, 8.5 Hz, 1H), 7.17 - 7.08 (m, 2H), 7.00 (br t, *J =* 8.5 Hz, 2H), 6.75 (d, *J =* 8.5 Hz, 1H), 5.17 - 4.99 (m, 1H), 4.94 (br t, *J* = 5.1 Hz, 1H), 4.71 (d, *J =* 8.6 Hz, 2H), 4.56 - 4.43 (m, 2H), 4.30 - 4.17 (m, 2H), 3.81 - 3.57 (m, 2H), 3.22 - 2.91 (m, 2H), 2.80 (s, 3H), 2.60 - 2.45 (m, 1H), 1.79 (br d, *J =* 11.8 Hz, 1H). MS(ESI) m/z : 473.2 [M+1].

**Compound 68B:** ¹H NMR (400 MHz, CDCl₃) *δ* 7.88 (d, *J =* 2.0 Hz, 1H), 7.37 (dd, *J* = 2.3, 8.4 Hz, 1H), 7.15 (dd, *J =* 5.3, 8.4 Hz, 2H), 7.06 - 6.98 (m, 2H), 6.76 (d, *J =* 8.5 Hz, 1H), 5.16 - 4.97 (m, 1H), 4.85 (br t, *J =* 5.1 Hz, 1H), 4.76 - 4.70 (m, 2H), 4.52 (br d, *J =* 13.3 Hz, 2H), 4.32 - 4.19 (m, 2H), 3.64 (br t*, J* = 11.8 Hz, 1H), 3.52 (br d, *J*= 10.8 Hz, 1H), 3.08 - 2.80 (m, 2H), 2.74 (s, 3H), 2.59 - 2.44 (m, 1H), 1.78 (br d, *J =* 11.5 Hz, 1H). MS(ESI) m/z : 473.2 [M+1].

Compound 68C: ¹H NMR (400 MHz, CDCl₃) *δ* 7.91 (d*, J =* 1.9 Hz, 1H), 7.44 (dd, *J =* 2.3, 8.5 Hz, 1H), 7.27 - 7.19 (m, 2H), 7.02 (br t, *J =* 8.5 Hz, 2H), 6.77 (d*, J =* 8.5 Hz, 1H), 4.76 - 4.70 (m, 3H), 4.58 - 4.38 (m, 3H), 4.30 - 4.27 (m, 2H), 3.24 - 3.21 (m, 1H), 2.85 - 2.82 (m, 1H), 2.34 (s, 3H), 2.21 - 2.12 (m, 2H), 1.82-1.80 (m, 2H). MS(ESI) m/z : 473.2 [M+1].

Compound 68D: ¹H NMR (400 MHz, CDCl₃) *δ* 7.91 (d, *J* = 2.0 Hz, 1H), 7.43 (dd, *J* = 2.3, 8.4 Hz, 1H), 7.27 - 7.20 (m, 2H), 7.06 - 6.98 (m, 2H), 6.77 (d, *J =* 8.5 Hz, 1H), 4.76 - 4.70 (m, 3H), 4.58 - 4.38 (m, 3H), 4.30 - 4.27 (m, 2H), 3.24 - 3.21 (m, 1H), 2.85 - 2.82 (m, 1H), 2.34 (s, 3H), 2.21 - 2.12 (m, 2H), 1.82-1.80 (m, 2H). MS(ESI) m/z : 473.2 [M+1].

### Example 16

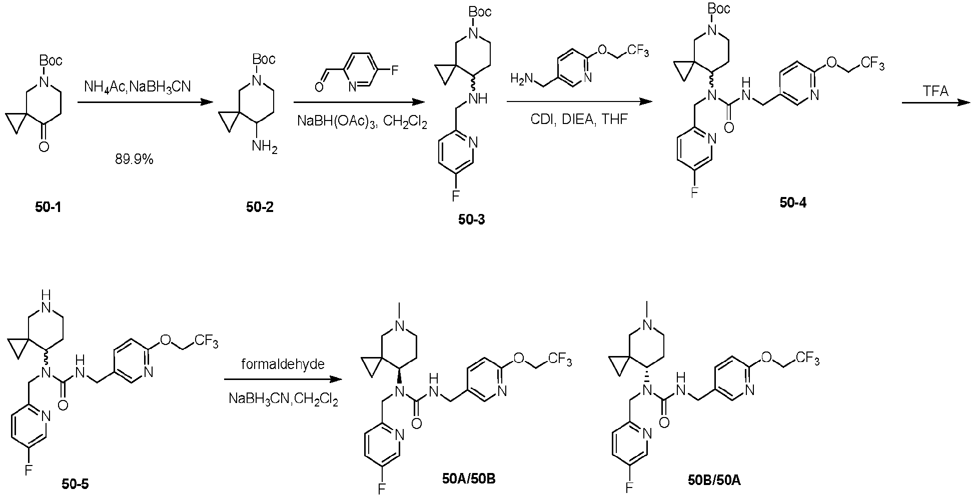

Methanol (30 mL), compound **50-1** (2.00 g, 1.00 *eq),* and ammonium acetate (1.37 g, 2.00 *eq*) were sequentially added to a 250 mL three-necked flask, and the mixture was stirred at 20 °C for 1 h. Sodium cyanoborohydride (1.12 g, 2.00 *eq*) was added to the reaction solution, and then the mixture was stirred at 60 °C for another 12 h until the reaction was completed. The reaction solution was then concentrated to dryness to obtain a crude product. The crude product was purified by column chromatography to obtain compound **50-2** (yellow liquid, 1.80 g, yield: 89.9%). Dichloromethane (20 mL), compound **50-2** (1.80 g, 1.00 *eq*)*,* and 5-fluoropicolinaldehyde (999 mg, 1.00 *eq*) were sequentially added to a 100 mL three-necked flask, and the mixture was stirred at 30 °C for 6 h under a nitrogen atmosphere. Sodium triacetoxyborohydride (3.37 g, 2.00 *eq*) was then added, and the mixture was stirred at 30 °C for another 13 h. After the temperature was reduced to room temperature, water (10 mL) was added to quench the reaction, and the mixture was extracted with dichloromethane (10 mL × 2). The organic phases were combined, washed with saturated brine (10 mL × 2), dried, and concentrated to dryness to obtain a crude product. The crude product was separated by high-performance liquid chromatography to obtain compound **50-3** (yellow liquid, 1.30 g).

Tetrahydrofuran (15 mL), 1-trifluoroethoxy-4-pyridinemethanamine (800 mg, 1.00 *eq),* and *N,N-*diisopropylethylamine (1.00 g, 2.00 *eq*) were sequentially added to a 100 mL round-bottomed flask, and the mixture was allowed to react at 20 °C for 15 min under a nitrogen atmosphere. After the reaction solution was cooled to 0 °C, carbonyldiimidazole (754 mg, 1.20 *eq*) was added, and then the mixture was stirred for 0.5 h. Tetrahydrofuran (10 mL) and a solution of compound **50-3** (1.30 g, 1.00 *eq*) were added, and the reaction solution was heated to 80 °C and stirred for 24 h until the reaction was completed. Water (20 mL) was then added to the reaction solution, and the mixture was extracted with ethyl acetate (30 mL × 2). The organic phases were combined, washed with 60 mL of saturated brine, dried, and concentrated to dryness to obtain a crude product. The crude product was separated and purified by high-performance liquid chromatography to obtain compound **50-4** (yellow liquid, 900 mg, yield: 40.9%).

Dichloromethane (8 mL), compound **50-4** (900 mg, 1.00 *eq*)*,* and trifluoroacetic acid (6.14 g, 33.9 *eq*) were sequentially added to a 100 mL three-necked flask, and the mixture was allowed to react at 10 °C for 1 h under a nitrogen atmosphere. The reaction solution was concentrated to dryness to obtain compound **50-5** (yellow liquid, 910 mg, crude product of trifluoroacetate), which was used directly in the next step.

Dichloromethane (20 mL), compound **50-5** (950 mg, 1.00 *eq,* trifluoroacetate), a formaldehyde solution (200 mg, 1.51 *eq*), and sodium cyanoborohydride (205 mg, 2.00 *eq*) were sequentially added to a 100 mL three-necked flask, and the mixture was stirred at 25 °C for 13 h. After the reaction solution was cooled to 0 °C, an aqueous sodium bicarbonate solution (20 mL) was added to quench the reaction, and the mixture was extracted with dichloromethane (30 mL × 2). The organic phases were combined, dried, and concentrated to dryness to obtain a crude product. The crude product was subjected to high-performance liquid chromatography and preparative chiral chromatography (chromatographic column: DAICEL CHIRALPAK AD (250 mm × 50 mm, 10 µm); mobile phase: [A: CO₂, B: (0.1% NH₃H₂O IPA)]; B%: 25%-25%) to obtain compounds **50A** (retention time: 1.259 min, yellow liquid, 90.0 mg, yield: 11.2%) and **50B** (retention time: 1.460 min, yellow liquid, 120 mg, yield: 14.8%).

**Compound 50A:** ¹H NMR (400 MHz, CDCl₃) *δ* 8.29 (d, *J =* 2.8 Hz, 1H), 8.03 (d, *J* = 2.0 Hz, 1H), 7.60 (dd, *J₁ =* 2.4 Hz *J₂* =8.4 Hz, 1H), 7.38 - 7.37 (m, 1H), 7.25 - 7.23 (m, 1H), 6.82 (d, *J =* 8.8 Hz, 1H), 6.53 (br, 1H), 4.79 - 4.72 (m, 2H), 4.44 - 4.26 (m, 5H), 2.99 -2.92 (m, 1H), 2.55 -2.54 (m, 1H) 2.27 (s, 3H), 2.20 -2.17 (m, 1H), 2.07 - 2.03 (m, 2H), 1.73 -1.70 (m, 1H), 0.57 -0.45 (m, 3H), 0.26 -0.24 (m, 1H). MS(ESI) m/z : 482.2 [M+1].

**Compound 50B:** ¹H NMR (400 MHz, CDCl₃) *δ* 8.29 (d, *J =* 2.8 Hz, 1H), 8.03 (d, *J =* 2.0 Hz, 1H), 7.60 (dd, *J₁ =* 2.4 Hz *J₂* =8.4 Hz, 1H), 7.38 - 7.37 (m, 1H), 7.26 - 7.23 (m, 1H), 6.82 (d, *J =* 8.8 Hz, 1H), 6.54 (br, 1H), 4.79 - 4.72 (m, 2H), 4.48 - 4.19 (m, 5H), 3.00 -2.92 (m, 1H), 2.58 -2.54 (m, 1H) 2.28 (s, 3H), 2.20 -2.18 (m, 1H), 2.11 - 2.04 (m, 2H), 1.73 -1.70 (m, 1H), 0.52 -0.47 (m, 3H), 0.27 -0.25 (m, 1H). MS(ESI) m/z : 482.2 [M+1].

### Example 17

Compounds 49A/49B and 83A/83B were obtained using a synthesis method and a resolution method similar to those in Example 16. The characterization data for compounds 49A/49B and 83A/83B are shown in the table below:

| Compound No./Structure | ¹H NMR | MS | SFC retention time |
|---|---|---|---|
| 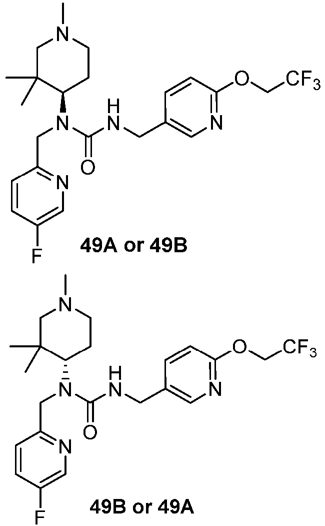 | Compound 49A: ¹H NMR (400 MHz, CDCl₃) *δ* 8.19 (d, *J =* 2.8 Hz, 1H), 7.86 (d, *J =* 2.0 Hz, 1H), 7.41 - 7.39 (m, 2H), 7.28 - 7.27 (m, 1H), 7.19 - 7.16 (m, 1H), 6.70 (d, *J =* 8.8 Hz, 1H), 6.15 (br, 1H), 4.69 - 4.63 (m, 2H), 4.29 - 4.17 (m, 5H), 2.87 -2.85 (m, 1H), 2.35 -2.32 (m, 1H) 2.15 (s, 3H), 1.99 - 1.97 (m, 2H), 1.87 - 1.85 (m, 1H), 1.45 -1.43 (m, 1H), 0.99 (s, 3H), 0.87 (s, 2H). | MS(ESI) m/z : 484.2[M+1]. | 1.114 min |
| | Compound 49B: ¹H NMR (400 MHz, CDCl₃) *δ* 8.19 (d, *J =* 2.8 Hz, 1H), 7.86 (d, *J =* 2.0 Hz, 1H), 7.41 - 7.39 (m, 2H), 7.28 - 7.27 (m, 1H), 7.19 - 7.16 (m, 1H), 6.70 (d, *J =* 8.8 Hz, 1H), 6.17 (br, 1H), 4.69 - 4.63 (m, 2H), 4.39 - 4.17 (m, 5H), 2.88 -2.87 (m, 1H), 2.37 -2.34 (m, 1H) 2.01 (s, 3H), 1.99 - 1.90 (m, 2H), 1.88 - 1.85 (m, 1H), 1.46 -1.43 (m, 1H), 1.00 (s, 3H), 0.84 (s, 2H). | MS(ESI) m/z : 484.2[M+1]. | 1.468 min |
| 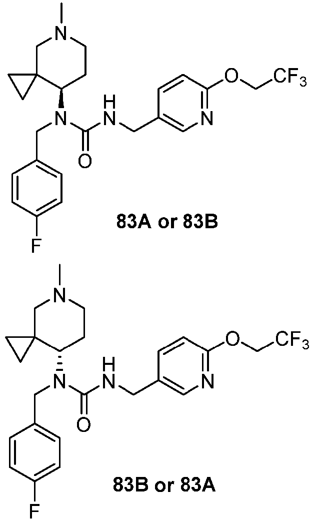 | Compound 83A: ¹H NMR (400 MHz, CDCl₃) *δ* 7.89 (s, 1H), 7.38 (dd, *J =* 2.1, 8.6 Hz, 1H), 7.14 - 7.09 (m, 2H), 7.01 (br t, *J =* 8.5 Hz, 2H), 6.77 (d, *J* = 8.4 Hz, 1H), 5.01 (br d, *J =* 10.9 Hz, 1H), 4.79 (br s, 1H), 4.74 (q, *J =* 8.6 Hz, 2H), 4.56 - 4.46 (m, 1H), 4.36 (br s, 1H), 4.33 - 4.17 (m, 3H), 3.59 - 3.51 (m, 1H), 3.43 (br d, *J =* 12.6 Hz, 1H), 3.04 - 2.95 (m, 1H), 2.76 (s, 3H), 2.66 (br d, *J =* 12.0 Hz, 1H), 2.43 - 2.32 (m, 1H), 1.94 (br dd, *J* = 2.5, 14.1 Hz, 1H), 0.89 - 0.84 (m, 2H), 0.71 (br d, *J* = 6.3 Hz, 1H), 0.50 - 0.45 (m, 1H). | MS(ESI) m/z : 481.3 [M+1]. | 1.272 min |
| | Compound 83B: ¹H NMR (400 MHz, CDCl₃) *δ* 7.91 (d, *J* = 2.1 Hz, 1H), 7.44 (dd, *J =* 2.4, 8.5 Hz, 1H), 7.11 (dd, *J =* 5.2, 8.6 Hz, 2H), 7.02 - 6.96 (m, 2H), 6.78 (d*, J =* 8.5 Hz, 1H), 4.74 (q, *J =* 8.6 Hz, 2H), 4.62 (br t, *J* = 5.6 Hz, 1H), 4.55 (br d, *J =* 10.0 Hz, 1H), 4.39 (br d, *J =* 5.3 Hz, 2H), 4.28 (dd, *J =* 5.8, 13.0 Hz, 2H), 3.00 (br d, *J* = 10.0 Hz, 1H), 2.64 (br d, *J =* 11.9 Hz, 1H), 2.31 (s, 3H), 2.28 - 2.23 (m, 1H), 2.12 (br d, *J* = 11.8 Hz, 1H), 2.05 - 1.95 (m, 1H), 1.79 - 1.72 (m, 1H), 0.57 - 0.48 (m, 3H), 0.32 - 0.26 (m, 1H). | MS(ESI) m/z : 481.3 [M+1]. | 1.470 min |

### Example 18

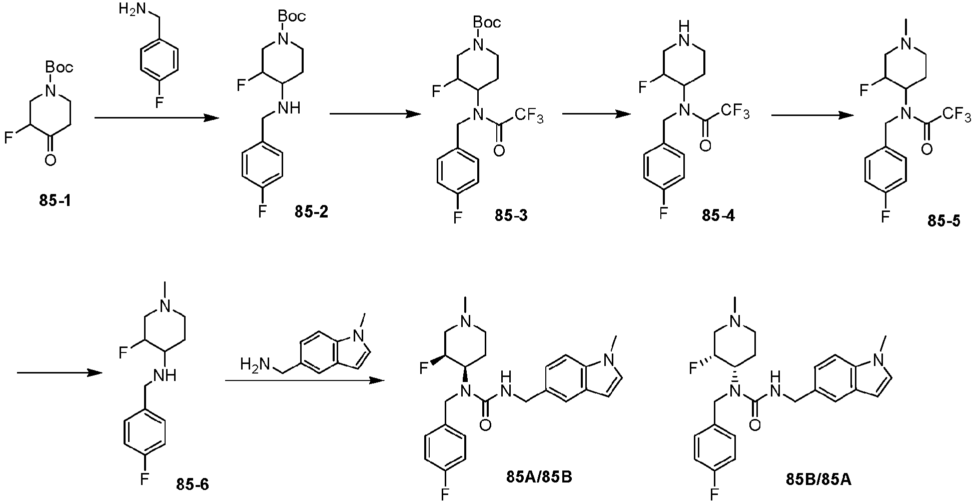

Dichloromethane (110 mL), compound 85-1 (22.0 g, 1.00 *eq*)*,* and compound p-fluorophenethylamine (14.0 g, 1.10 *eq)* were sequentially added to a 250 mL three-necked flask, and the mixture was stirred at 20 °C for 6 h. After the reaction was completed, sodium triacetoxyborohydride (32.3 g, 1.50 *eq*) was added, and the mixture was stirred at 20 °C for another 12 h. After the reaction was completed, 80 mL of water was added to the reaction solution, and the mixture was extracted with dichloromethane (80 mL × 2).The organic phases were combined, dried over anhydrous sodium sulfate, filtered, and then concentrated to dryness to obtain a crude product of compound 85-2 (yellow oily substance, 33.5 g, crude product).

Dichloromethane (670 mL), compound 85-2 (33.5 g, 1.00 *eq*)*,* triethylamine (28.6 mL, 2.00 *eq*)*,* trifluoroacetic anhydride (17.1 mL, 1.20 *eq*)*,* and 4-dimethylaminopyridine (6.27 g, 0.50 *eq*) were sequentially added to a 1 L three-necked flask, and the mixture was stirred at 20 °C for 1 h. After the reaction was completed, the mixture was washed with water (400 mL × 2). The organic phases were combined, dried, and concentrated to dryness to obtain a crude product. The crude product was purified by column chromatography to obtain compound **85-3** (yellow oily substance, 37.5 g, yield: 69.2%).

Dichloromethane (187 mL), compound **85-3** (37.5 g, 1.00 *eq*)*,* and trifluoroacetic acid (33.0 mL, 5.00 *eq*) were sequentially added to a 500 mL three-necked flask, and the mixture was stirred at 20 °C for 12 h. After the reaction was completed, the reaction solution was directly concentrated to dryness to obtain a crude product of compound **85-4** (yellow oily substance, 28.6 g, yield: 100%).

Tetrahydrofuran (286 mL), compound **85-4** (28.6 g, 1.00 *eq*), and an aqueous formaldehyde solution (33.1 mL, 37% purity, 5.00 *eq)* were sequentially added to a 500 mL three-necked flask, and sodium cyanoborohydride (11.2 g, 2.00 *eq*) was added in portions. The mixture was stirred at 20 °C for 1.5 h. After the reaction was completed, the mixture was adjusted to pH 8 with a saturated aqueous sodium carbonate solution and then extracted with dichloromethane (100 mL × 2). The organic phases were combined, dried over anhydrous sodium sulfate, filtered, and then concentrated to dryness to obtain a crude product of compound **85-5** (yellow oily substance, 34.5 g, yield: 87.7%).

Water (217 mL), compound **85-5** (32.5 g, 1.00 *eq*), and concentrated hydrochloric acid (325 mL, 12.0 M, 40.4 *eq*) were sequentially added to a 1 L three-necked flask, and the mixture was stirred at 100 °C for 12 h. After the reaction was completed, the mixture was adjusted to pH 8-9 with 156 g of sodium hydroxide and then extracted with dichloromethane (400 mL × 2). The organic phases were combined, dried over anhydrous sodium sulfate, filtered, and then concentrated to dryness to obtain a crude product. The crude product was subjected to chiral separation to obtain compound **85-6** (yellow oily substance, 2.19 g, crude product).

(1-Methyl-1*H*-indol-5-yl)methanamine (333 mg, 1.00 *eq*) was added to 5 mL of tetrahydrofuran, and diisopropylethylamine (672 mg, 906 µL, 2.50 *eq*) was added. The reaction solution was cooled to 0 °C, and carbonyldiimidazole (371 mg, 1.10 *eq*) was added. The mixture was stirred for one hour, and then a solution of compound **85-6** (500 mg, 1.00 *eq*) in 3 mL of tetrahydrofuran was added. The reaction solution was then heated to 70 °C and allowed to react for 12 h. After the reaction was completed, water was added, and the mixture was extracted with dichloromethane. The organic phases were combined, dried, and concentrated to dryness to obtain a crude product. The crude product was separated and purified by preparative chromatography to obtain compound 85 (white solid, 760 mg, yield: 85.6%), which was then further separated by SFC (chromatographic column: DAICEL CHIRALCEL OX (250 mm × 50 mm, 10 µm); mobile phase: [A: CO₂, B: (0.1% NH₃H₂O EtOH)]; B%: 25%-25%) to obtain **compound 85A** (retention time: 2.018 min, white solid, 266 mg, yield: 41.7%) and **compound 85B** (retention time: 2.461 min, white solid, 235 mg, yield: 36.3%).

**Compound 85A:** ¹H NMR (400 MHz, CDCl₃) *δ* 7.24 (s, 1H), 7.22 - 7.15 (m, 3H), 7.04 (d, *J* = 3.0 Hz, 1H), 6.98 (t, *J* = 8.6 Hz, 2H), 6.95 - 6.91 (m, 1H), 6.39 (d, *J =* 2.9 Hz, 1H), 5.04 - 4.86 (m, 1H), 4.68 - 4.61 (m, 2H), 4.57 (s, 1H), 4.49 (s, 1H), 4.45 - 4.41 (m, 2H), 3.77 (s, 3H), 3.19 (br t, *J =* 11.9 Hz, 1H), 3.03 - 2.95 (m, 1H), 2.40 (br d, *J* = 13.3 Hz, 1H), 2.34 (s, 3H), 2.24 - 2.16 (m, 2H), 1.66-1.61 (m, 1H). MS(ESI) m/z : 427.2 [M+1].

**Compound 85B:** ¹H NMR (400 MHz, CDCl₃) *δ* 7.24 (s, 1H), 7.21 - 7.15 (m, 3H), 7.05 - 7.03 (m, 1H), 7.01 - 6.96 (m, 2H), 6.94 - 6.91 (m, 1H), 6.39 (d, *J =* 2.9 Hz, 1H), 5.04 - 4.86 (m, 1H), 4.68 - 4.61 (m, 2H), 4.59 - 4.56 (m, 1H), 4.52 - 4.48 (m, 1H), 4.45 - 4.41 (m, 2H), 3.77 (s, 3H), 3.24 - 3.14 (m, 1H), 3.02 - 2.95 (m, 1H), 2.43 - 2.37 (m, 1H), 2.35 (s, 3H), 2.26 - 2.19 (m, 2H), 1.67-1.62 (m, 1H). MS(ESI) m/z : 427.2 [M+1].

### Example 19

Compounds 86A/86B and 87A/87B were obtained using a synthesis method and a resolution method similar to those in Example 18. The characterization data for compounds 86A/86B and 87A/87B are shown in the table below:

| Compound No./Structure | ¹H NMR | MS | SFC retention time |
|---|---|---|---|
| 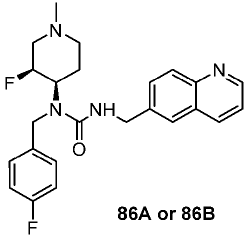 | Compound 86A: ¹H NMR (400 MHz, CDCl₃) *δ* 8.88 (br d, *J =* 3.1 Hz, 1H), 8.06 - 7.97 (m, 2H), 7.44 - 7.36 (m, 3H), 7.25 - 7.20 (m, 2H), 7.02 (t, *J =* 8.5 Hz, 2H), 4.91 - 4.82 (m, 2H), 4.63 (s, 1H), 4.57 - 4.49 (m, 3H), 3.24 - 3.15 (m, 1H), 2.99 (br d, *J =* 7.4 Hz, 1H), 2.39 (br d, *J =* 13.3 Hz, 1H), 2.34 (s, 3H), 2.29 (br d, *J =* 13.3 Hz, 1H), 2.25 - 2.18 (m, 2H), 1.69 - 1.61 (m, 1H). | MS(ESI) m/z : 425.2 [M+1]. | 1.709 min |
| 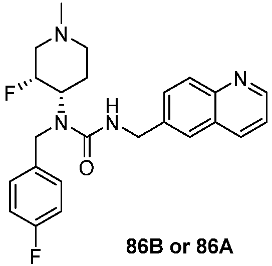 | Compound 86B: ¹H NMR (400 MHz, CDCl₃) *δ* 8.90 - 8.86 (m, 1H), 8.05 - 7.98 (m, 2H), 7.43 - 7.36 (m, 3H), 7.25 - 7.20 (m, 2H), 7.05 - 6.99 (m, 2H), 5.00 (br s, 1H), 4.89 - 4.83 (m, 2H), 4.65 - 4.62 (m, 1H), 4.56 - 4.49 (m, 3H), 3.22 - 3.14 (m, 1H), 3.01 - 2.95 (m, 1H), 2.37 (br d, *J =* 13.3 Hz, 1H), 2.33 (s, 3H), 2.27 (br d, *J =* 13.4 Hz, 1H), 2.23 - 2.18 (m, 2H), 1.69 - 1.60 (m, 1H). | MS(ESI) m/z : 425.2 [M+1]. | 2.030 min |
| 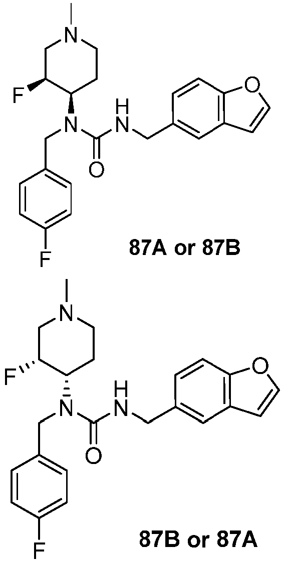 | Compound 87A: ¹H NMR (400 MHz, CDCl₃) *δ* 7.61 (d, *J =* 2.00 Hz, 1 H), 7.37 (d, *J =* 8.50 Hz, 1 H), 7.16 - 7.23 (m, 3 H), 6.96-7.06 (m, 4 H), 6.67 - 6.71 (m, 1 H), 4.86 - 4.99 (m, 1 H), 4.68 (br t, *J =* 5.13 Hz, 1 H), 4.46 - 4.65 (m, 2 H), 4.35 - 4.44 (m, 2 H), 3.49 (s, 1 H), 3.11 - 3.22 (m, 1 H), 2.90 - 3.00 (m, 1 H), 2.31 (s, 3 H), 2.12 - 2.24 (m, 2 H), 1.21 - 1.33 (m, 1 H). | MS(ESI) m/z : 414.2 [M+1]. | 2.034 min |
| | Compound 87B: ¹H NMR (400 MHz, CDCl₃) *δ* 7.60 (br s, 1 H), 7.37 (br d, *J*=7.50 Hz, 1 H), 7.20 (br s, 3 H), 6.96-7.06 (m, 4 H), 6.68 (br s, 1 H), 4.99 (br s, 1 H), 4.80 - 4.91 (m, 1 H), 4.33 - 4.74 (m, 5 H), 3.06 - 3.24 (m, 1 H), 2.94 (br s, 1 H), 2.31 (br s, 3 H), 2.11 - 2.24 (m, 2 H), 1.21 - 1.33 (m, 1 H). | MS(ESI) m/z : 414.2 [M+1]. | 2.340 min |

### Example 20

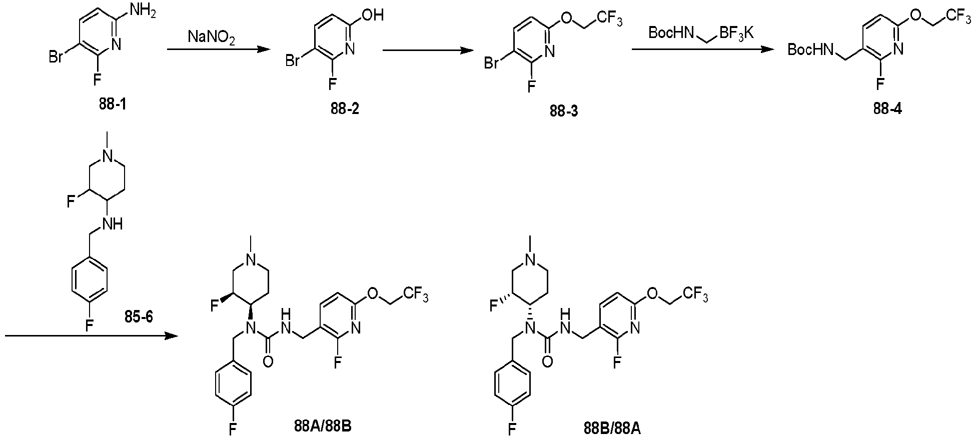

Compound **88-1** (8 g, 41.88 mmol, 1 *eq*) was added to hydrochloric acid (6 M, 120 mL, 17.2 *eq*) in a 500 mL round-bottomed flask at 0 °C. A solution of sodium nitrite (2.89 g, 41.88 mmol, 1 *eq*) in water (10 mL) was then added, and the mixture was stirred at 38 °C for 8 h. The reaction mixture was filtered, and the filter cake was concentrated under reduced pressure to obtain a residue. The residue was purified by column chromatography (petroleum ether/ethyl acetate = 1/0 to 3/1) to obtain compound **88-2** (3.5 g, 18.23 mmol, yield: 43.5%) as a white solid.

Trifluoroethyl trifluoromethanesulfonate (10.16 g, 43.75 mmol, 3 *eq*) was added to a mixed solution of compound **88-2** (2.8 g, 14.58 mmol, 1 *eq)* and cesium carbonate (9.50 g, 29.17 mmol, 2 *eq*) in dimethylaniline (28 mL). The mixture was stirred at 50 °C for 1 h. The reaction mixture was then filtered and washed with ethyl acetate (100 mL × 3). The organic layer was washed with a saturated aqueous sodium chloride solution (100 mL × 2), dried over anhydrous sodium sulfate, and filtered, and the filtrate was concentrated under reduced pressure to obtain a residue. The residue was purified by column chromatography (petroleum ether/ethyl acetate = 1/0 to 5/1) to obtain compound **88-3** (3 g, 10.95 mmol, yield: 75.1%) as a colorless oily substance.

Compound **88-3** (700 mg, 2.55 mmol, 1 *eq*)*,* potassium {[(tert-butoxycarbonyl)amino]methyl}trifluoroborate (726.75 mg, 3.07 mmol, 1.2 *eq*)*,* potassium phosphate (1.63 g, 7.66 mmol, 3 *eq*)*,* and mesylate[(di(1-adamantyl)-*n-*butylphosphine)-2-(2'-amino-1,1'-biphenyl)]palladium(II) (93.02 mg, 0.128 mmol, 0.05 *eq*) were dissolved in dioxane (10 mL) and water (2 mL), and the mixture was purged 3 times with nitrogen, then stirred at 80 °C for 1 h under a nitrogen atmosphere, and poured into water (100 mL). The aqueous phase was extracted with ethyl acetate (100 mL × 3). The organic phases were combined, washed with saturated brine (100 mL × 2), dried over anhydrous sodium sulfate, and filtered, and the filtrate was concentrated under vacuum. The residue was purified by column chromatography (petroleum ether/ethyl acetate = 1/0 to 5/1) to obtain compound **88-4** (0.7 g, 2.16 mmol, yield: 84.5%) as a light yellow solid.

2-Chloropyridine (315.14 mg, 2.78 mmol, 3 *eq*) and trifluoroacetic anhydride (391.53 mg, 1.39 mmol, 1.5 *eq*) were added to a solution of compound **88-4** (300 mg, 0.925 mmol, 1 *eq*) in dichloromethane (10 mL). The mixture was then stirred at 20 °C for 1 h, and then 3-fluoro-*N*-[(4-fluorophenyl)methyl]-1-methyl-piperidin-4-amine (489.07 mg, 2.04 mmol, 2.2 *eq*) was added at 20 °C. The resulting mixture was stirred at 20 °C for another 1 h and then poured into water (20 mL). The aqueous phase was extracted with ethyl acetate (20 mL × 3). The organic phase was washed with saturated brine (20 mL × 2), dried over anhydrous sodium sulfate, and filtered, and the filtrate was concentrated under vacuum. The residue was purified by column chromatography (dichloromethane/methanol = 1/0 to 10/1) and then further subjected to SFC resolution (chromatographic column: DAICEL CHIRALCEL OX (250 mm × 30 mm, 10 µm); mobile phase: [A: CO₂, B: (0.1% NH₃H₂O MeOH)]; B%: 15%-15%) to obtain **compound 88A** (retention time: 1.025 min, 123 mg, yield: 26.3%) as a white solid and compound 88B (retention time: 1.231 min, 116 mg, yield: 25.1%) as a white solid.

**Compound 88A:** ¹H NMR (400 MHz, DMSO-*d₆*) *δ* 7.76 - 7.64 (m, 1H), 7.25 - 7.15 (m, 2H), 7.15 - 7.05 (m, 2H), 6.99 (t, *J =* 5.6 Hz, 1H), 6.87 (d, *J* = 8.0 Hz, 1H), 4.92 (q, *J =* 9.2 Hz, 2H), 4.82 - 4.59 (m, 2H), 4.41 (d, *J =* 17.6 Hz, 1H), 4.29 - 4.08 (m, 3H), 2.97 (t, *J =* 12.0 Hz, 1H), 2.83 - 2.70 (m, 1H), 2.24 - 2.04 (m, 4H), 2.02 - 1.86 (m, 2H), 1.41 - 1.22 (m, 1H). MS(ESI) m/z : 491.3 [M+1].

**Compound 88B:** ¹H NMR (400 MHz, DMSO-*d₆*) *δ* 7.70 (t, *J =* 8.8 Hz, 1H), 7.25 - 7.15 (m, 2H), 7.14 - 7.04 (m, 2H), 7.03 - 6.93 (m, 1H), 6.87 (d, *J =* 8.0 Hz, 1H), 4.92 (q*, J =* 8.8 Hz, 2H), 4.82 - 4.58 (m, 2H), 4.41 (d*, J =* 18.0 Hz, 1H), 4.30 - 4.05 (m, 3H), 2.97 (t, *J =* 11.6 Hz, 1H), 2.82 - 2.70 (m, 1H), 2.24 - 2.04 (m, 4H), 2.02 - 1.85 (m, 2H), 1.41 - 1.20 (m, 1H). MS(ESI) m/z : 491.3 [M+1].

### Example 21

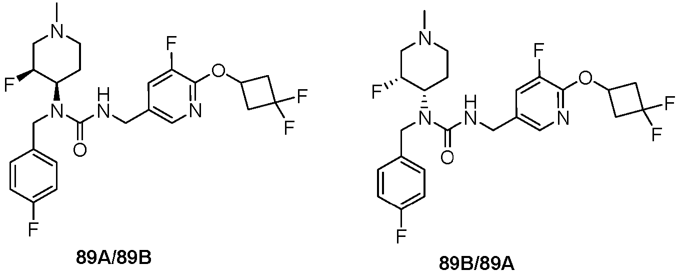

**Compound 89A** (retention time: 0.994 min, white solid) and **compound 89B** (retention time: 1.055 min, white solid) were obtained using a synthesis method and a resolution method similar to those in Example 20.

**Compound 89A:** ¹H NMR (400 MHz, CDCl₃) *δ* 8.03 (d, *J =* 3.3 Hz, 1H), 7.56 (s, 1H), 7.14 - 7.07 (m, 3H), 6.96 (br t, *J =* 8.5 Hz, 2H), 4.92 - 4.74 (m, 1H), 4.66 - 4.56 (m, 2H), 4.54 - 4.34 (m, 3H), 4.13 (br s, 3H), 4.08 - 4.02 (m, 1H), 3.08 (br dd, *J =* 7.9, 12.8 Hz, 1H), 2.76 - 2.66 (m, 2H), 2.29 - 2.23 (m, 4H), 2.20 - 2.09 (m, 2H), 1.41 - 1.22 (m, 1H). MS(ESI) m/z: 499.2 [M+1].

**Compound 89B:** ¹H NMR (400 MHz, CDCl₃) *δ* 8.11 (d*, J* = 3.3 Hz, 1H), 7.67 - 7.61 (m, 1H), 7.23 - 7.11 (m, 2H), 7.09 - 6.95 (m, 3H), 5.01 - 4.81 (m, 1H), 4.70 (s, 1H), 4.66 - 4.42 (m, 3H), 4.33 - 4.21 (m, 3H), 3.39 (t, *J =* 7.1 Hz, 1H), 3.26 - 3.05 (m, 3H), 3.02 (br dd, *J =* 1.9, 3.9 Hz, 1H), 2.89 - 2.71 (m, 2H), 2.20 - 2.09 (m, 4H), 1.41 - 1.22 (m, 1H). MS(ESI) m/z: 499.2 [M+1].

### Example 22

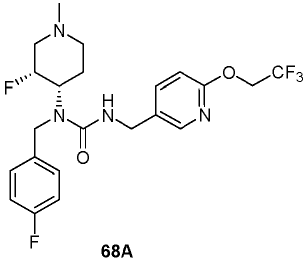

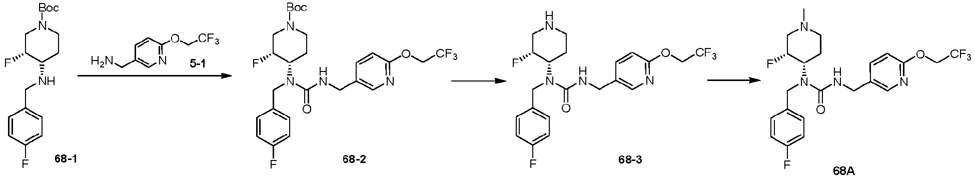

Tetrahydrofuran (15 mL) and compound 5-1 (1.04 g, 1.00 *eq,* HCl) were sequentially added to a 100 mL three-necked flask, and diisopropylethylamine (1.11 g, 2.00 *eq)* was added dropwise under a nitrogen atmosphere. The mixture was stirred at 20 °C for 15 min. The reaction solution was then cooled to 0 °C, and *N,N'*-carbonyldiimidazole (765 mg, 1.10 *eq)* was added thereto in portions. The reaction was monitored to find that compound 5-1 had completely reacted. Compound **68-1** (1.40 g, 1.00 *eq)* was dissolved in tetrahydrofuran (5 mL), and the resulting solution was slowly added dropwise to the reaction solution. The mixture was incubated with stirring at 25 °C for 12 h. Water (100 mL) was added to the reaction solution, and the mixture was extracted with ethyl acetate (50 mL × 3). The organic phases were combined, washed with 80 mL of saturated brine, dried, and concentrated to dryness to obtain a crude product. The crude product was purified by column chromatography to obtain compound **68-2** (colorless oily substance, 966 mg, yield: 38.8%).

Anhydrous dichloromethane (27 mL), compound **68-2** (966 mg, 1.00 *eq*)*,* and trifluoroacetic acid (15.4 g, 78.0 *eq*) were sequentially added to a 50 mL single-necked flask, and the mixture was incubated with stirring at 25 °C for 20 min. The reaction solution was concentrated to dryness by rotary evaporation to obtain a crude product of compound **68-3** (light yellow oily substance, 850 mg, yield: 91.4%).

Tetrahydrofuran (8 mL), compound **68-3** (800 mg, 1.00 *eq*)*,* sodium cyanoborohydride (219 mg, 2.00 *eq*)*,* and formaldehyde (212 mg, 37%, 1.50 *eq*) were sequentially added to a 50 mL single-necked flask. The mixture was incubated with stirring at 25 °C for 12 h. Water (50 mL) was added to the reaction solution, and the mixture was extracted with ethyl acetate (30 mL × 3). The organic phases were combined, washed with 40 mL of saturated brine, dried, and concentrated to dryness, and the residue was purified by column chromatography (petroleum ether/ethyl acetate = 1/0 to 5/1) to obtain **compound 68A** (white solid, 643 mg, yield: 78.1%). ¹H NMR (400 MHz, CDCl₃) *δ* 7.87 (d*, J =* 1.9 Hz, 1H), 7.35 (dd, *J* = 2.3, 8.5 Hz, 1H), 7.17 - 7.08 (m, 2H), 7.00 (br t, *J =* 8.5 Hz, 2H), 6.75 (d, *J =* 8.5 Hz, 1H), 5.17 - 4.99 (m, 1H), 4.94 (br t, *J =* 5.1 Hz, 1H), 4.71 (d, *J =* 8.6 Hz, 2H), 4.56 - 4.43 (m, 2H), 4.30 - 4.17 (m, 2H), 3.81 - 3.57 (m, 2H), 3.22 - 2.91 (m, 2H), 2.80 (s, 3H), 2.60 - 2.45 (m, 1H), 1.79 (br d, *J =* 11.8 Hz, 1H). MS(ESI) m/z : 473.2 [M+1].

### Test Example 1. Assay for 5-HT_{2A} Receptor Inverse Agonist Activity

### 1.1 Experimental materials:

Cell line: adherent cells NIH3T3-5-HT_{2A} R
Cell culture medium: DMEM + 10% FBS (purchased from GBICO)
Cell culture plate: 96-well white/clear bottom plate (purchased from Perkin Elmer)
Detection kit: Bright-Glo^{™} Luciferase (purchased from Promega)
Detection instrument: BioTek multimode microplate reader

### 1.2 Test drugs

Pimavanserin: purchased from MCE (MedChemExpress)
Other compounds: prepared according to the previous examples

### 1.3 Experimental method:

NIH₃T3-5HT_{2A} R cells in the logarithmic growth phase were seeded into a 96-well white/clear bottom plate at a density of 1000 cells/well and then cultured overnight in an incubator at 37 °C with 5% CO₂. The next day, the test compound was diluted with PBS at a 3.16-fold concentration gradient to obtain 9 concentrations (the highest concentration of the test compound was 10 µM) and added to the cells. Duplicate wells were set up for each concentration. PBS was used as a negative control group, and pimavanserin at the same concentration was used as a positive control group. After the drug addition, the cells were cultured in the incubator at 37 °C with 5% CO₂ for another 120 h. On the sixth day, a Bright-Glo^{™} Luciferase reagent was added to the cells in a volume equal to that of the cell culture medium. The plate was then incubated in the dark at room temperature for 20 min, with the plate shaker vibrating once every 5 min. The luminescence intensity was detected using a microplate reader, and the cell inhibition rate was calculated. Data were processed using GraphPad Prism 7.0 to obtain the cell inhibition rate curve and calculate IC₅₀. The experimental results are shown in Table 1. $Cell inhibition rate \left(%\right) = \left[100-\left({Lum}_{test drug}-{Lum}_{culture medium}\right)/\left({Lum}_{cell control}-{Lum}_{culture medium}\right)\right] \times 100 %$

### Test Example 2. hERG Inhibitory Activity

### 1. Test materials and instruments

### 1.1 Positive control compound

Name: cisapride

### 1.2 Vehicle

Name: DMSO (dimethyl sulfoxide)

### 1.3 Cells

Species & strain: CHO-hERG cell line (Chinese hamster ovary cells stably expressing hERG channels)
Culture medium: 90% F12, 10% fetal bovine serum, 100 µg/mL G418, and 100 µg/mL Hygromycin B
Culture conditions: incubator at 37 °C with 5% CO₂
Cryopreservation condition: liquid nitrogen

### 1.4 Experimental instruments

Patch clamp amplifier (Axoclamp 200B, Multiclamp 700B, Axon, USA)
Digital-to-analog converter (DigiData 1440A, DigiData 1550B, Axon, USA)
Inverted microscope (IX51, IX71, Olympus, Japan)
Rapid administration system (RSC-200, Bio-Logic, France)
Micromanipulator (MX7600R, Syskiyou, USA)
Electrode puller (P-97, Sutter, USA)
Glass electrode (BF150-86-10, Sutter, USA)
Vibration isolation table and shielding mesh (63-534, TMC, USA)
Data acquisition and analysis software (pClamp 10, Axon, USA)
Carbon dioxide incubator (HERAcell 150i, Thermo, USA)
Biosafety cabinet (MODEL 1384, Thermo, USA)
Water purification system (Milli Q, Millipore, USA)

### 2. Experimental method

### 2.1 Cell culture and treatment

CHO cells stably expressing hERG were cultured in a cell culture dish with a diameter of 35 mm and incubated in an incubator at 37 °C with 5% CO₂. The cells were passaged at a ratio of 1:5 every 48 h. On the day of the test, the cell culture medium was aspirated, and the cells were rinsed once with the extracellular fluid. Then, a 0.25% Trypsin-EDTA (Invitrogen) solution was added, and the cells were digested at room temperature for 3-5 min. The digestion solution was then aspirated, and the cells were resuspended in the extracellular fluid and transferred to an experimental dish for electrophysiological recording for later use.

### 2.2 Compound preparation

On the day of the test, the compound was diluted with DMSO to an intermediate concentration. Then, 10 µL of the intermediate-concentration compound was transferred to 4990 µL of extracellular fluid, achieving a 500-fold dilution to obtain the final concentration to be tested.

Preparation of positive control compound cisapride: 10 µL of 150 µM cisapride DMSO mother solution was transferred to 4990 µL of extracellular fluid, achieving a 500-fold dilution to obtain the final concentration (300 nM) to be tested.

### 2.3 Electrophysiological recording process

hERG potassium channel currents were recorded at room temperature by the whole-cell patch clamp technique in the CHO (Chinese hamster ovary) cells stably expressing hERG potassium channels. Glass microelectrodes were formed by pulling a glass electrode blank (BF150-86-10, Sutter) using a puller. The tip resistance after perfusion of electrode internal solution was about 2-5 MΩ. The glass microelectrodes could be connected to a patch clamp amplifier after being inserted into amplifier probes. The clamping voltage and data recording were controlled and recorded by a computer with a pClamp 10 software, with a sampling frequency of 10 kHz and a filtering frequency of 2 kHz. After achieving the whole-cell recording, the cells were clamped at -80 mV. The step voltage evoking hERG potassium current (I_{hERG}) was changed from -80 mV to +20 mV by applying a 2 s depolarization voltage, followed by repolarization to -50 mV for 1 s, and then returned to -80 mV. This voltage stimulation was applied every 10 s, and after it was determined that the hERG potassium currents were stable (1 min), the administration process was started. Each test concentration of the compound was applied for at least 1 min, and at least 2 cells (n ≥ 2) were tested for each concentration.

### 2.4 Data processing and analysis

Data analysis was performed using the pClamp 10 software and GraphPad Prism 5.0 software.

The degree of inhibition of hERG potassium current (the peak hERG tail current evoked at -50 mV) by different compound concentrations was calculated using the following formula:$Inhibition % = \left[1-\left(I/Io\right)\right] \times 100 %$ where Inhibition % represents the percentage of inhibition of hERG potassium current by the compound, and I and Io represent the amplitudes of hERG potassium current after administration and before administration, respectively.

The IC₅₀ of the compound was calculated using the GraphPad Prism 5 software by equation fitting as follows, with the test results shown in Table 1: $Y = Bottom + \left(Top-Bottom\right) / \left(1+{10}^{∧}\left(\left({LogIC}_{50}-X\right)\times HillSlope\right)\right)$ where X is the Log value of the test concentration of the test sample, Y is the inhibition percentage at the corresponding concentration, and Bottom and Top are the minimum and maximum inhibition percentages, respectively.

### 3. Test results

**Table 1: In-vitro test results for the compounds of the present disclosure**

| Compound No. | 5-HT_{2A} inverse agonist activity IC₅₀ (nM) | hERG inhibitory activity IC₅₀ (µM) | Compound No. | 5-HT_{2A} inverse agonist activity IC₅₀ (nM) | hERG inhibitory activity IC₅₀ (µM) |
|---|---|---|---|---|---|
| Pimavanserin | 344 | 1.14 | 59 | 86.1 | 1.48 |
| 1 | 152 | 4.93 | 60 | 64.4 | - |
| 2 | 72.8 | - | 61 | 79.2 | - |
| 3 | 149.3 | - | 66 | 22.74 | 2.99 |
| 4 | >1000 | - | 67 | >1000 | - |
| 5 | 13.1 | - | 68A | 76.4 | 5.19 |
| 6 | 25.4 | - | 68B | > 1000 | - |
| 7 | >1000 | 1.32 | 68C | > 1000 | - |
| 8 | >1000 | - | 68D | > 1000 | - |
| 9 | 28.2 | 3.12 | 69A | > 1000 | - |
| 10 | 29.6 | 3.86 | 69B | 155.9 | - |
| 11 | 672.3 | - | 70A | > 1000 | - |
| 12 | 163.0 | 2.22 | 70B | 280.1 | - |
| 13 | 184 | - | 72A | 415.6 | - |
| 19 | 33.15 | - | 72B | 43.7 | - |
| 27 | - | >30 | 77 | > 1000 | - |
| 28 | - | >30 | 79 | 770.6 | - |
| 30 | - | 22.02 | 81A | > 1000 | - |
| 39 | > 1000 | 22.43 | 81B | > 1000 | - |
| 41 | 128.3 | 10.12 | 82A | > 1000 | - |
| 42 | - | 6.19 | 82B | 663.9 | - |
| 44 | - | 4.35 | 83A | 10.4 | - |
| 46A | > 1000 | - | 83B | 211 | - |
| 46B | - | - | 84 | > 1000 | - |
| 47A | - | 10.49 | 85A | >1000 | - |
| 47B | - | 10.63 | 85B | 88.7 | - |
| 49A | 105.5 | - | 86A | >1000 | - |
| 49B | > 1000 | - | 86B | 36.8 | - |
| 50A | 2.6 | 2.2 | 87A | 664.7 | - |
| 50B | 380.8 | - | 87B | 13.7 | - |
| 56A | > 1000 | 15.46 | 88A | 142.0 | - |
| 56B | 324.9 | 12.57 | 88B | >1000 | - |
| 57 | 51.9 | 1.44 | 89A | 140.3 | - |
| 58 | 41 | - | 89B | >1000 | - |

The results show that: the multiple compounds of the present disclosure had superior 5-HT_{2A} inverse agonist activity compared to pimavanserin, and had lower cardiotoxicity.

### Test Example 3. Evaluation of In-Vitro Liver Microsomal Stability

### 1. Solution preparation

1) Preparation of test sample working solution: A test sample was diluted to 100 µM with methanol.
2) Preparation of liver microsome working solution: Liver microsomes were diluted to 0.56 mg/mL with 100 mM phosphate-buffered saline.
3) Preparation of working solution of reduced nicotinamide adenine dinucleotide phosphate (NADPH): A proper amount of NADPH was weighed out and diluted to 20 mM with phosphate-buffered saline, and then an equal volume of a 60 mM MgCl₂ solution was added.
4) Preparation of stop solution: Tolbutamide was diluted to 20 ng/mL with acetonitrile to serve as a stop solution containing an internal standard.

### 2. Incubation process

1) Anti-adsorption EP tubes for incubation were prepared and labeled with species, test samples, control samples (testosterone and dextromethorphan), time points (0 min, 5 min, 10 min, 20 min, 30 min, 60 min, Blank60, and NCF60), etc.
2) 2 µL of test or control sample working solution and 178 µL of liver microsome working solution were added to each tube. For the Blank tube, 2 µL of acetonitrile was added instead of the test sample. The tubes were preincubated in a water bath at 37 °C for about 10 min, with 3 replicates set up for each sample.
3) After the pre-incubation was completed, 20 µL of NADPH working solution was added to each tube except for the 0 min and NCF60 tubes to start the reaction. For the NCF60 tube, 20 µL of phosphate-buffered saline (containing 30 mM MgCl₂) was added. In the incubation system, the final concentration of the test or control sample was 1 µM, the final concentration of liver microsomes was 0.5 mg/mL, the final concentration of NADPH was 1 mM, and the final concentration of MgCl₂ was 3 mM.
4) For the 0 min sample, 600 µL of stop solution was added, followed by the addition of the NADPH working solution. For the other samples, after each of them was incubated for the corresponding time, 600 µL of stop solution was added to stop the reaction.
5) After the reaction was stopped, each sample was vortexed for 30 s and then centrifuged at 13500 rpm for 10 min. Then, 100 µL of the supernatant was transferred to an EP tube, and 100 µL of Milli-Q water was added. The mixture was well mixed by vortexing and analyzed by LC-MS/MS.
6) Testosterone and dextromethorphan were used as positive control samples under the same conditions to assess the stability and reliability of the system.

### 3. Data analysis

The remaining percentage of the test sample after 60 min was tested, and the test results are shown in Table 2.

**Table 2: Test data for the compounds of the present disclosure in human and rat liver microsomes**

| Compound | Species | Remaining amount% at 60 min | Compound | Species | Remaining amount% at 60 min |
|---|---|---|---|---|---|
| Pimavanserin | Dog | 68.5 | Compound 30 | Rat | 71.34 |
| | Human | 70.7 | | Human | 45.97 |
| Compound 1 | Dog | 88.7 | Compound 39 | Rat | 25.13 |
| | Human | 85.7 | | Human | 48.30 |
| Compound 2 | Dog | 76.7 | Compound 46A | Dog | 40.77 |
| | Human | 79.7 | | Human | 1.21 |
| Compound 3 | Dog | 100.0 | Compound 46B | Dog | 1.05 |
| | Human | 94.1 | | Human | 5.34 |
| Compound 5 | Dog | 99.8 | Compound 50A | Rat | 58.8 |
| | Human | 99.9 | | Human | 89.7 |
| Compound 6 | Dog | 98.8 | Compound 66 | Dog | 85.1 |
| | Human | 90.8 | | Human | 73.7 |
| Compound 9 | Dog | 45.3 | Compound 67 | Dog | 81.4 |
| | Human | 93.1 | | Human | 66.2 |
| Compound 10 | Dog | 58.6 | Compound 68A | Dog | 81.75 |
| | Human | 71.9 | | Human | 42.89 |
| Compound 12 | Rat | 69.21 | Compound 68B | Dog | 69.33 |
| | Human | 77.06 | | Human | 30.07 |
| Compound 19 | Dog | 78.39 | Compound 68C | Dog | 23.5 |
| | Human | 72.49 | | Human | 20.2 |
| Compound 27 | Rat | 63.98 | Compound 68D | Dog | 19.2 |
| | Human | 50.93 | | Human | 11.9 |
| Compound 28 | Rat | 54.78 | | | |
| | Human | 38.94 | | | |

The results show that: the multiple compounds of the present disclosure had superior *in-vitro* stability in dog and human liver microsomes compared to pimavanserin, and had better druggability.

### Test Example 4. Evaluation of In-vivo Pharmacodynamics

### 1. Experimental protocol

Based on the *in-vivo* pharmacokinetic data for the series of compounds, the drug reached Cₘₐₓ 1.0-1.5 h after single intragastric administration in SD rats. At this time point, 4-iodo-2,5-dimethoxy-α-methyl-phenethylamine hydrochloride (i.e., DOI, a 5-HT_{2A} receptor agonist) was intraperitoneally injected into the SD rats at a dose of 2.5 mg/kg to induce head twitch behavior in the SD rats, thereby establishing a model.

The low, medium and high dose groups for compound 68A were set to 0.22, 0.66, and 2.0 mg/kg, respectively. The dose of pimavanserin tartrate (Pim-T) was set at 0.7 mg/kg. The routes of administration of compound 68A and Pim-T were both oral intragastric administration.

### 2. Experimental grouping and administration

The compounds were dissolved in DMSO:20% solutol (5%:95%). SD rats (200-250 g) were randomly divided into the following 6 groups according to the body weight: a vehicle control (Control) group, a model (Model) group, a Pim-T 0.7 mg/kg group, and compound 68A 0.22, 0.66, and 2.0 mg/kg groups, with 7 animals in each group. Information on animal grouping and administration is detailed in Table 3.

**Table 3: Animal grouping and administration**

| Group No. | Group | Type of drug | Administration dose (mg/kg) | Drug concentration (mg/mL) | Route of administration | Administration volume (mL/200 g) | Animal Number |
|---|---|---|---|---|---|---|---|
| 1 | Control | DMSO:20% solutol (5%:95%) | -- | -- | Intragastric | 1 | 7 |
| 2 | Model | DMSO:20% solutol (5%:95%) | -- | -- | Intragastric | 1 | 7 |
| 3 | Pim-T | Pim-T | 0.70 | 0.140 | Intragastric | 1 | 7 |
| 4 | 68A-0.22 | 68A | 0.22 | 0.044 | Intragastric | 1 | 7 |
| 5 | 68A-0.66 | 68A | 0.66 | 0.133 | Intragastric | 1 | 7 |
| 6 | 68A-2.0 | 68A | 2.00 | 0.400 | Intragastric | 1 | 7 |

| | | | | | | | |
|---|---|---|---|---|---|---|---|
| Note: The doses of Pim-T (pimavanserin tartrate) and compound 68A were calculated based on the free base. | | | | | | | |

### 3. Experimental procedures

The rats were fasted starting at 5:00 PM the day before the experiment. On the day of the experiment, the animals were acclimatized in the testing laboratory for at least 1 h. Rats in each group were subjected to single intragastric administration at the corresponding dose listed in Table 3. One hour after administration, all groups except for the Control group were intraperitoneally injected with DOI at 2.5 mg/kg, while the Control group received an equal volume of normal saline. The behavior of the rats was observed immediately after the injection. The observations were conducted in a randomized, double-blind manner to eliminate interference of human factors. The number of head twitches in the rats was recorded within 1.0-1.5 h after the administration.

Experimental data were expressed as mean ± standard deviation (Mean ± SEM). One-way analysis of variance (ANOVA) was performed using an SPSS statistics 20.0 software to compare differences between the groups at each time point. All tests were bilateral tests, and *P* < 0.05 indicates statistical significance.

### 4. Data analysis

Compared to the Control group, the number of head twitches in the rats in the model group was significantly increased (P < 0.05). Compared to the model group, the number of head twitches in the rats in the Pim-T 0.7 mg/kg group was significantly reduced (P < 0.05), and the number of head twitches in the rats in the compound 68A 0.22, 0.66, and 2.0 mg/kg groups was extremely significantly reduced. (P < 0.01). At equimolar doses, the number of head twitches in the compound 68A 0.66 mg/kg group was lower than that in the Pim-T 0.7 mg/kg group. The specific results are shown in Table 4 and FIG. 1.

**Table 4: Number of head twitches in SD rats 1.0-1.5 h after single intragastric administration**

| Group No. | Group | Number of head twitches |
|---|---|---|
| 1 | Control | 0 |
| 2 | Model | 29.9 ± 5.3 ^{#} |
| 3 | Pim-T | 14.6 ± 3.6 * |
| 4 | 68A-0.22 | 11.9 ± 5.1 ** |
| 5 | 68A-0.66 | 3.7 ± **1.6** ** |
| 6 | 68A-2.0 | 0.9 ± 0.6 ** |

| | | |
|---|---|---|
| Note: ^{#} indicates *P* < 0.05, as compared to the Control group; * indicates P < 0.05 and ** indicates P < 0.01, as compared to the Model group. | | |

The results show that: 1.0-1.5 h after single intragastric administration in SD rats, compound 68A could significantly reduce the number of head twitches in the rats within the dose range of 0.22-2.0 mg/kg, with the effective dose being 0.22 mg/kg. At equimolar doses, the pharmacodynamic effect of compound 68A was superior to that of Pim-T.

### Test Example 5. Evaluation of In-Vivo Tissue Distribution

### 1. Experimental protocol

SD rats (200-250 g) were subjected to single intragastric administration of compound 68A at a dose of 11 mg/kg. Plasma and brain, heart, liver and lung tissue samples were collected at 0.25 h, 1 h, and 6 h after administration, with 3 animals used for each time point.

### 2. Experimental procedures

The rats were fasted starting at approximately 6:00 PM the day before the experiment, with free access to water during the fasting period. On the day of the experiment, the rats were weighed and randomly divided into 3 groups according to the body weight, with one group of animals assigned to each time point. The compounds were dissolved in DMSO:20% solutol (5%:95%). The rats were subjected to single intragastric administration of compound 68A at a dose of 11 mg/kg, with an administration volume of 5 mL/kg and a drug solution concentration of 2.2 mg/mL. At each sampling time point, the animals were anesthetized with ether, and about 1 mL of blood was collected from the heart and then placed in a heparinized EP tube. The blood sample was centrifuged at 10000 rpm for 10 min to separate the plasma. After cardiac perfusion to remove blood, the brain, heart, fiver, and lung tissues were collected. The blood stains on the tissues were blotted dry with filter paper, and the tissues were separately weighed, then wrapped in weighing paper, and stored at -80 °C for further assay.

### 3. Sample assay and data analysis

Plasma samples and tissue homogenates (each tissue was homogenized in water at a weight-to-volume ratio of 1:4) were pretreated and then analyzed by LC-MS/MS to determine the concentrations of the test substance in plasma and tissues. Experimental data were expressed as mean ± standard deviation (Mean ± SD), and the specific results are shown in Table 5.

**Table 5: Plasma and tissue concentrations after single intragastric administration of compound 68A in SD rats**

| Plasma/tissue | hour | Mean ± SD | CV (%) | Ratio to plasma |
|---|---|---|---|---|
| Plasma | 0.25 | 338 ± 70.5 | 20.9 | / |
| | 1 | 247 ± 90.4 | 36.6 | / |
| | 6 | 274 ± 62.6 | 22.8 | / |
| Brain | 0.25 | 3216 ± 1428 | 44.4 | 9.51 |
| | 1 | 2376 ± 872 | 36.7 | 9.63 |
| | 6 | 2871 ± 624 | 21.7 | 10.5 |
| Heart | 0.25 | 1544 ± 382 | 24.7 | 4.57 |
| | 1 | 963 ± 387 | 40.2 | 3.90 |
| | 6 | 1137 ± 270 | 23.8 | 4.16 |
| Liver | 0.25 | 11787 ± 1664 | 14.1 | 34.9 |
| | 1 | 4260 ± 1255 | 29.5 | 17.3 |
| | 6 | 5243 ± 1477 | 28.2 | 19.2 |
| Lung | 0.25 | 9440 ± 3776 | 40.0 | 27.9 |
| | 1 | 8067 ± 849 | 10.5 | 32.7 |
| | 6 | 8200 ± 1601 | 19.5 | 30.0 |

The results show that: at 0.25 h, 1 h, and 6 h after single intragastric administration in SD rats, the concentrations of compound 68A in tissues were higher than that in plasma, indicating that compound 68A had good permeability; particularly, its distribution in brain tissue was relatively high, with a brain-to-plasma ratio of 9.51-10.5, and the concentration in the brain remained stable between 0.25 h and 6 h.

## Claims

1. A compound of formula (A) or a pharmaceutically acceptable salt, a stereoisomer or a deuteride thereof: 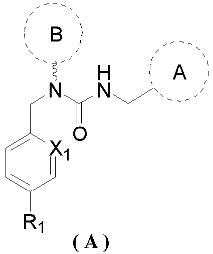
wherein R₁ is halogen;
ring B is selected from the group consisting of 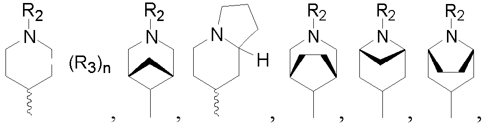 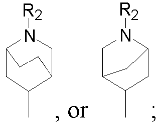
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 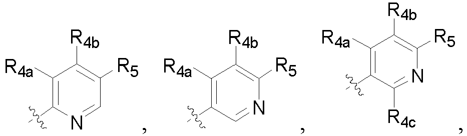 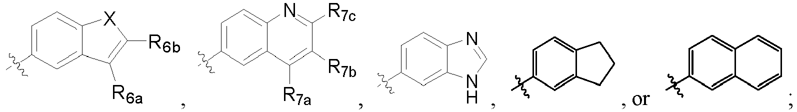
X is selected from the group consisting of NR_{6c}, O or S;
X₁ is selected from the group consisting of N or CH;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is selected from the group consisting of -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

2. The compound or the pharmaceutically acceptable salt, the stereoisomer or the deuteride thereof according to claim 1, being a compound of formula (I) or a pharmaceutically acceptable salt, a stereoisomer or a deuteride thereof: 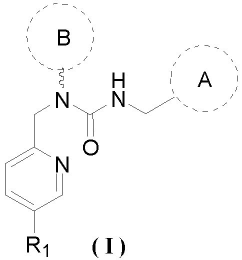 wherein
R₁ is halogen;
ring B is selected from the group consisting of 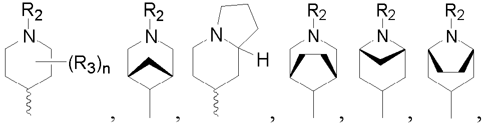 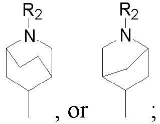
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 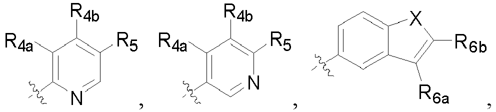 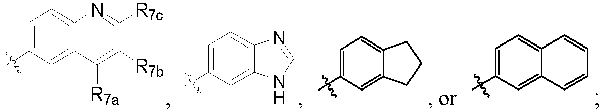
X is selected from the group consisting of NR_{6c}, O or S;
R₄ₐ, R_{4b}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

3. The compound or the pharmaceutically acceptable salt, the stereoisomer or the deuteride thereof according to claim 1, being a compound of formula (II) or a pharmaceutically acceptable salt, a stereoisomer or a deuteride thereof: 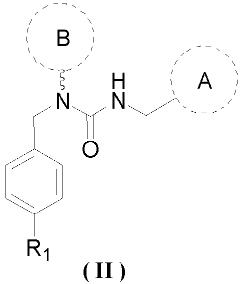 wherein
R₁ is halogen;
ring B is selected from the group consisting of 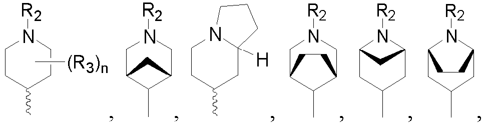 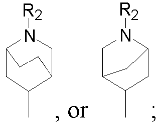
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 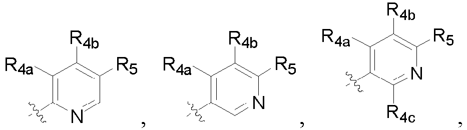 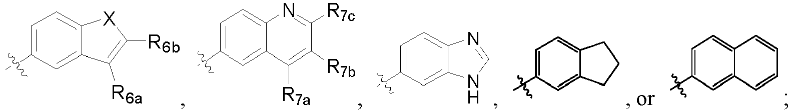
X is selected from the group consisting of NR_{6c}, O or S;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

4. The compound or the pharmaceutically acceptable salt, the stereoisomer or the deuteride thereof according to claim 1, being a compound of formula (IIA) or a pharmaceutically acceptable salt, a stereoisomer or a deuteride thereof: 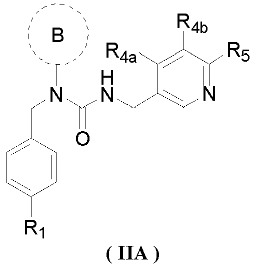 wherein
R₁ is halogen;
ring B is selected from the group consisting of 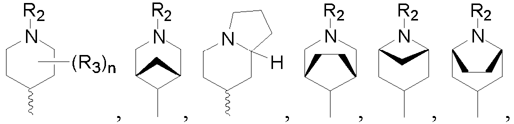 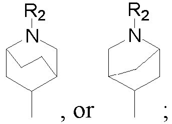
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl; R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, or C₃₋₆ halocycloalkyl;
n is selected from the group consisting of 0, 1, 2, or 3.

5. The compound or the pharmaceutically acceptable salt, the stereoisomer or the deuteride thereof according to claim 1, being a compound of formula (III) or a pharmaceutically acceptable salt, a stereoisomer or a deuteride thereof: 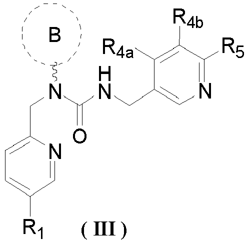 wherein
R₁ is halogen;
ring B is selected from the group consisting of 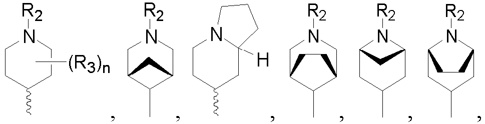 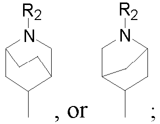
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ alkyl, C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

6. The compound or the pharmaceutically acceptable salt, the stereoisomer or the deuteride thereof according to any one of claims 1-5, wherein
ring B is 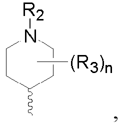
preferably 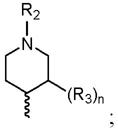 R₃ is selected from the group consisting of halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2.

7. The compound or the pharmaceutically acceptable salt, the stereoisomer or the deuteride thereof according to any one of claims 1-6, wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl, preferably 2,2,2-trifluoroethyl, cyclopropyl, cyclobutyl, 3,3-difluorocyclobutyl, cyclopropylmethyl, or 2,2-dimethylcyclopropylmethyl.

8. The compound or the pharmaceutically acceptable salt, the stereoisomer or the deuteride thereof according to any one of claims 1-5, wherein
R₁ is halogen;
ring B is 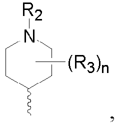
preferably 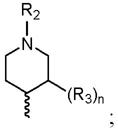
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 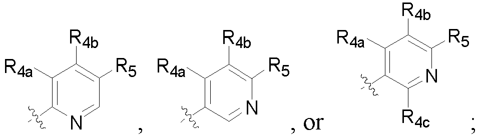
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3.

9. The compound or the pharmaceutically acceptable salt, the stereoisomer or the deuteride thereof according to claim 1, being a compound of formula (IV) or a pharmaceutically acceptable salt, a stereoisomer or a deuteride thereof: 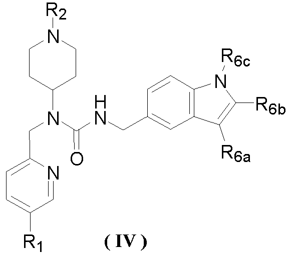 wherein
R₁ is halogen;
R₂ is selected from the group consisting of hydrogen or C₁₋₃ alkyl;
R₆ₐ and R_{6b} are each independently selected from the group consisting of hydrogen, halogen, or C₁₋₃ haloalkyl;
R_{6c} is selected from the group consisting of hydrogen or C₁₋₃ alkyl.

10. The compound or the pharmaceutically acceptable salt, the stereoisomer or the deuteride thereof according to claim 1, being a compound of formula (V) or a pharmaceutically acceptable salt, a stereoisomer or a deuteride thereof: 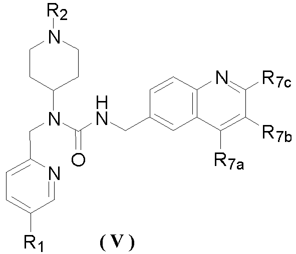 wherein
R₁ is halogen;
R₂ is selected from the group consisting of hydrogen or C₁₋₃ alkyl;
R₇ₐ, R_{7b}, and R_{7c} are each independently selected from the group consisting of hydrogen, halogen, or C₁₋₃ haloalkyl.

11. The compound or the pharmaceutically acceptable salt, the stereoisomer or the deuteride thereof according to claim 1, selected from the group consisting of the following compounds or pharmaceutically acceptable salts, stereoisomers or deuterides thereof:

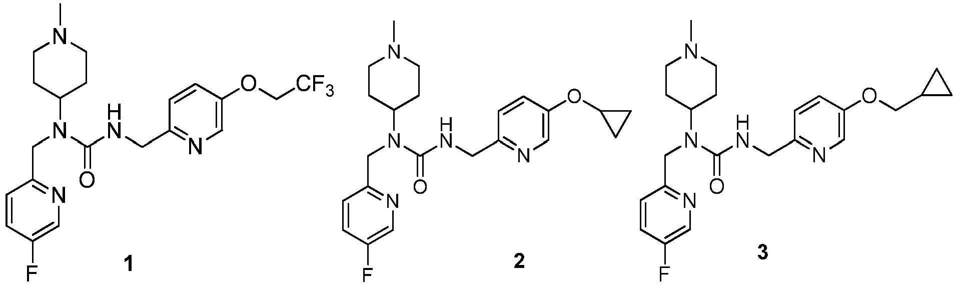

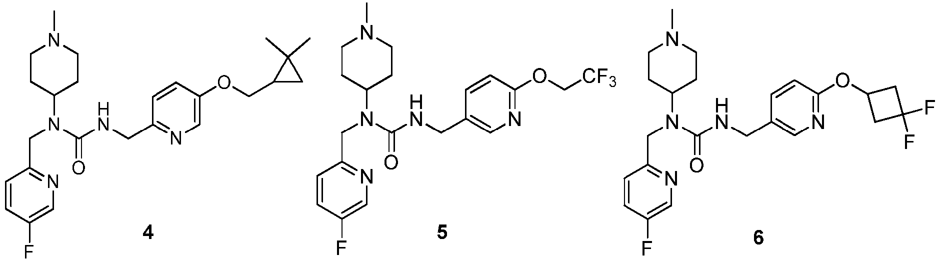

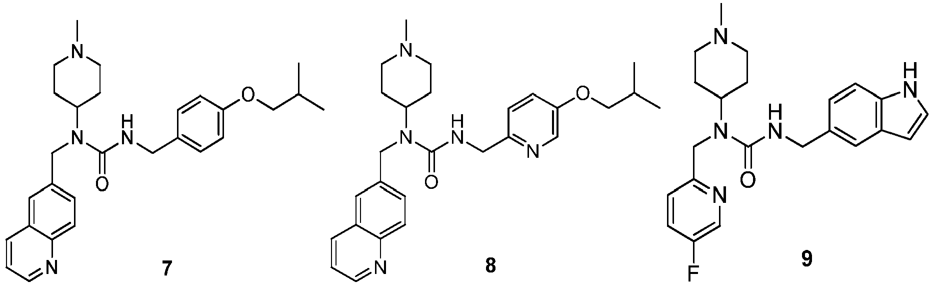

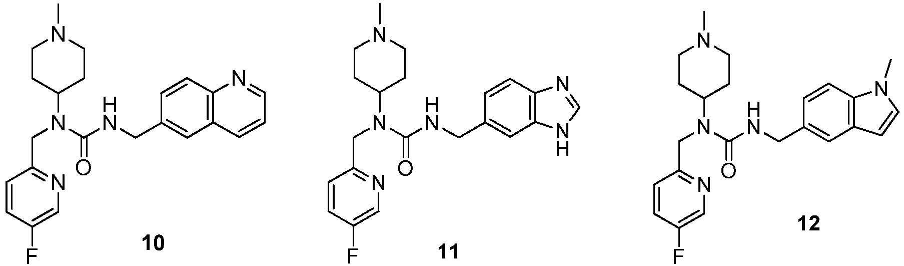

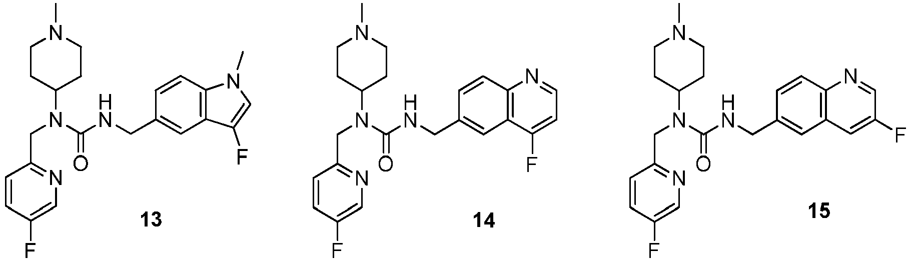

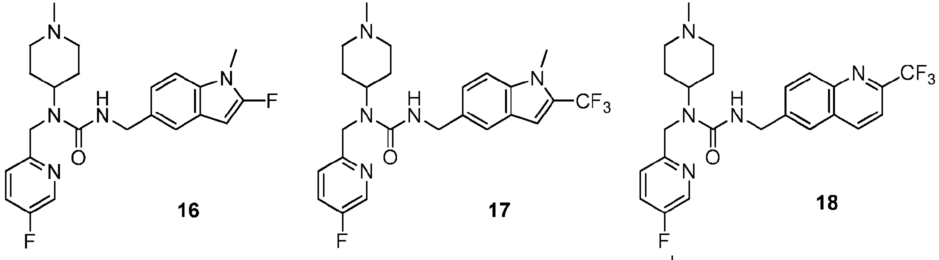

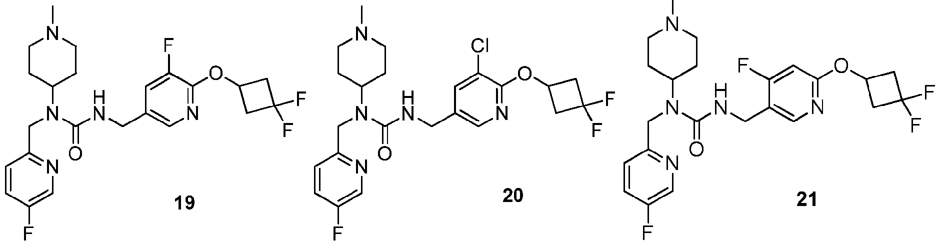

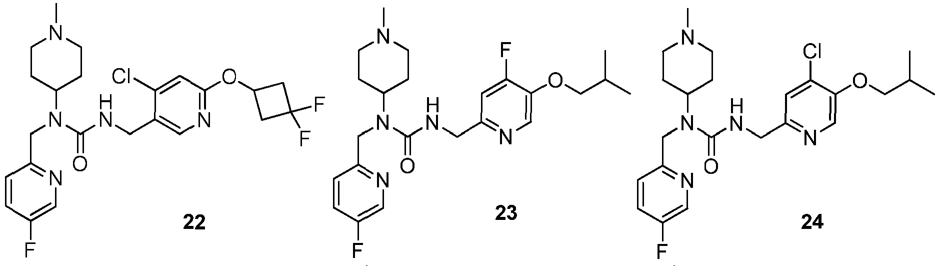

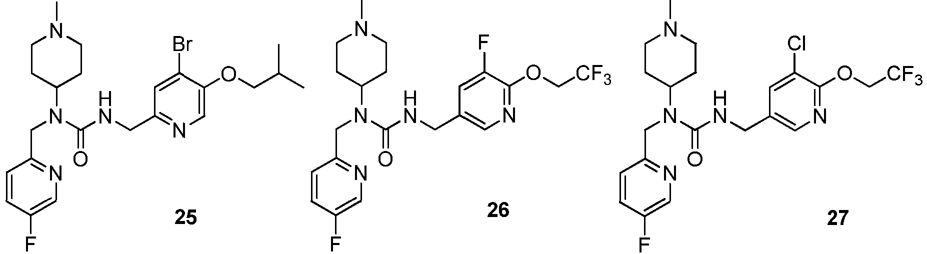

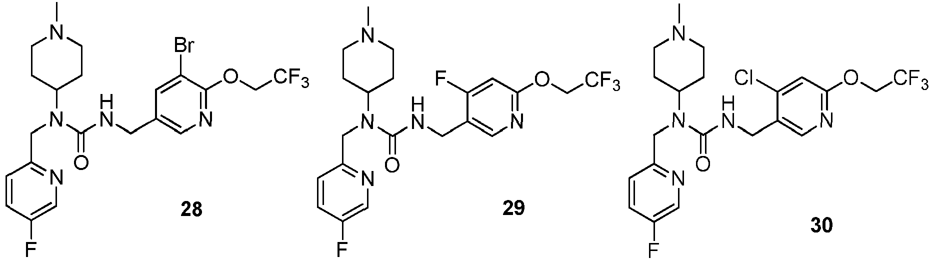

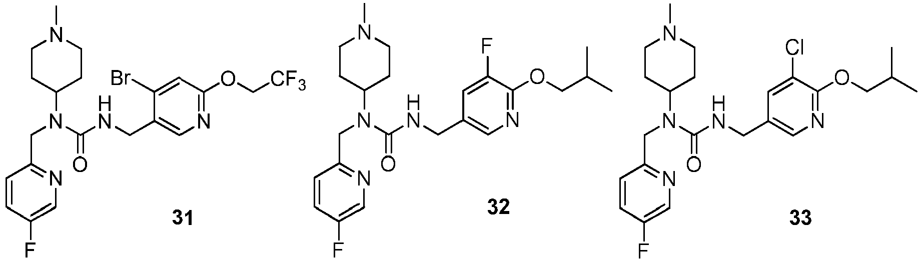

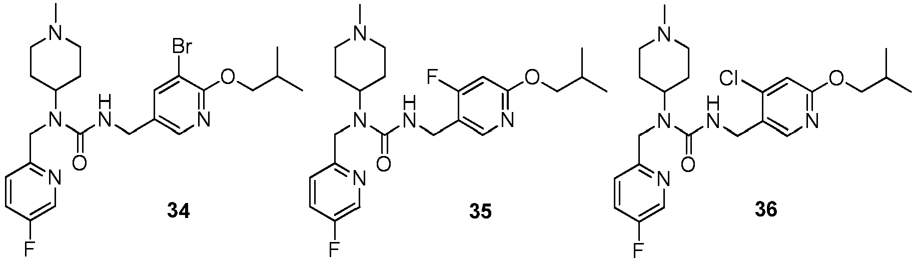

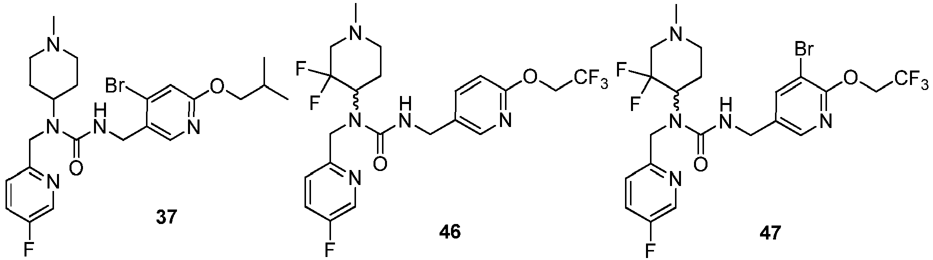

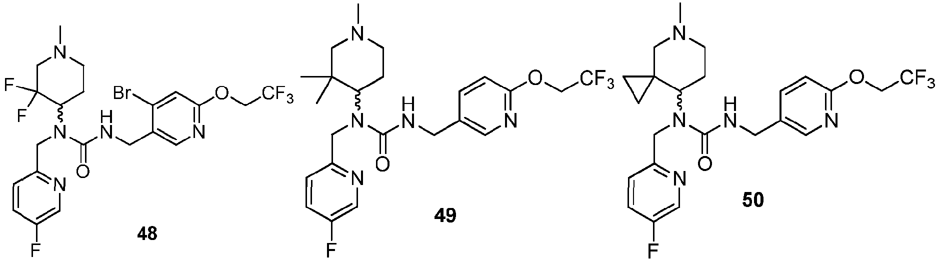

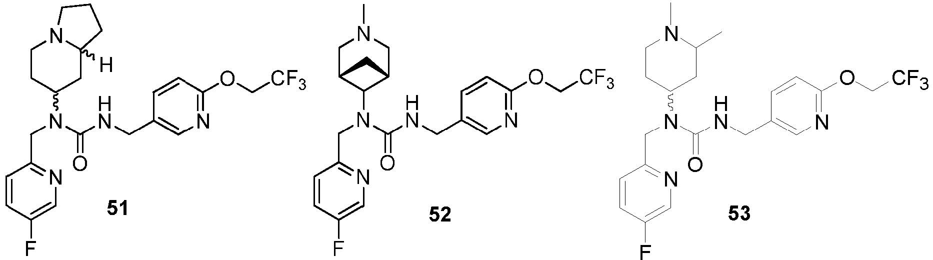

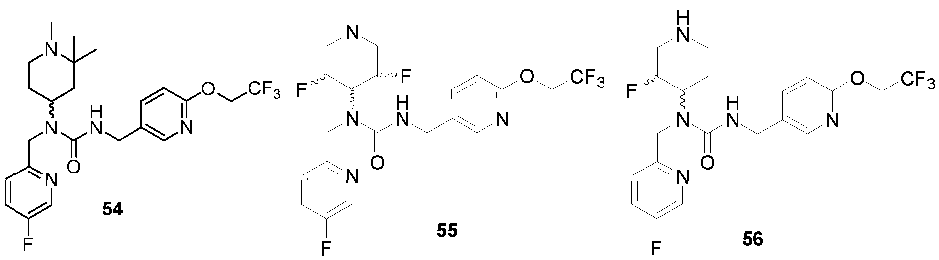

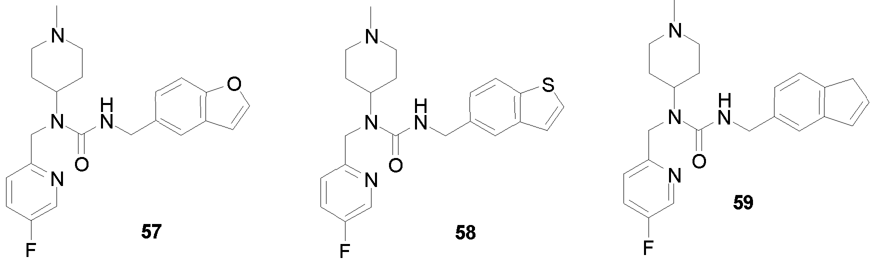

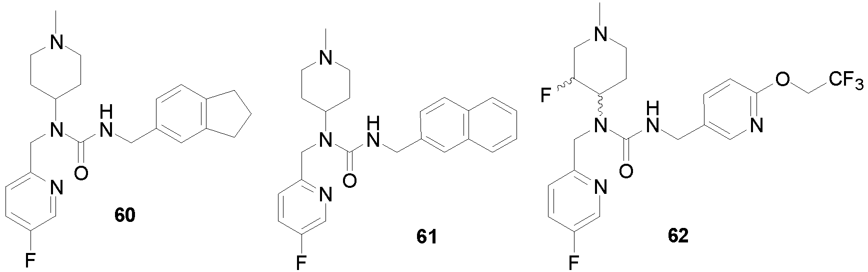

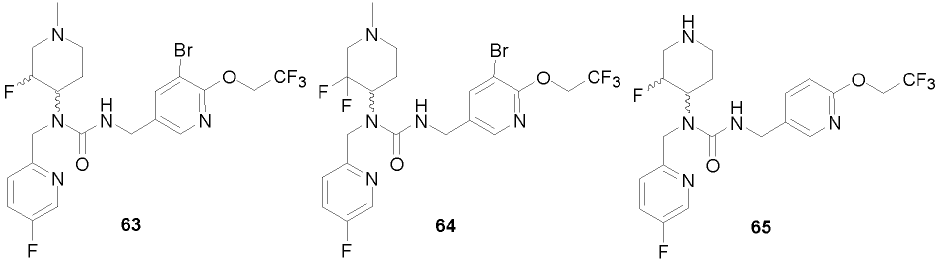

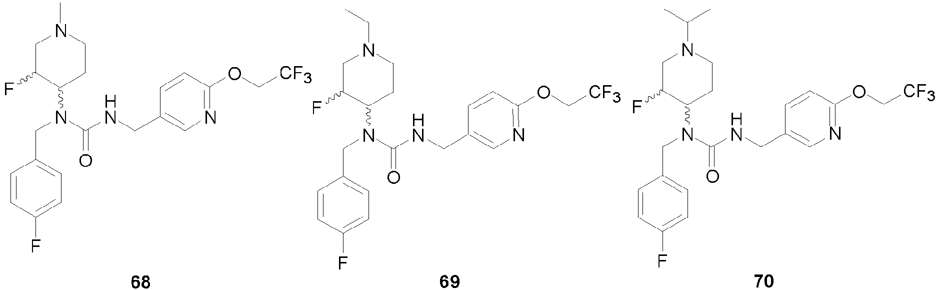

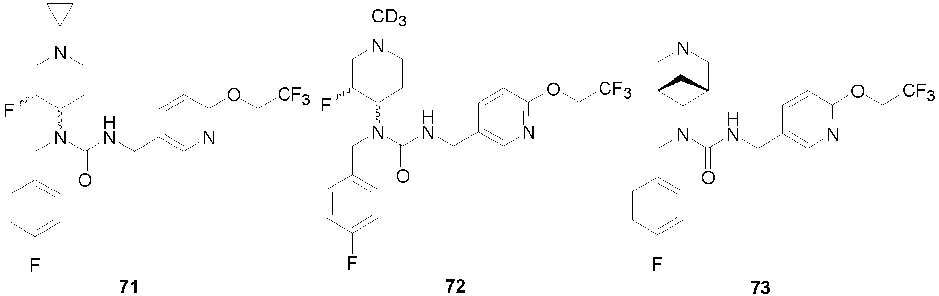

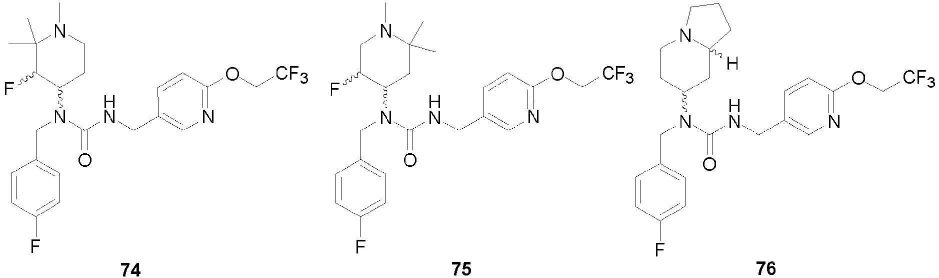

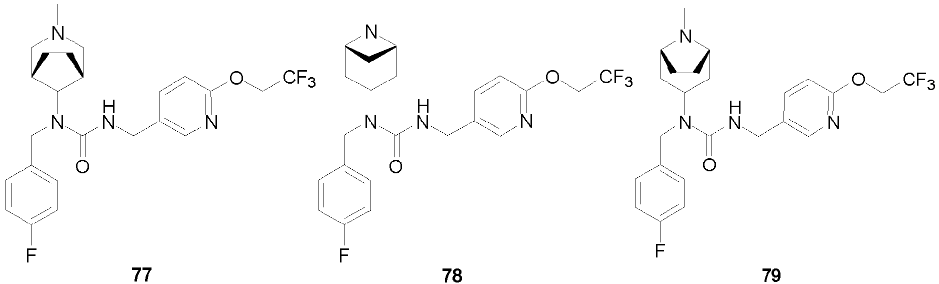

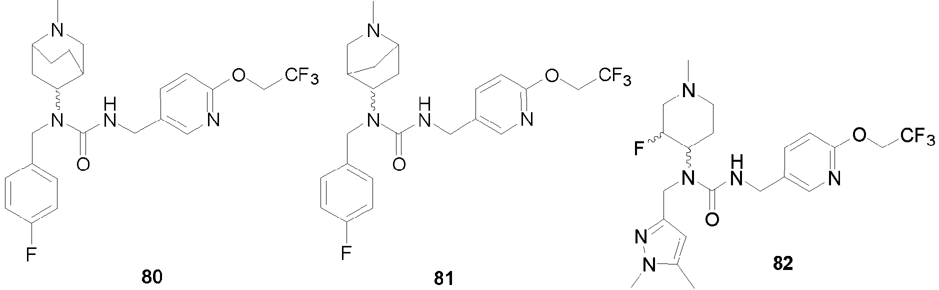

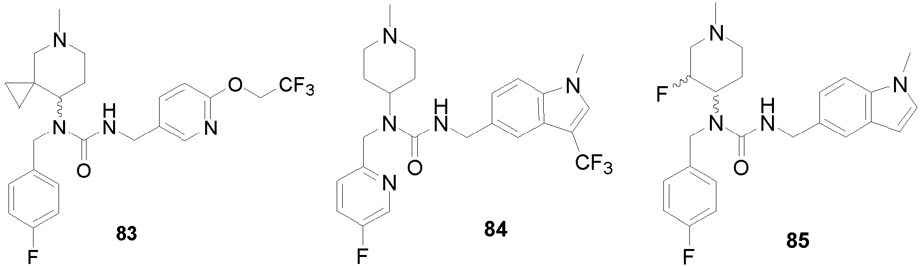

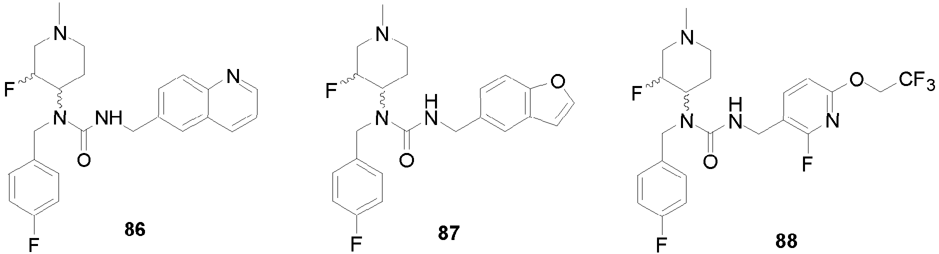

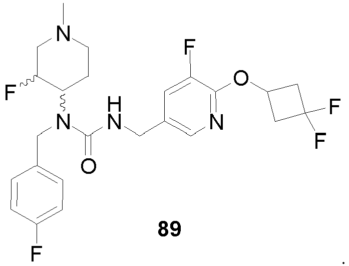

12. The compound or the pharmaceutically acceptable salt or the deuteride thereof according to claim 1, selected from the group consisting of the following compounds or pharmaceutically acceptable salts or deuterides thereof:

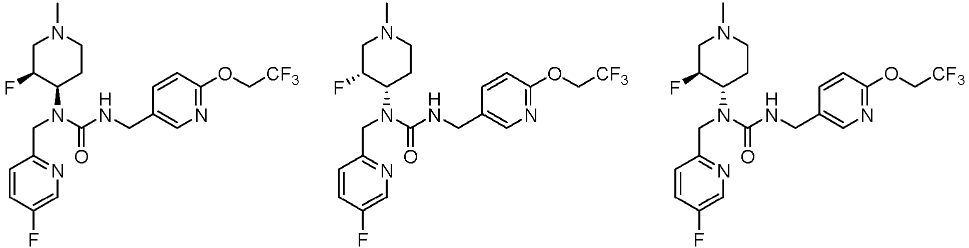

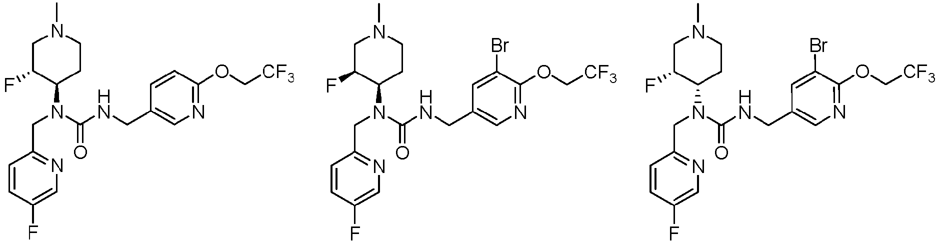

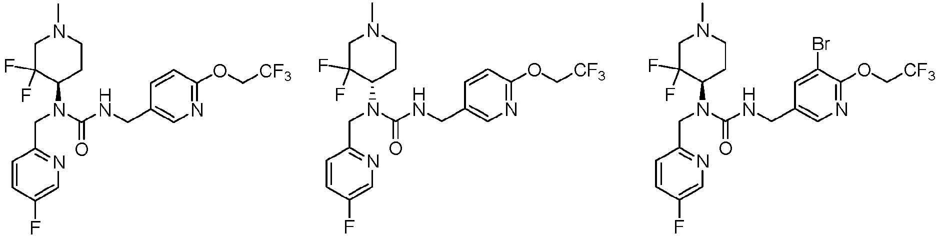

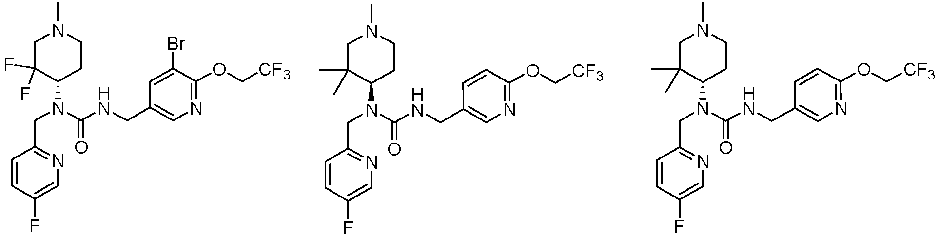

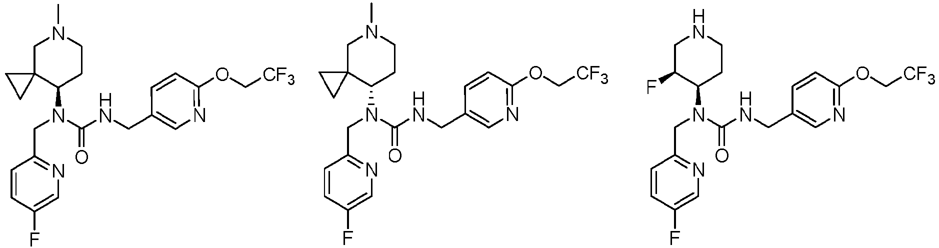

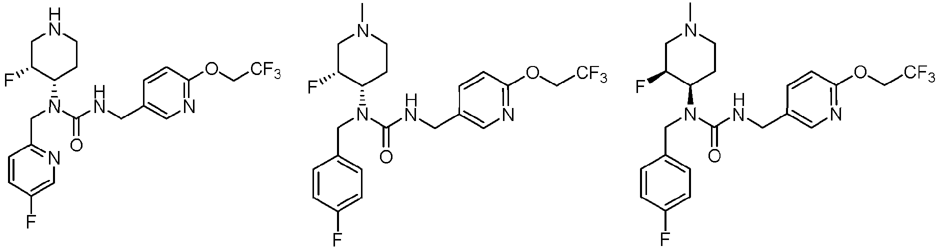

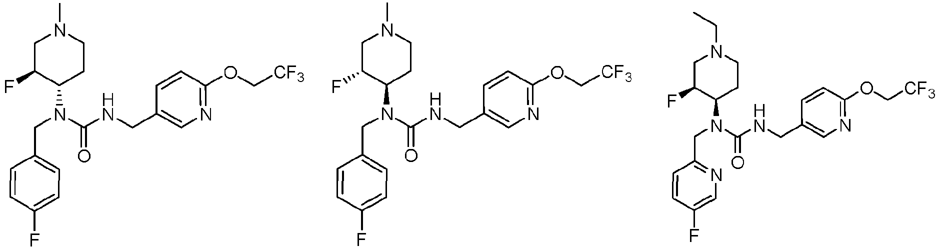

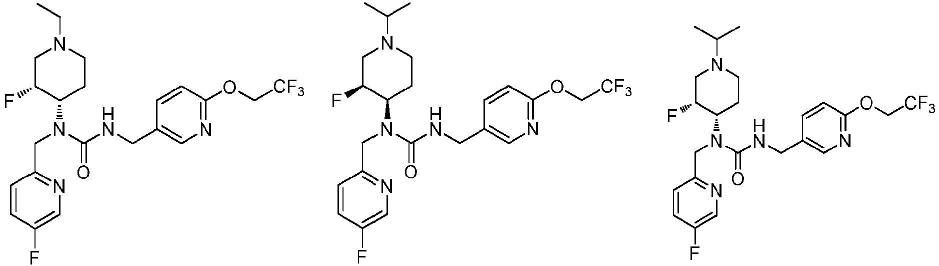

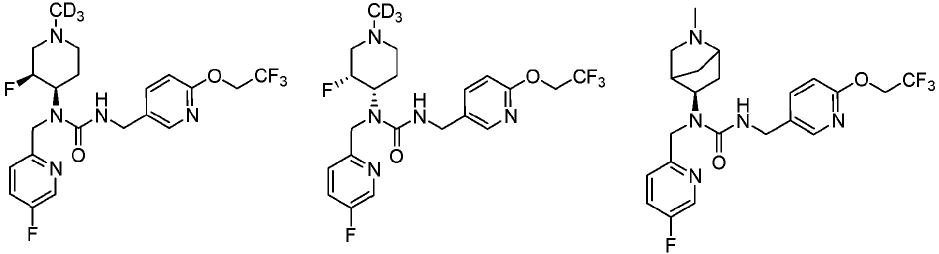

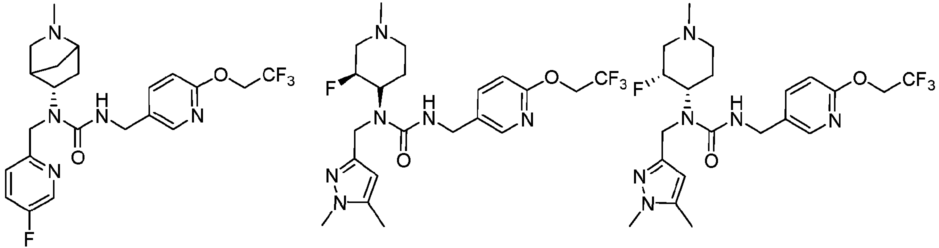

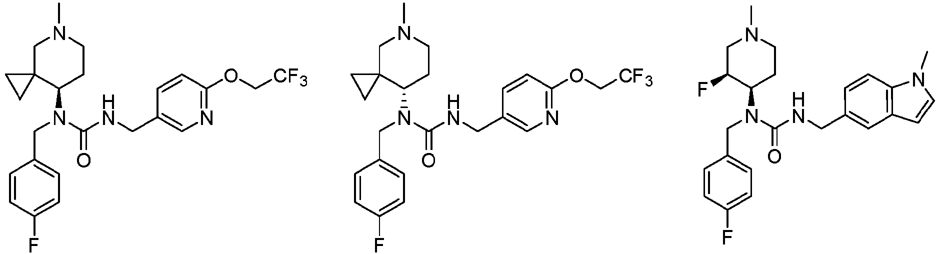

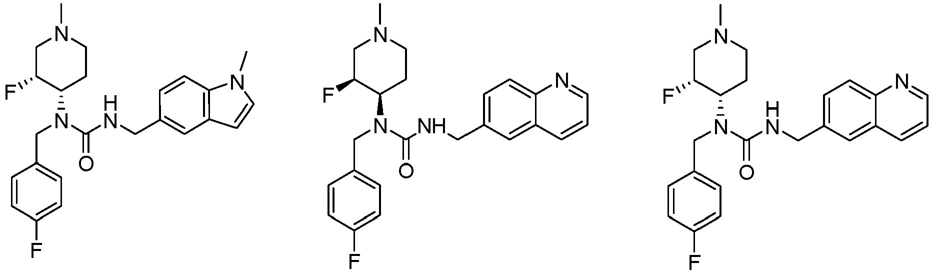

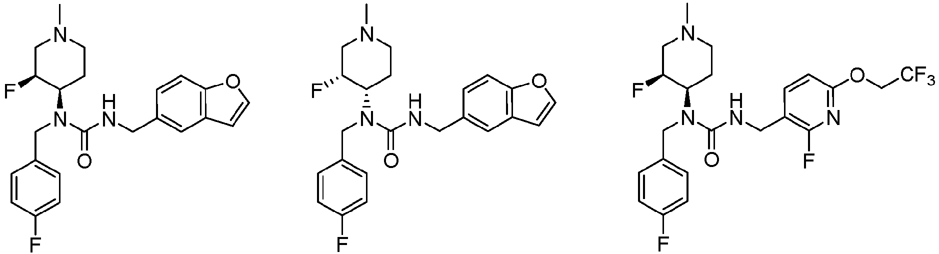

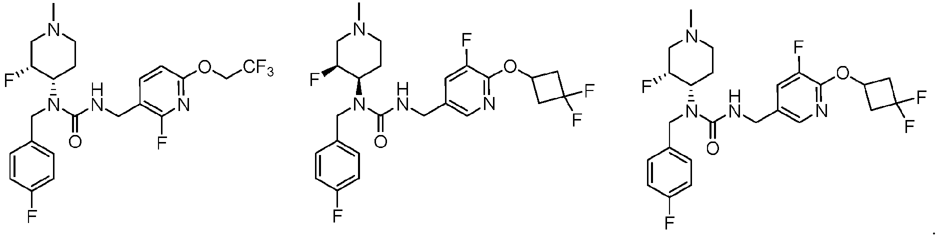

13. A pharmaceutical composition, comprising the compound or the pharmaceutically acceptable salt, the stereoisomer or the deuteride thereof according to any one of claims 1-12, and a pharmaceutically acceptable carrier.

14. Use of the compound or the pharmaceutically acceptable salt, the stereoisomer or the deuteride thereof according to any one of claims 1-12 or the pharmaceutical composition according to claim 13 in the preparation of a medicament for treating a 5-HT_{2A} receptor-related disease.

15. The use according to claim 14, wherein the 5-HT_{2A} receptor-related disease comprises: schizophrenia, psychosis, schizoaffective disorder, manic disorder, psychotic depression, affective disorder, dementia, anxiety disorder, sleep disorder, appetite disorder, bipolar disorder, psychosis secondary to hypertension, migraine, hypertension, thrombosis, vasospasm, ischemia, motor tics, depression, major depressive disorder, anxiety, sleep disturbance and appetite disturbance, nonmotor symptoms caused by Parkinson's disease (comprising delusion, hallucination, depression, anxiety, cognitive disorder, or sleep disorder), dementia-related mental diseases, negative symptoms of schizophrenia, Parkinson's disease, Huntington's chorea, Alzheimer's disease, spinocerebellar atrophy, Tourette's syndrome, Friedreich's ataxia, Machado-Joseph disease, Lewy body dementia, movement disorder, dystonia, myoclonus, tremor, or progressive supranuclear palsy and frontotemporal dementia.

## Amended claims

### Amended claims under Art. 19.1 PCT

1. A compound of formula (A) or a pharmaceutically acceptable salt, a stereoisomer or a deuteride thereof: 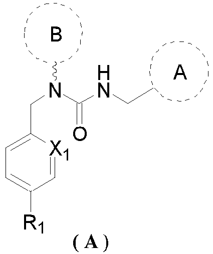
wherein R₁ is halogen;
ring B is selected from the group consisting of 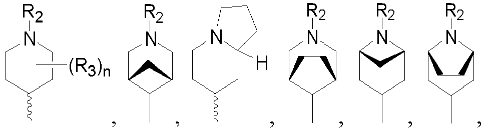 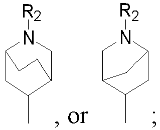
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 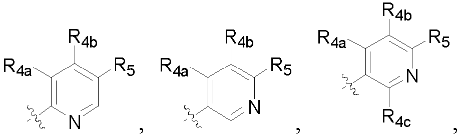 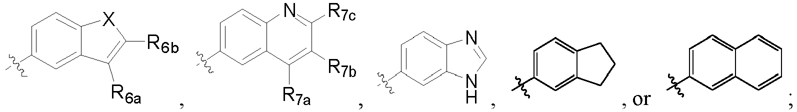
X is selected from the group consisting of NR_{6c}, or S;
X₁ is selected from the group consisting of N or CH;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

2. The compound or the pharmaceutically acceptable salt, the stereoisomer or the deuteride thereof according to claim 1, being a compound of formula (I) or a pharmaceutically acceptable salt, a stereoisomer or a deuteride thereof: 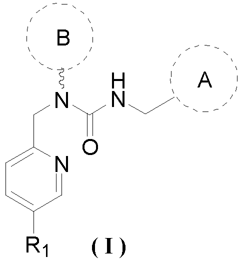 wherein
R₁ is halogen;
ring B is selected from the group consisting of 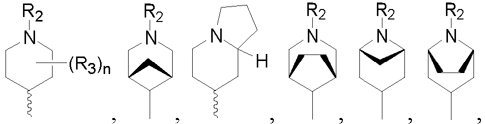 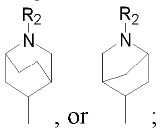
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 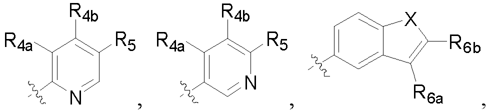 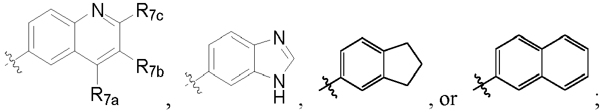
X is selected from the group consisting of NR_{6c}, or S;
R₄ₐ, R_{4b}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

3. The compound or the pharmaceutically acceptable salt, the stereoisomer or the deuteride thereof according to claim 1, being a compound of formula (II) or a pharmaceutically acceptable salt, a stereoisomer or a deuteride thereof: 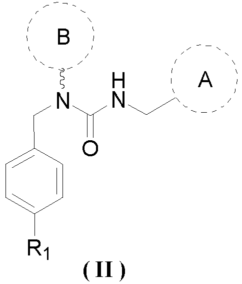 wherein
R₁ is halogen;
ring B is selected from the group consisting of 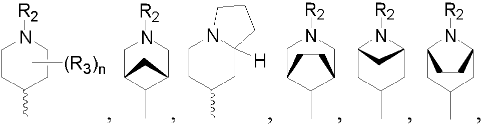 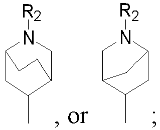
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 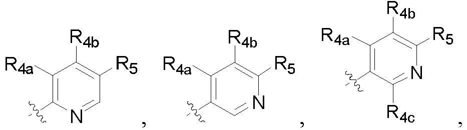 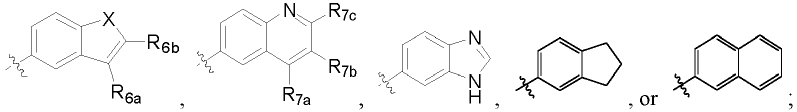
X is selected from the group consisting of NR_{6c} or S;
R₄ₐ, R_{4b}, R_{4c}, R₆ₐ, R_{6b}, R_{6c}, R₇ₐ, R_{7b}, and R_{7c} are each independently selected from the group consisting of hydrogen, halogen, C₁₋₃ alkyl, or C₁₋₃ haloalkyl;
R₅ is OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

4. The compound or the pharmaceutically acceptable salt, the stereoisomer or the deuteride thereof according to claim 1, being a compound of formula (IIA) or a pharmaceutically acceptable salt, a stereoisomer or a deuteride thereof: 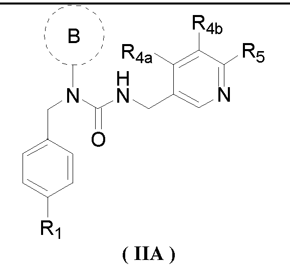 wherein
R₁ is halogen;
ring B is selected from the group consisting of 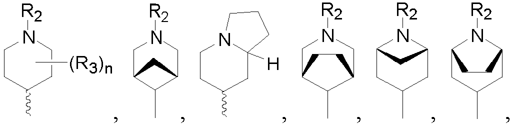 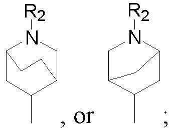
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl; R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, or C₃₋₆ halocycloalkyl;
n is selected from the group consisting of 0, 1, 2, or 3.

5. The compound or the pharmaceutically acceptable salt, the stereoisomer or the deuteride thereof according to claim 1, being a compound of formula (III) or a pharmaceutically acceptable salt, a stereoisomer or a deuteride thereof: 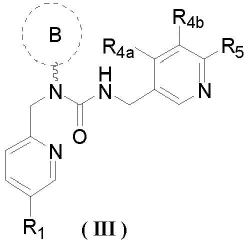 wherein
R₁ is halogen;
ring B is selected from the group consisting of 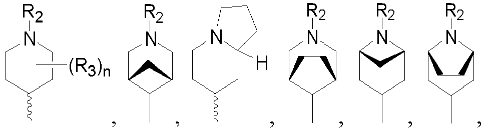 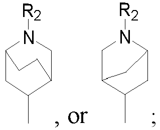
R₂ is independently selected from the group consisting of hydrogen, C₁₋₃ alkyl, or C₃₋₆ cycloalkyl; each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
R₄ₐ and R_{4b} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 0, 1, 2, or 3.

6. The compound or the pharmaceutically acceptable salt, the stereoisomer or the deuteride thereof according to any one of claims 1-5, wherein
ring B is 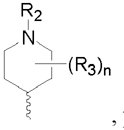
preferably 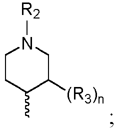
R₃ is selected from the group consisting of halogen, or two R₃ attached to the same carbon atom, together with the carbon atom to which they are attached, form C₃₋₆ cycloalkyl; R₃ is preferably F, or two R₃ attached to the same carbon atom, together with the carbon atom to which they are attached, form cyclopropyl;
n is 1 or 2.

7. The compound or the pharmaceutically acceptable salt, the stereoisomer or the deuteride thereof according to any one of claims 1-6, wherein R₅' is selected from the group consisting of 2,2,2-trifluoroethyl, cyclopropyl, cyclobutyl, 3,3-difluorocyclobutyl, cyclopropylmethyl, or 2,2-dimethylcyclopropylmethyl.

8. The compound or the pharmaceutically acceptable salt, the stereoisomer or the deuteride thereof according to any one of claims 1-5, wherein
R₁ is halogen;
ring B is 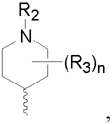
preferably 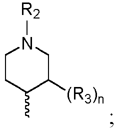
R₂ is independently selected from the group consisting of hydrogen or C₁₋₃ alkyl;
each R₃ is independently selected from the group consisting of hydrogen, halogen, or C₁₋₃ alkyl, or two R₃ attached to the same carbon atom, together with the carbon atom to which they are attached, form C₃₋₆ cycloalkyl;
ring A is selected from the group consisting of 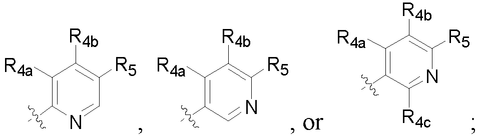
R₄ₐ, R_{4b}, and R_{4c} are each independently selected from the group consisting of hydrogen, halogen, C₁₋₃ alkyl, or C₁₋₃ haloalkyl;
R₅ is -OR₅', wherein R₅' is selected from the group consisting of C₁₋₆ haloalkyl, C₃₋₆ cycloalkyl, C₃₋₆ halocycloalkyl, C₃₋₆ cycloalkyl C₁₋₃ alkyl, or C₃₋₆ cycloalkyl C₁₋₃ alkyl optionally substituted with C₁₋₃ alkyl;
n is selected from the group consisting of 1, 2, or 3.

9. The compound or the pharmaceutically acceptable salt, the stereoisomer or the deuteride thereof according to claim 1, being a compound of formula (IV) or a pharmaceutically acceptable salt, a stereoisomer or a deuteride thereof: 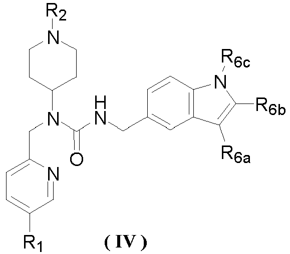 wherein
R₁ is halogen;
R₂ is selected from the group consisting of hydrogen or C₁₋₃ alkyl;
R₆ₐ and R_{6b} are each independently selected from the group consisting of hydrogen, halogen, or C₁₋₃ haloalkyl;
R_{6c} is selected from the group consisting of hydrogen or C₁₋₃ alkyl.

10. The compound or the pharmaceutically acceptable salt, the stereoisomer or the deuteride thereof according to claim 1, being a compound of formula (V) or a pharmaceutically acceptable salt, a stereoisomer or a deuteride thereof: 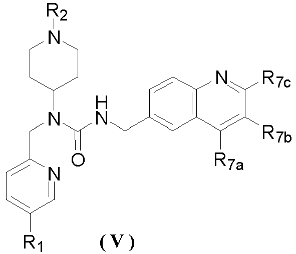 wherein
R₁ is halogen;
R₂ is selected from the group consisting of hydrogen or C₁₋₃ alkyl;
R₇ₐ, R_{7b}, and R_{7c} are each independently selected from the group consisting of hydrogen, halogen, or C₁₋₃ haloalkyl.

11. The following compound or the pharmaceutically acceptable salt, the stereoisomer or the deuteride thereof, selected from the group consisting of the following compounds or pharmaceutically acceptable salts, stereoisomers or deuterides thereof:

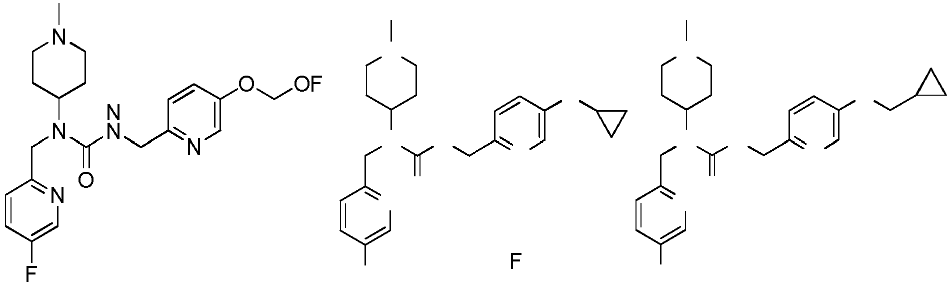

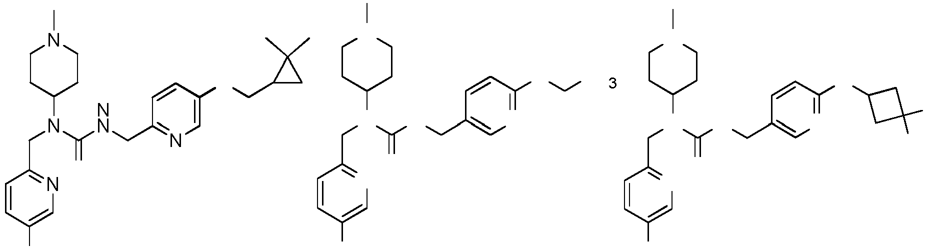

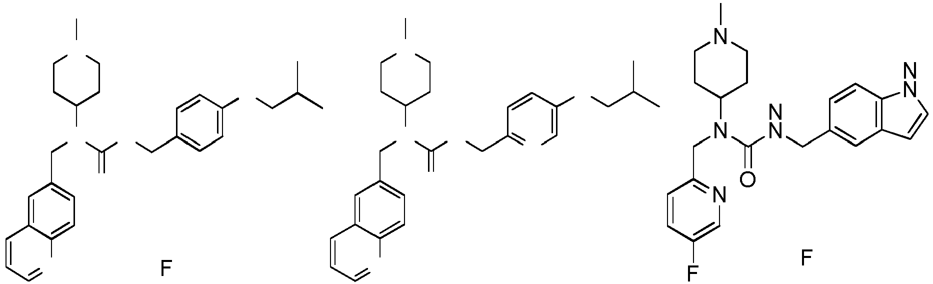

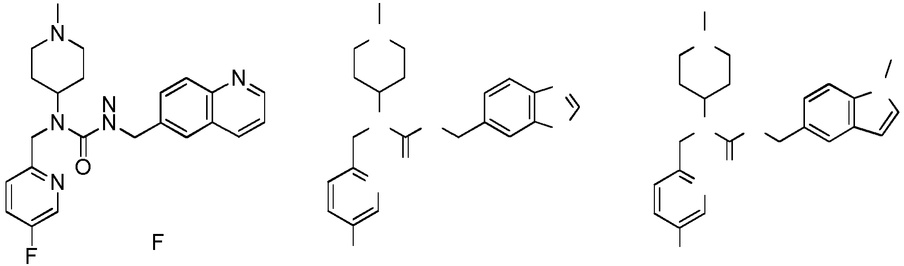

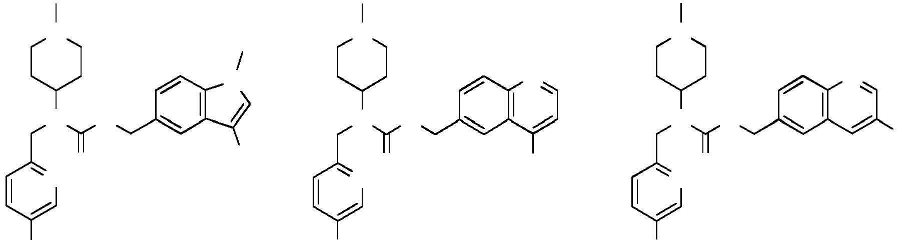

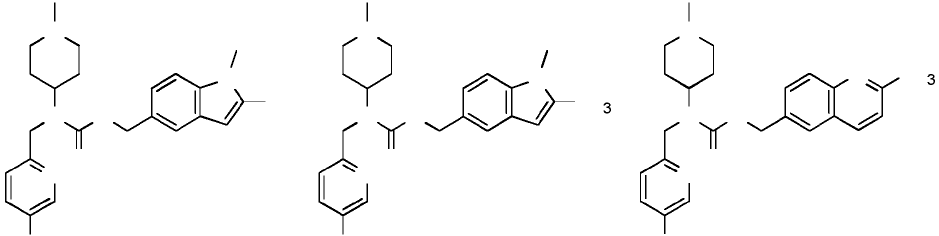

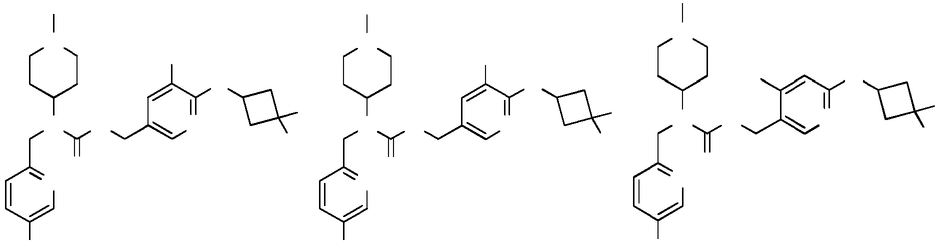

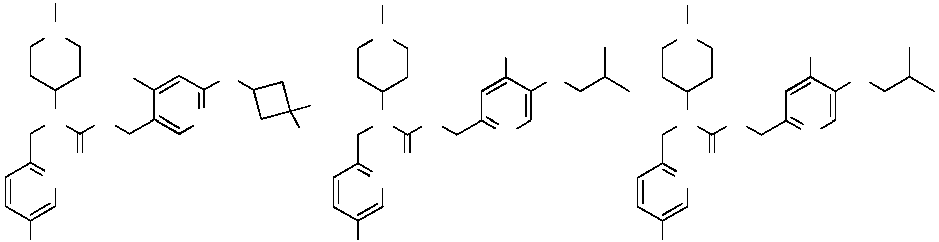

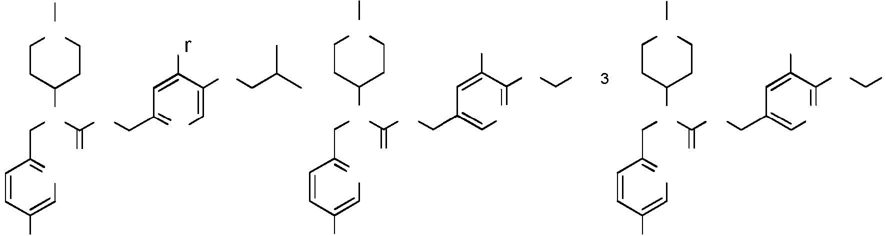

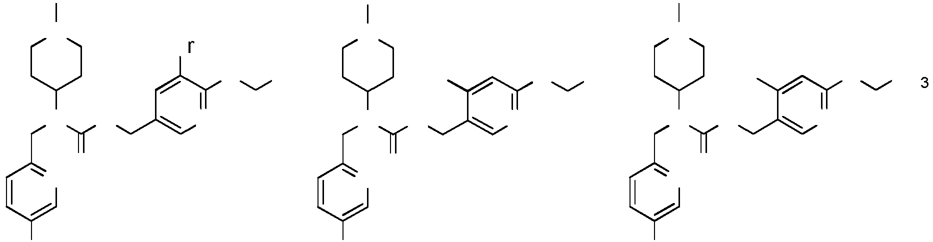

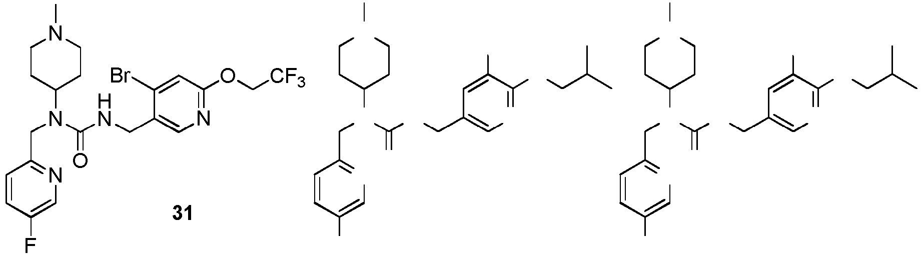

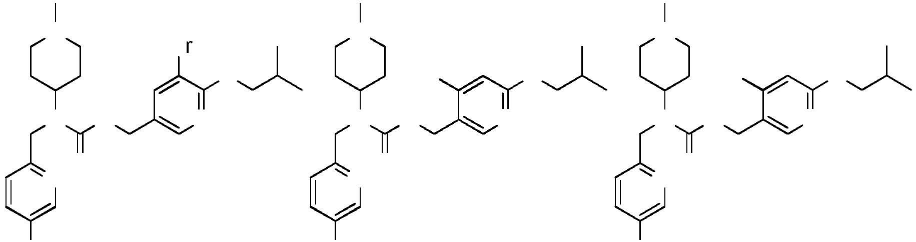

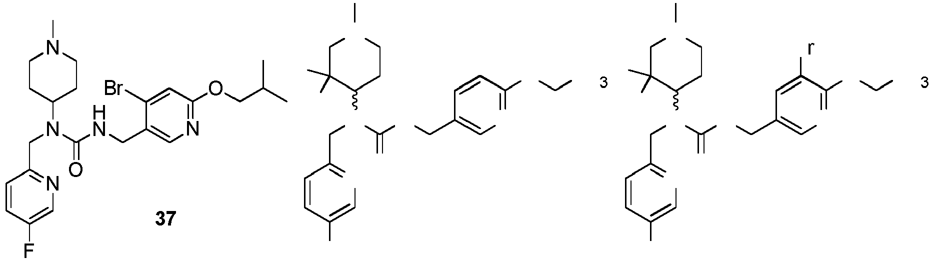

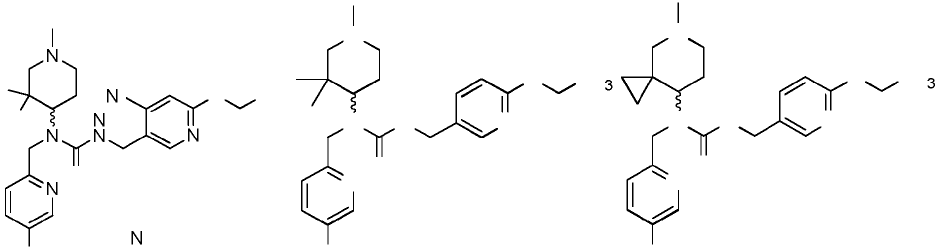

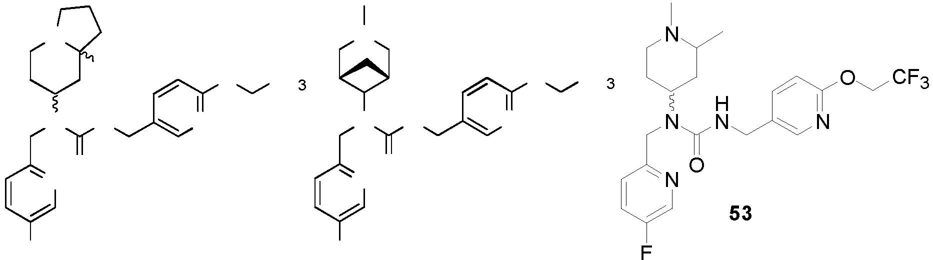

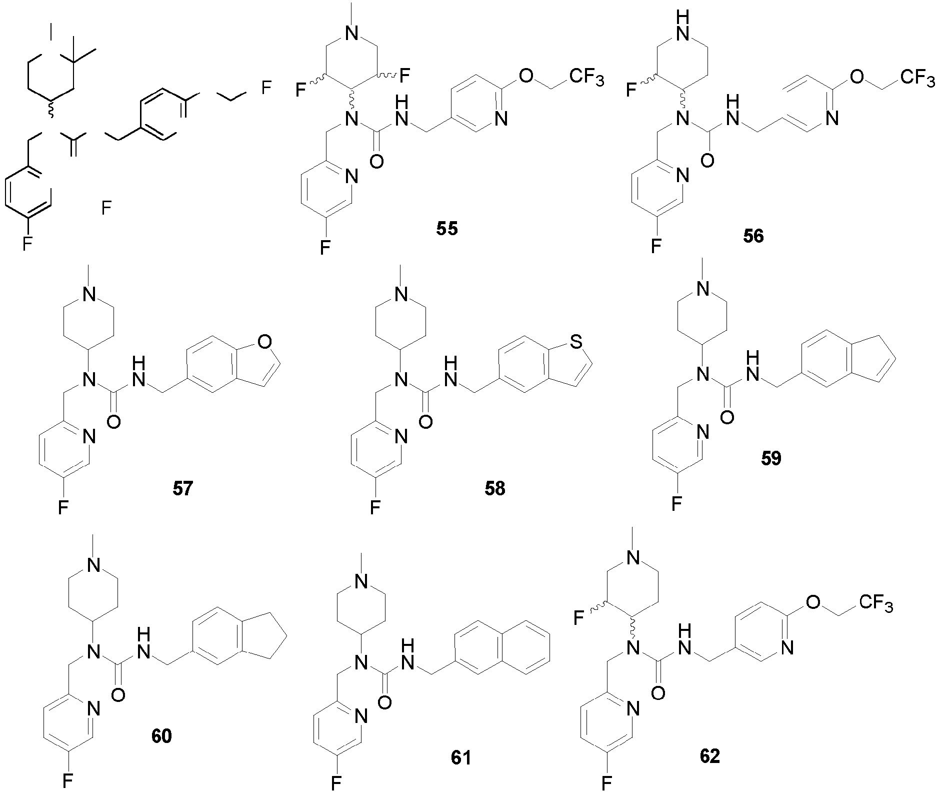

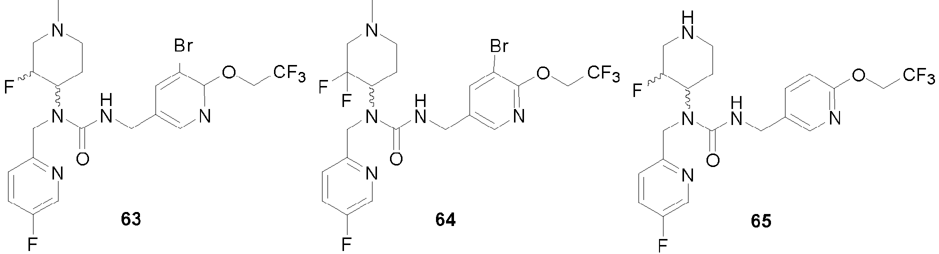

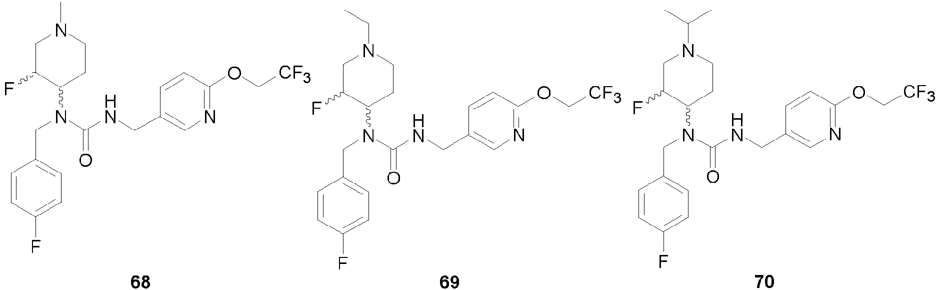

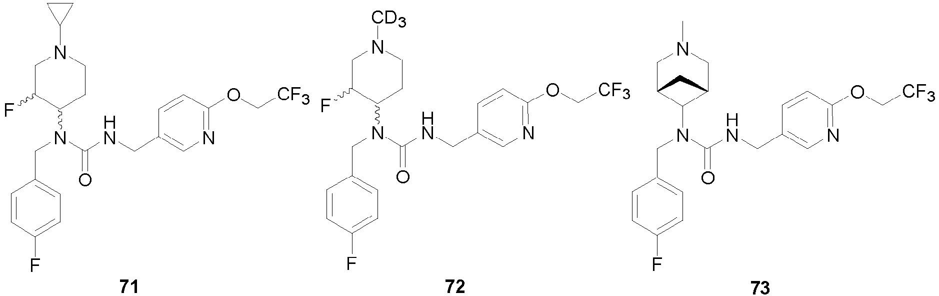

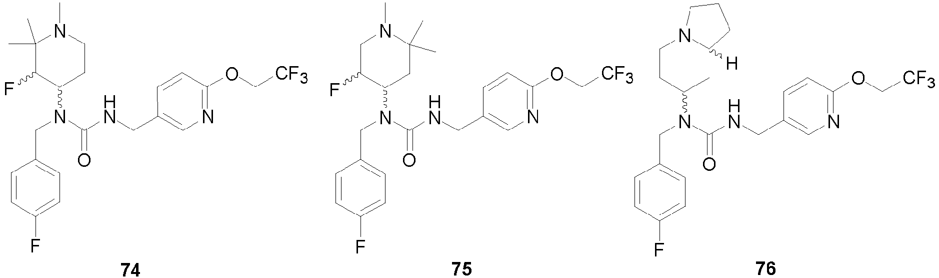

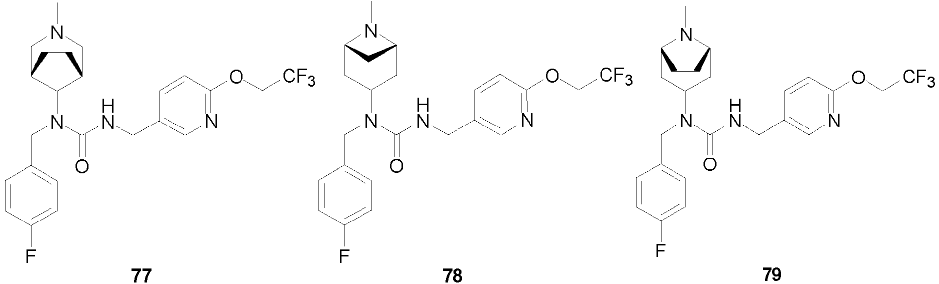

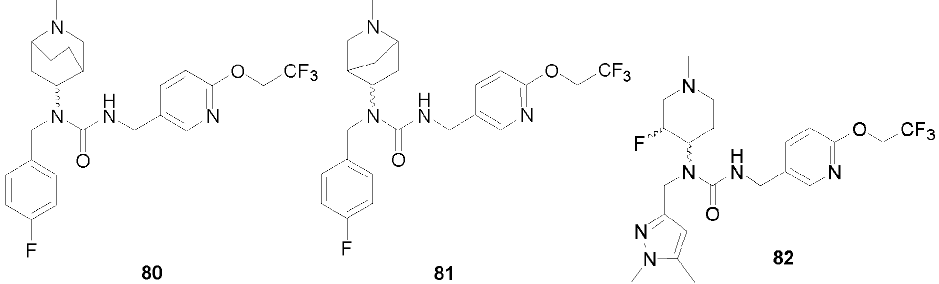

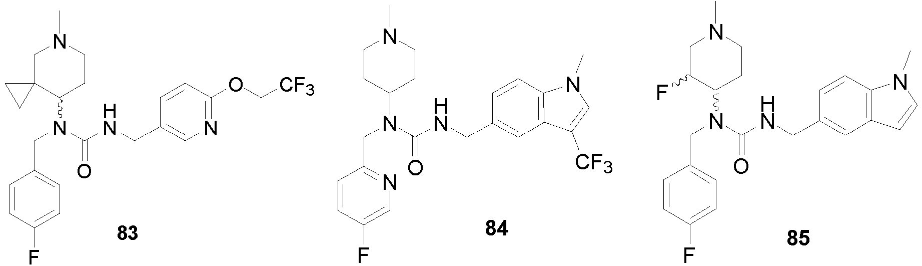

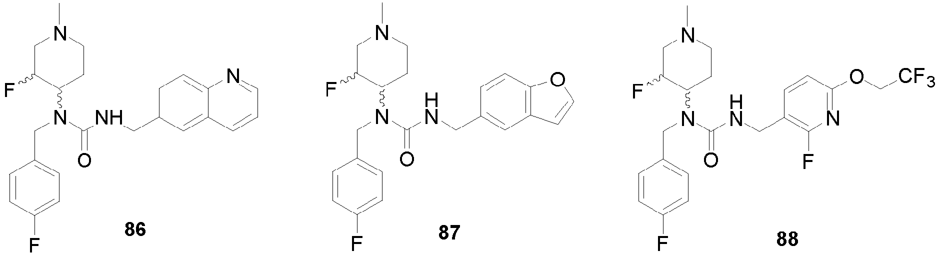

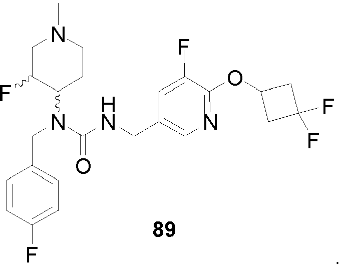

12. The following compound or the pharmaceutically acceptable salt or the deuteride thereof, selected from the group consisting of the following compounds or pharmaceutically acceptable salts or deuterides thereof:

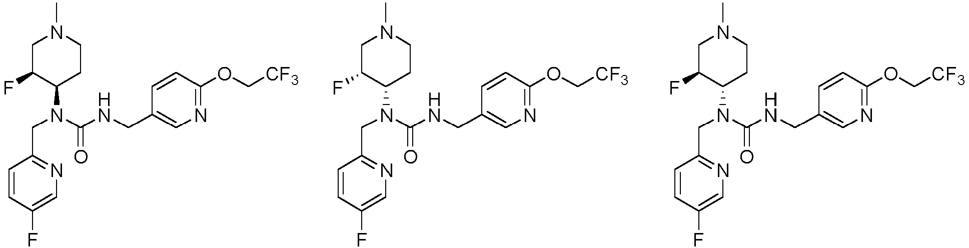

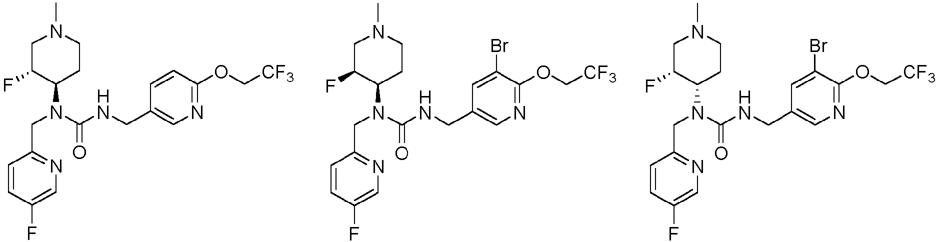

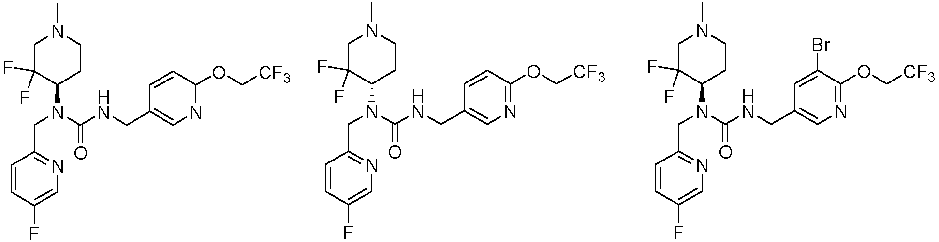

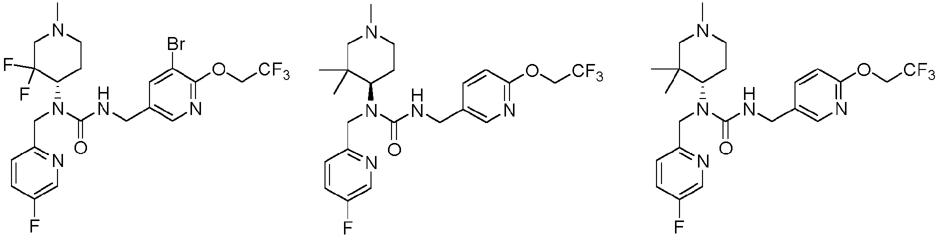

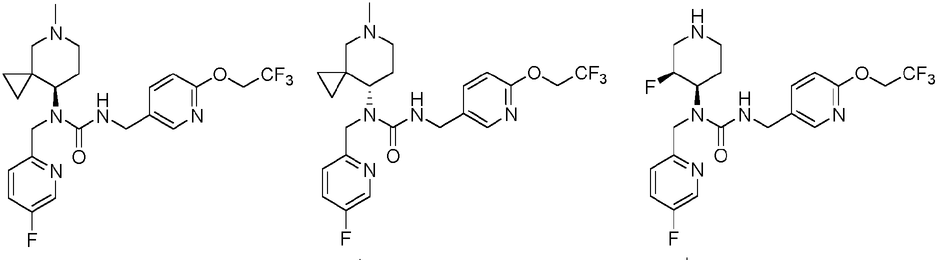

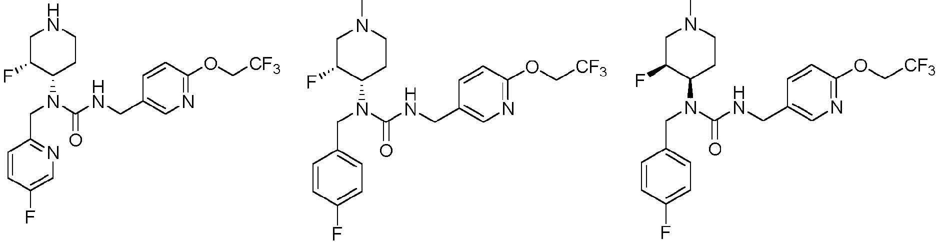

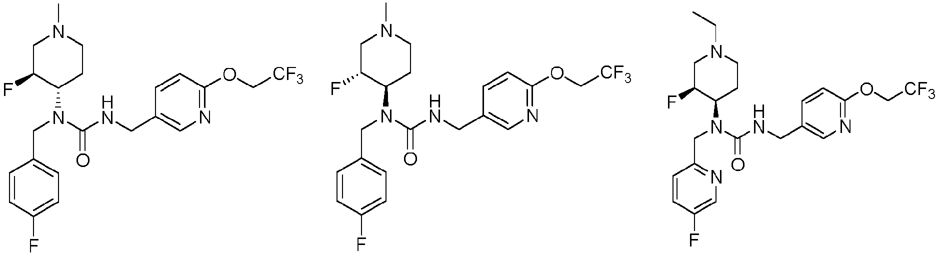

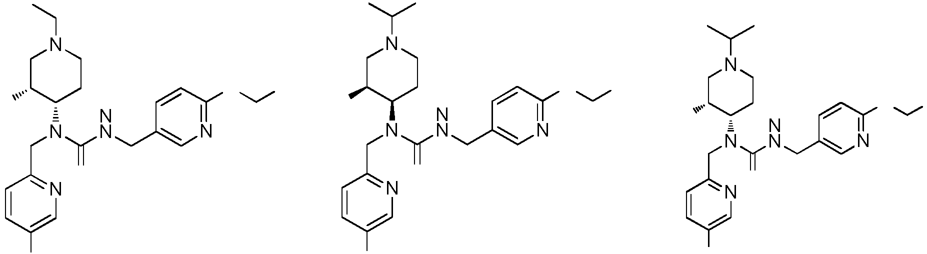

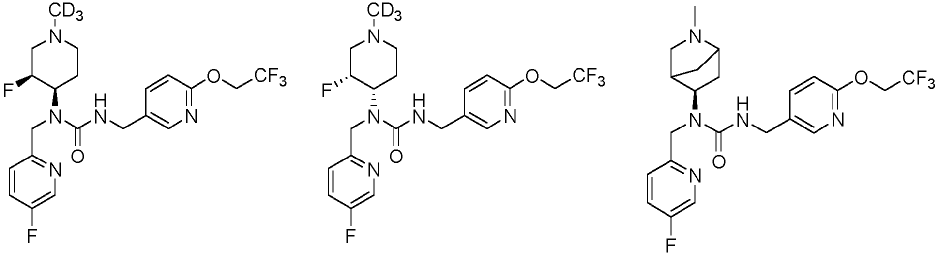

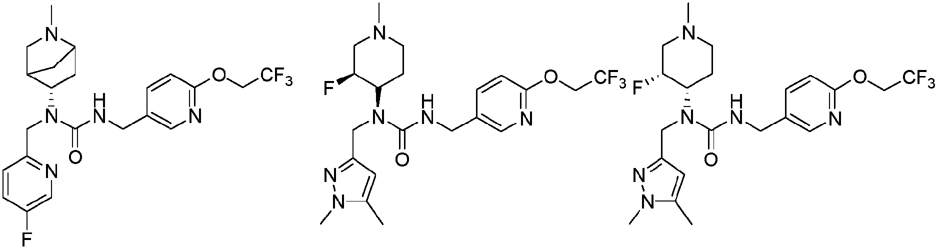

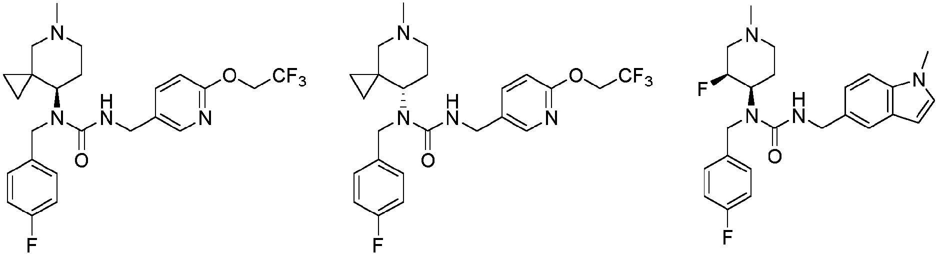

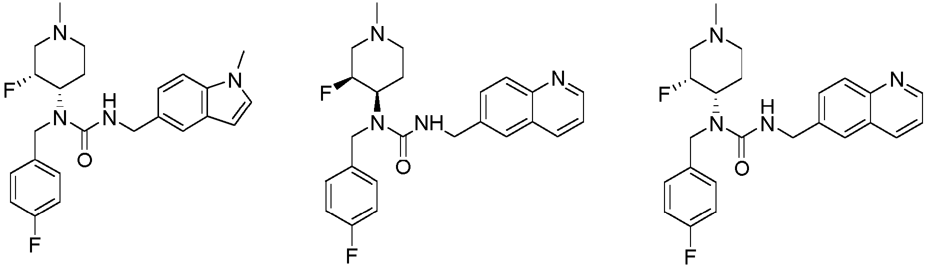

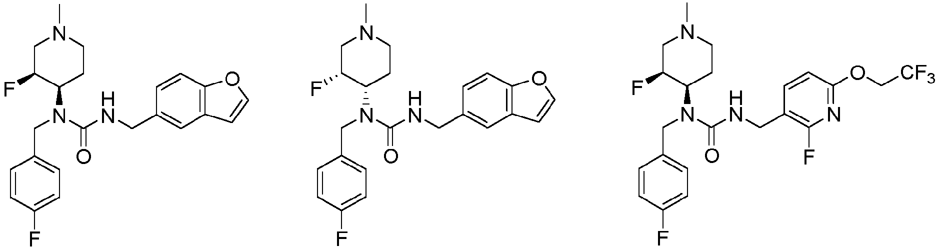

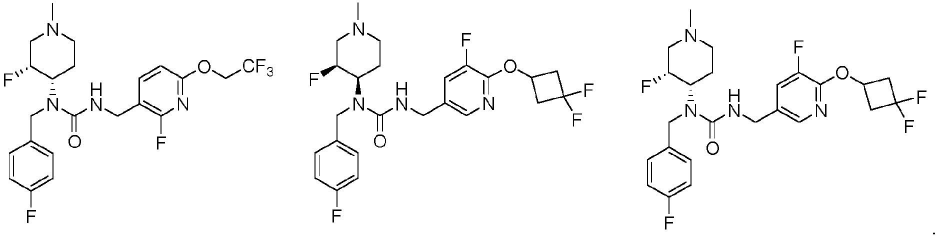

13. A pharmaceutical composition, comprising the compound or the pharmaceutically acceptable salt, the stereoisomer or the deuteride thereof according to any one of claims 1-12, and a pharmaceutically acceptable carrier.

14. Use of the compound or the pharmaceutically acceptable salt, the stereoisomer or the deuteride thereof according to any one of claims 1-12 or the pharmaceutical composition according to claim 13 in the preparation of a medicament for treating a 5-HT_{2A} receptor-related disease.

15. The use according to claim 14, wherein the 5-HT_{2A} receptor-related disease comprises: schizophrenia, psychosis, schizoaffective disorder, manic disorder, psychotic depression, affective disorder, dementia, anxiety disorder, sleep disorder, appetite disorder, bipolar disorder, psychosis secondary to hypertension, migraine, hypertension, thrombosis, vasospasm, ischemia, motor tics, depression, major depressive disorder, anxiety, sleep disturbance and appetite disturbance, non-motor symptoms caused by Parkinson's disease (comprising delusion, hallucination, depression, anxiety, cognitive disorder, or sleep disorder), dementia-related mental diseases, negative symptoms of schizophrenia, Parkinson's disease, Huntington's chorea, Alzheimer's disease, spinocerebellar atrophy, Tourette's syndrome, Friedreich's ataxia, Machado-Joseph disease, Lewy body dementia, movement disorder, dystonia, myoclonus, tremor, or progressive supranuclear palsy and frontotemporal dementia.
